(19) Europäisches Patentamt

European Patent Office

Office européen des brevets

(11) **EP 4 270 402 A1**

(12) **EUROPEAN PATENT APPLICATION**

(43) Date of publication:
**01.11.2023   Bulletin 2023/44**

(21) Application number: **22382392.3**

(22) Date of filing: **25.04.2022**

(51) International Patent Classification (IPC):
**G16H 10/20** (2018.01)       **G16H 10/60** (2018.01)
**G16H 15/00** (2018.01)       **G16H 50/70** (2018.01)

(52) Cooperative Patent Classification (CPC):
**G16H 10/20; G16H 10/60; G16H 15/00;**
**G16H 50/70;** G16H 50/20; G16H 80/00

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Fujitsu Limited**
**Kawasaki-shi, Kanagawa 211-8588 (JP)**

(72) Inventors:
• **GARCIA SANTA, Nuria**
**28010 Madrid (ES)**

• **CETINA, Kendrick**
**28011 Madrid (ES)**

(74) Representative: **Haseltine Lake Kempner LLP**
**Cheapside House**
**138 Cheapside**
**London EC2V 6BJ (GB)**

Remarks:
Amended claims in accordance with Rule 137(2)
EPC.

(54) **GENOGRAM CREATION AND DIAGNOSIS**

(57) A computer-implemented method comprising: using a trained family medical history, FMH, model trained to use Named Entity Recognition, NER, and Relation Extraction, RE, to extract entities and relations between the entities from unstructured data, extracting information from unstructured medical data for at least one family member of a target patient, the information including: a family member role indicating a relationship of the family member with the target patient; a status of the family member; and at least one observation of the family member; and transforming the extracted information into a genogram comprising a plurality of nodes respectively representing a plurality of people including the target patient and the at least one family member, wherein an edge between two nodes indicates a relationship between the people represented by the two nodes.

Figure 1

**Description**

The present invention relates to genogram creation and diagnosis, and in particular to a computer-implemented method, a computer program, an information processing apparatus, a wearable device, and a system.

It is common that members of a family have similar genetic backgrounds, lifestyles and environments. These factors may provide clues about medical conditions that may run in a family. The existence of medical disorders among relatives may mean that future generations in the family may be at increased risk of developing such disorders.

Therefore, being aware of one's family health history allows one to take steps to reduce potential health risks. Healthcare professionals may advise regular check-ups for patients with a condition that runs in their family.

In light of the above, improved methods for genogram creation and diagnosis are desirable.

According to an embodiment of a first aspect there is disclosed herein a computer-implemented method comprising: using a trained (deep-learning) family medical history, FMH, model trained to use Named Entity Recognition, NER, and Relation Extraction, RE, to extract entities and relations between the entities from unstructured data, extracting information from unstructured (medical (history)) data for at least one family member of a target patient, the information including: a family member role (keyword) indicating a relationship of the family member with the target patient; a status of the family member (including at least one of alive, dead, and healthy); and at least one observation of the family member; transforming the extracted information into a genogram (comprising a plurality of nodes respectively representing a plurality of people including the target patient and the at least one family member, wherein an edge between two (said) nodes indicates a relationship between the people represented by the two (said) nodes).

As a comparative example, conventional tools may suffer from the following problems, among others:

- The user is not able to collaborate appropriately, making difficult the genogram creation. If for example a physician creates a genogram and undertakes a consultation with a target patient beforehand, the target patient may be nervous or may not know some important information and this may make the creation of the genogram difficult and result in a genogram which may not be useful. If for example a target patient creates a genogram, if they lack expertise and knowledge of genogram creation then the genogram creation may be difficult and time-consuming and result in a genogram with mistakes and which may not be useful.
- A long time taken to build the genogram (e.g. around 30 minutes on average).
- The definition of a current status of only one individual is allowed.
- No guided-assistance to help and make recommendations to users in the genogram creation
- Lack of flexibility in genogram representation (e.g. no personalized representations or requirements for the genogram creation).
- Genogram must be created manually from scratch.

Embodiments disclosed herein may solve these problems among others.

The computer-implemented method may further comprise training a family medical history model to obtain the trained FMH model, the training comprising: receiving unstructured training (medical history) data; dividing the unstructured training (medical history) data into sentences and tokenizing each sentence to extract tokens, (each token comprising (only) a word or a punctuation mark); performing noun detection among the tokens; identifying family member role keywords among the detected nouns (by comparing at least some of the detected nouns with a dictionary of family member role keywords) and storing the family member role keywords as part of an annotation dataset; identifying among the tokens at least one status keyword associated with a family member role keyword (by using a dictionary of status keywords) and storing the at least one status keyword in association with the family member role keyword in the annotation dataset; identifying among the tokens at least one observation associated with a family member role keyword and storing the at least one observation in association with the family member role keyword in the annotation dataset; using the (entities and relations in the) annotation dataset to annotate the unstructured training (medical history) data; and using the annotations in the unstructured training (medical history) data as ground truth information (and using at least one trained language model,) training the family medical history model to use NER and RE to extract as entities, from the unstructured training (medical history) data, information (entities and relations) included in the annotations. The information included in the annotations may be considered to correspond to the "extracted information" described above with reference to the definition of the first aspect.

Identifying at least one observation associated with a family member role keyword may include, the family member role keyword being a (first) subject family member role keyword: analyzing tokens occurring in the unstructured training (medical history) data between the appearance of the (first) subject family member role keyword and the next appearance of a family member role keyword and identifying at least one token (among those tokens) that fulfils at least one observation criterion as at least one observation associated with the subject family member role keyword.

The computer-implemented method may further comprise, for another family member role keyword (being a second subject family member role keyword) occurring in the unstructured training (medical history) data before the appearance of the (first) subject family member role keyword and separated from the (first) subject family member role keyword by a conjunctive element (being a comma or the word "and" or a sign indicating the word "and"), identifying the at least one token which was identified as the at least one observation associated with the (first) subject family member role keyword as at least one observation associated with the other (second) subject family member role keyword.

The computer-implemented method may further comprise, before identifying at least one observation associated with a family member role keyword, performing syntax tree dependency analysis to generate syntax tags for tokens, and the at least one observation criterion may comprise a criterion that a token must have (or be inside) a syntax tag of pobj or dobj or conj to be considered an observation.

Identifying at least one observation associated with a family member role keyword may include identifying a modality of the at least one observation. Storing the at least one observation may comprise storing in the annotation dataset the modality in association with the at least one observation.

Identifying at least one status keyword associated with a family member role keyword may comprise, if/when the at least one status keyword is part of a token comprising a family member role keyword, identifying the at least one status keyword as being associated with the family member role keyword.

Identifying at least one status keyword associated with a family member role keyword may comprise implementing a first status search comprising: analyzing tokens occurring in the unstructured training (medical history) data between the appearance of a family member role keyword, being a first family member role keyword, and the next appearance of a family member role keyword to identify at least one status keyword (among those tokens) (not already associated with a family member role keyword), and identifying the at least one status keyword as being associated with the first family member role keyword.

Identifying at least one status keyword associated with a family member role keyword may comprise implementing a second status search comprising: identifying a second (another) family member role keyword occurring in the unstructured training (medical history) data before the appearance of the first family member role keyword and separated from the first family member role keyword by a conjunctive element (being a comma or the word "and"); and identifying the at least one status keyword identified in the first status search as being associated with the second family member role keyword.

The computer-implemented method may further comprise identifying among the tokens a family side keyword (according to a dictionary of family side keywords) associated with a family member role keyword and storing the family side keyword in association with the family member role keyword in the annotation dataset, (wherein the family side keyword is one of a group including maternal and paternal).

Identifying a family side keyword associated with a family member role keyword may comprise, if/when the family side keyword is part of a token comprising a family member role keyword, identifying the family side keyword as being associated with the family member role keyword.

The computer-implemented method may further comprise using part-of-speech, POS, analysis to assign POS tags to tokens/words in the unstructured (medical history) data.

Performing noun detection may comprise detecting tokens/words with a POS tag of noun.

Annotating the unstructured training (medical history) data may comprise annotating the unstructured training (medical history) data using a JavaScript Object Notation, JSON, format.

Training the family medical history model may further comprise, in a step proceeding using the annotations in the unstructured training (medical history) data as ground truth information, transforming the annotated unstructured training (medical history) data to: a Begin-entity, Inside-entity, Other, BIO, format; or a Begin-entity, Inside-entity, Other, End, Single, BIOES, format; or a Begin-entity, Inside-entity, Last, Other, Unique, BILOU, format.

The computer-implemented method may further comprise training a family medical history model to obtain the trained family medical history model, the training comprising: receiving unstructured training (medical history) data; dividing the unstructured training (medical history) data into sentences and tokenizing each sentence to extract tokens, (the tokens comprising family member role keywords and/or status keywords); performing noun detection among the tokens (and part of speech extraction on the sentences); identifying family member role keywords among the detected nouns (by comparing at least some of the detected nouns with a dictionary of family member role keywords); extracting at least one group (of words/tokens/entities) (a plurality of groups each) comprising a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword; generating syntax tree tags for tokens (to analyze relationships between detected nouns/entities), and based on the syntax tree tags, identifying at least one observation associated with a family member role keyword, wherein the group including the family member role keyword includes the at least one observation; using the extracted groups to annotate the unstructured training (medical history) data; and using the annotations in the unstructured training (medical history) data as ground truth information (and using at least one trained language model,) training the family medical history model to use NER and RE to extract as entities, from the unstructured training (medical history) data, the information included in the annotations.

Identifying at least one observation associated with a family member role keyword may include identifying a modality of the at least one observation. The group including the family member role keyword and the at least one observation may include the modality.

The computer-implemented method may further comprise, for any token comprising a family member role keyword and not a status keyword, (or for any token comprising a family member role keyword) the family member role keyword being a first family member role keyword, implementing a first status search comprising: analyzing tokens occurring in the unstructured training (medical history) data between the appearance of the first family member role keyword and the next appearance of a family member role keyword to identify a status keyword (among those tokens), wherein the group including the first family member role keyword includes the identified status keyword.

The computer-implemented method may further comprise, for any token comprising a family member role keyword and not a status keyword which has not been extracted as a group comprising a status keyword after the first status search, implementing a second status search comprising: identifying a second (another) family member role keyword occurring in the unstructured training (medical history) data before the appearance of the first family member role keyword and separated from the first family member role keyword by a conjunctive element (being a comma or the word "and"), wherein the group comprising the second (other) family member role keyword includes the status keyword which is also included in the group comprising the first family member role keyword of the first status search.

For any token comprising a family member role keyword and a family side keyword (according to a dictionary of family side keywords), the group including the family member role keyword may include the family side keyword, (wherein the family side keyword is one of a group including maternal and paternal).

The computer-implemented method may further comprise: using the trained family medical history model to extract further said information from further unstructured (medical history) data for at least one of the family members of the target patient (the further information comprising at least one of: a family member role indicating a relationship of the family member with the target patient; a status of the family member (including at least one of a group comprising alive, dead, and healthy); and at least one observation of the family member); comparing the extracted information with the extracted further information and, when there is at least one inconsistency comprising a first entity in the extracted information and a second entity in the extracted further information, identifying the inconsistency (the first and second entities being an observation or a status of one of the family members of the target patient); (and displaying information indicating the inconsistency).

Contradiction may be used in place of inconsistency.

The computer-implemented method may further comprise: replacing the first entity with the second entity in the extracted information when the further unstructured (medical history) data is more reliable than the unstructured (medical history) data, and updating the genogram by using the second entity instead of the first entity; or recommending to a user replacement of the first entity with the second entity in the extracted information when the further unstructured (medical history) data is more reliable than the unstructured (medical history) data, and recommending to the user updating the genogram by using the second entity instead of the first entity.

The computer-implemented method may further comprise, before the step of replacing the first entity or recommending replacement of the first entity, determining that the further unstructured (medical history) data is more reliable than the unstructured (medical history) data when the further unstructured (medical history) data is focused on the family member associated with the first and second entities as a patient (and when the unstructured (medical history) data is focused on the target patient as a patient).

The family member role for a said family member may include information indicating a family side of the family member (the family side being maternal or paternal).

Each observation may include information indicating an observation modality (the observation modality being positive or negative).

The computer-implemented method may further comprise (before transforming the extracted information into the genogram) extracting information indicating a gender of at least one family member, optionally based on the family member role the at least one family member (and based on rules dictating correspondences between gender and family member roles).

The computer-implemented method may further comprise extracting the information from (the) unstructured (medical history) data for at least one family member for a plurality of family members of the target patient, and the computer-implemented method may further comprise (before transforming the extracted information into the genogram) extracting information of at least one relationship (relationships) between the family members based on the family member role of each family member (and based on rules dictating relationships between family member roles).

The computer-implemented method may further comprise including the extracted information of the at least one relationship (relationships) between the family members as at least one edge in the genogram.

Each node corresponding to a family member may be a data structure comprising a family member role, a status of the family member, and at least one observation of the family member.

The computer-implemented method may further comprise displaying (using a/the GUI) the genogram.

The computer-implemented method may further comprise using a (predetermined/stored/saved) template to transform the extracted information into the genogram.

The computer-implemented method may further comprise receiving user input to determine the template.

The computer-implemented method may further comprise receiving user input to generate the template.

The computer-implemented method may further comprise receiving user input to select the template from a list of templates.

The computer-implemented method may further comprise receiving user input to edit the template (before the template is used to transform the extracted information into the genogram).

The templates that may be selected by the user are stored, optionally as JSON configuration files; and/or optionally in a server.

The computer-implemented method may further comprise receiving user input to create a new template to be used to transform the extracted information into the genogram.

The computer-implemented method may further comprise storing information indicated by the user input as a JSON configuration file defining the new/edited template.

The computer-implemented method may further comprise receiving user input to select/define: a form of the nodes (optionally for each family member role and/or optionally depending on the gender of at least one family member); and/or a form of the edges (optionally for each type of relationship); and/or a configuration in which (or how) information will be displayed for each node.

The computer-implemented method may further comprise, after transforming the extracted information into the genogram, receiving user input to edit: a form of the nodes (optionally for each family member role and/or optionally depending on the gender of at least one family member); and/or a form of the edges (optionally for each type of relationship); and/or a configuration in which (or how) information will be displayed for each node; and/or text (displayed and) associated with each node.

Receiving user input to select/define/edit a form of the nodes may comprise receiving user input to select/define a shape and/or color and/or size of the nodes (optionally for each family member role).

Receiving user input to select/define/edit a form of the edges may comprise receiving user input to select/define a shape and/or color and/or size of the edges (optionally for each type relationship).

Receiving user input to select/define/edit a configuration in which information will be displayed for each node may comprise receiving user input to select/define a size and/or a position and/or a color and/or a format of text indicating the information.

The computer-implemented method may further comprise receiving user input to select the first entity or the second entity to be used in the genogram (or to select whether or not the second entity should replace the first entity in the extracted information and in the genogram).

The computer-implemented method may further comprise using a graphical user interface, GUI, to receive the user input.

The computer-implemented method may further comprise, when the user provisionally selects a form of the nodes (using the GUI), displaying, using the GUI, a preview of the (provisionally selected) form of the nodes.

The computer-implemented method may further comprise, when the user provisionally selects a form of the edges (using the GUI), displaying, using the GUI, a preview of the (provisionally selected) form of the edges.

The computer-implemented method may further comprise, when the user provisionally selects a configuration in which information will be displayed for each node (using the GUI), displaying, using the GUI, a preview of the (provisionally selected) configuration in which the information will be displayed for each node.

The computer-implemented method may further comprise, when the user provisionally selects a template (using the GUI), displaying, using the GUI, a preview of the (provisionally selected) template.

The computer-implemented method may further comprise displaying, using the GUI, drop-down boxes (menus) to receive input from the user to select/edit: a form of the nodes (optionally for each family member role); and/or a form of the edges (optionally for each type of relationship); and/or a configuration in which (or how) information will be displayed for each node; and/or a template.

The computer-implemented method may further comprise receiving the user input in the form of the user selecting, using the GUI, at least one node and/or edge of the genogram to edit.

The computer-implemented method may further comprise recommending, using the GUI, to the user the replacement of the first entity with the second entity in the extracted information when the further unstructured (medical history) data is more reliable than the unstructured (medical history) data, and recommending, using the GUI, to the user updating the genogram by using the second entity instead of the first entity.

The computer-implemented method may further comprise outputting a diagnosis for the target patient based on the genogram.

The computer-implemented method may further comprise outputting a list of high-risk diseases or conditions for the target patient based on the genogram.

The computer-implemented method may further comprise receiving at least one physiological measurement of the target patient and outputting a diagnosis for the target patient based on the at least one physiological measurement and the genogram.

The computer-implemented method may further comprise receiving at least one symptom of the target patient and outputting a diagnosis for the target patient based on the at least one symptom and the genogram.

The computer-implemented method may further comprise receiving at least one physiological measurement of the target patient and at least one symptom of the target patient and outputting a diagnosis for the target patient based on the at least one physiological measurement, the at least one symptom, and the genogram.

The computer-implemented method may further comprise: predicting at least one genogram diagnosis, the at least one genogram diagnosis being at least one diagnosis of the target patient based on the genogram; predicting at least one physiological diagnosis, the at least one physiological diagnosis being at least one diagnosis of the target patient based on (the) at least one symptom of the target patient and/or (the) at least one physiological measurement of the target patient; and comparing the at least one genogram diagnosis with the at least one physiological diagnosis and, when at least one diagnosis among the at least one genogram diagnosis is the same as a diagnosis among the at least one physiological diagnosis, outputting the at least one diagnosis as at least one final diagnosis.

The computer-implemented method may further comprise: predicting at least one genogram diagnosis, the at least one genogram diagnosis being at least one diagnosis of the target patient based on the genogram; predicting at least one symptom diagnosis, the at least one symptom diagnosis being at least one diagnosis of the target patient based on (the) at least one symptom of the target patient; predicting at least one measurement diagnosis, the at least one measurement diagnosis being at least one diagnosis of the target patient based on (the) at least one physiological measurement of the target patient, and comparing the at least one genogram diagnosis, the at least one symptom diagnosis, and the at least one measurement diagnosis and, when at least one diagnosis among the at least one genogram diagnosis is the same as a diagnosis among the at least one symptom diagnosis and at least one diagnosis among the at least one measurement diagnosis, outputting the at least one diagnosis as at least one final diagnosis.

According to an embodiment of a second aspect there is disclosed herein a computer-implemented method comprising: predicting at least one genogram diagnosis, the at least one genogram diagnosis being at least one diagnosis of a target patient based on a genogram, (the genogram comprising a plurality of nodes respectively representing a plurality of people including the target patient and at least one family member of the target patient, wherein an edge between two (said) nodes indicates a relationship between the people represented by the two (said) nodes); predicting at least one symptom diagnosis, the at least one symptom diagnosis being at least one diagnosis of the target patient based on at least one symptom of the target patient; predicting at least one measurement diagnosis, the at least one measurement diagnosis being at least one diagnosis of the target patient based on at least one measurement of the target patient, and comparing the at least one genogram diagnosis, the at least one symptom diagnosis, and the at least one measurement diagnosis and, when at least one diagnosis among the at least one genogram diagnosis is the same as a diagnosis among the at least one symptom diagnosis and at least one diagnosis among the at least one measurement diagnosis, outputting the at least one diagnosis as at least one final diagnosis.

The at least one physiological measurement may comprise at least one of: a heart rate; a temperature; at least one electrocardiogram, ECG, measurement; at least one electromyogram, EMG, measurement; at least one galvanic skin response, GSR, sensor measurement; a measurement of sweat gland activity; at least one optical sensor measurement; a pH measurement; a blood pressure measurement; a pacemaker pulse detection measurement; a measurement of skin anomalies by light intensity; and a blood-glucose level measurement.

The computer-implemented method may further comprise receiving the at least one symptom of the target patient through speech input from the user.

The computer-implemented method may further comprise converting the speech input into text data and using Named Entity Recognition, NER, to extract the at least one symptom.

The computer-implemented method may further comprise outputting recommendations to the user based on the at least one (final) diagnosis.

The recommendations may comprise recommended medication or recommended action.

Predicting the at least one genogram diagnosis may comprise: maintaining a genogram diagnosis score for each of a plurality of possible diagnoses; adjusting at least one of the genogram diagnosis scores based on a set of genogram diagnosis rules and based on the genogram; and determining at least one of the possible diagnoses having the highest genogram diagnosis score as the at least one genogram diagnosis.

The set of genogram diagnosis rules may comprise adding to a diagnosis score for at least one disease and/or condition if a family member of the target patient has or has had that at least one disease and/or condition.

Each genogram diagnosis score may comprise: a primary score based on information in the genogram about at least one sibling of the target patient; a secondary score based on information in the genogram about at least one parent of the target patient; and a tertiary score based on information in the genogram about at least one grandparent of the target patient and/or at least one sibling of a parent of the target patient and/or at least one cousin of the target patient.

For each genogram diagnosis score, the primary, secondary, and tertiary scores may be summed together with primary, secondary, and tertiary weightings, respectively, to generate the genogram diagnosis score.

The genogram diagnosis rules may comprise: if a sibling of the target patient has or has had at least one disease and/or condition, adding to a primary diagnosis score for that at least one disease and/or condition; if a parent of the target patient has or has had at least one disease and/or condition, adding to a secondary diagnosis score for that at least one disease and/or condition; if a grandparent of the target patient and/or a sibling of a parent of the target patient and/or a cousin of the target patient has or has had at least one disease and/or condition, adding to a tertiary diagnosis score for that at least one disease and/or condition.

Adding to a primary diagnosis score for at least one disease and/or condition if a sibling of the target patient has or has had that at least one disease and/or condition may comprise: adding a contribution to the primary diagnosis score for that at least one disease and/or condition for each sibling of the target patient who has or has had that at least one disease and/or condition.

Adding to a secondary diagnosis score for at least one disease and/or condition if a parent of the target patient has or has had that at least one disease and/or condition may comprise: adding a contribution to the secondary diagnosis score for that at least one disease and/or condition for each parent of the target patient who has or has had that at least one disease and/or condition.

Adding to a tertiary diagnosis score for at least one disease and/or condition if a parent of the target patient has or has had that at least one disease and/or condition may comprise: adding a contribution to the tertiary diagnosis score for that at least one disease and/or condition for each grandparent of the target patient and sibling of a parent of the target patient cousin of the target patient who has or has had that at least one disease and/or condition.

Predicting the at least one symptom diagnosis may comprise: maintaining a symptom diagnosis score for each of a plurality of possible diagnoses; adjusting at least one of the symptom diagnosis scores based on a set of symptom diagnosis rules and based on the at least one symptom of the target patient; and determining at least one of the possible diagnoses having the highest symptom diagnosis score as the at least one symptom diagnosis.

Predicting the at least one physiological diagnosis may comprise: maintaining a physiological diagnosis score for each of a plurality of possible diagnoses; adjusting at least one of the physiological diagnosis scores based on a set of physiological diagnosis rules and based on the at least one physiological measurement of the target patient; and determining at least one of the possible diagnoses having the highest physiological diagnosis score as the at least one physiological diagnosis.

Determining the at least one genogram diagnosis may comprise disregarding a diagnosis if its genogram diagnosis score is below a genogram diagnosis score threshold; and/or determining the at least one symptom diagnosis may comprise disregarding a diagnosis if its symptom diagnosis score is below a symptom diagnosis score threshold; and/or determining the at least one physiological diagnosis may comprise disregarding a diagnosis if its physiological diagnosis score is below a physiological diagnosis score threshold.

The set of symptom diagnosis rules may comprise adding to a said symptom diagnosis score for a diagnosis of heart failure if the at least one symptom comprises: tiredness; and/or chest pain; and/or bad breath; and/or tachycardia; and/or nausea; and/or an accelerated heartrate; and/or a persistent cough.

The set of symptom diagnosis rules may comprise: if the at least one symptom comprises tiredness, adding to at least one symptom diagnosis score for any of heart failure and congestive heart failure; and/or if the at least one symptom comprises chest pain, adding to at least one symptom diagnosis score for any of heart failure, congestive heart failure, muscle strain, gastroesophageal reflux disease, asthma, costochondritis, and valvular heart disease; and/or if the at least one symptom comprises bad breath, adding to at least one symptom diagnosis score for any of heart failure, congestive heart failure, pneumonia, coronavirus, asthma, emphysema, and valvular heart disease; and/or if the at least one symptom comprises tachycardia, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises nausea, adding to at least one symptom diagnosis score for any of heart failure and congestive heart failure; and/or if the at least one symptom comprises an accelerated heartrate, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises a persistent cough, adding to at least one symptom diagnosis score for any of heart failure, congestive heart failure, chronic obstructive pulmonary disease, gastroesophageal reflux disease, asthma, and coronavirus.

The set of symptom diagnosis rules may comprise: if the at least one symptom comprises tiredness, adding to at least one symptom diagnosis score for any of heart failure and congestive heart failure; and/or if the at least one symptom comprises chest pain, adding to at least one symptom diagnosis score for any of heart failure, congestive heart failure, and valvular heart disease; and/or if the at least one symptom comprises bad breath, adding to at least one symptom diagnosis score for any of heart failure, congestive heart failure, and valvular heart disease; and/or if the at least one symptom comprises tachycardia, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises nausea, adding to at least one symptom diagnosis score for any of heart failure and congestive heart failure; and/or if the at least one symptom comprises an accelerated heartrate, adding to at least one symptom diagnosis score for any of heart failure, congestive heart failure, coronary artery disease, heart attack, congenital heart defects, valvular heart disease, hypertension, and cardiomyopathies; and/or if the at least one symptom comprises a persistent cough, adding to at least one symptom diagnosis score for any of heart failure and congestive heart failure.

The set of physiological diagnosis rules may comprise adding to a said physiological diagnosis score for a diagnosis of heart failure if the at least one symptom comprises: P-wave=='enlarged'; and/or QRS_duration=='prolonged'; and/or left_axis_deviation==True; and/or time_to_ID > 50; and/or CSA_muscle=='reduction'; and/or muscle_strength=='reduction'; and/or muscle_fatigability=='high'; and/or submaximal_contraction=='low'; and/or breathing_rate=='low'; and/or heart rate=='high'; and/or GSR < 15; and/or blood_pressure_fluid=='high'.

The set of physiological diagnosis rules may comprise: if the at least one physiological measurement comprises an enlarged P-wave (detected from an ECG), adding to at least one physiological diagnosis score for any of atrial enlargement, chronic respiratory disease, heart failure, congestive heart failure, cardiomyopathies, congenital heart defects, and valvular heart disease; and/or if the at least one physiological measurement comprises a prolonged QRS duration (detected from an ECG), adding to at least one physiological diagnosis score for any of heart failure, congestive heart failure, atrial fibrillation, coronary disease, arrhythmia, ischemic heart disease, and valvular heart disease; and/or if the at least one physiological measurement comprises a left axis deviation (detected from an ECG), adding to at least one physiological diagnosis score for any of ischemic heart disease, heart failure, congestive heart failure, and congenital heart defects; if the at least one physiological measurement comprises a time to ID of more than 50 seconds (detected from an ECG), adding to at least one physiological diagnosis score for any of heart failure and coronary heart disease; and/or if the at least one physiological measurement comprises a cross-sectional area muscle reduction (detected from an EMG), adding to at least one physiological diagnosis score for any of heart failure, congestive heart failure, and skeletal muscle atrophy; and/or if the at least one physiological measurement comprises a muscle strength reduction (detected from an EMG), adding to at least one physiological diagnosis score for any of heart failure, congestive heart failure, skeletal muscle atrophy, and muscular dystrophy; and/or if the at least one physiological measurement comprises a high muscle fatigability (detected from an EMG), adding to at least one physiological diagnosis score for any of heart failure, congestive heart failure, skeletal muscle atrophy, and muscular dystrophy; and/or if the at least one physiological measurement comprises a low submaximal contraction (detected from an EMG), adding to at least one physiological diagnosis score for any of heart failure, congestive heart failure, skeletal muscle atrophy, muscular dystrophy, and neurological disorders; if the at least one physiological measurement comprises a low breathing rate (detected from a pacemaker pulse detection measurement), adding to at least one physiological diagnosis score for any of heart failure, congestive heart failure, bradypnea, lung disorders, chronic bronchitis, and pneumonia; and/or if the at least one physiological measurement comprises a high heart rate (detected from a pacemaker pulse detection measurement), adding to at least one physiological diagnosis score for any of heart failure, congestive heart failure, valvular heart disease, coronary heart disease, and cardiomyopathies; and/or if the at least one physiological measurement comprises an enlarged heart rate (detected from a pacemaker pulse detection measurement), adding to at least one physiological diagnosis score for any of heart failure, congestive heart failure, stroke, and cardiomyopathies; and/or if the at least one physiological measurement comprises a skin conductivity of less than 15 kohms (detected from a GSR sensor measurement), adding to at least one physiological diagnosis score for any of depression and anxiety; and/or if the at least one physiological measurement comprises a high blood pressure, adding to at least one physiological diagnosis score for any of heart failure, congestive heart failure, heart attack, stroke, arterial disease, and aortic aneurysm.

The set of physiological diagnosis rules may comprise: if the at least one physiological measurement comprises an enlarged P-wave (detected from an ECG), adding to at least one physiological diagnosis score for any of atrial enlargement, heart failure, congestive heart failure, cardiomyopathies, congenital heart defects, and valvular heart disease; and/or if the at least one physiological measurement comprises a prolonged QRS duration (detected from an ECG), adding to at least one physiological diagnosis score for any of heart failure, congestive heart failure, atrial fibrillation, coronary disease, arrhythmia, ischemic heart disease, and valvular heart disease; and/or if the at least one physiological measurement comprises a left axis deviation (detected from an ECG), adding to at least one physiological diagnosis score for any of ischemic heart disease, heart failure, congestive heart failure, and congenital heart defects; if the at least one physiological measurement comprises a time to ID of more than 50 seconds (detected from an ECG), adding to at least one physiological diagnosis score for any of heart failure and coronary heart disease; and/or if the at least one physiological measurement comprises a cross-sectional area muscle reduction (detected from an EMG), adding to at least one physiological diagnosis score for any of heart failure and congestive heart failure; and/or if the at least one physiological measurement comprises a muscle strength reduction (detected from an EMG), adding to at least one physiological diagnosis score for any of heart failure and congestive heart failure; and/or if the at least one physiological measurement comprises a high muscle fatigability (detected from an EMG), adding to at least one physiological diagnosis score for any of heart failure and congestive heart failure; and/or if the at least one physiological measurement comprises a low submaximal contraction (detected from an EMG), adding to at least one physiological diagnosis score for any of heart failure and congestive heart failure; if the at least one physiological measurement comprises a low breathing rate (detected from a pacemaker pulse detection measurement), adding to at least one physiological diagnosis score for any of heart failure and congestive heart failure; and/or if the at least one physiological measurement comprises a high heart rate (detected from a pacemaker pulse detection measurement), adding to at least one physiological diagnosis score for any of heart failure, congestive heart failure, valvular heart disease, coronary heart disease, and cardiomyopathies; and/or if the at least one physiological measurement comprises an enlarged heart rate (detected from a pacemaker pulse detection measurement), adding to at least one physiological diagnosis score for any of heart failure, congestive heart failure, stroke, and cardiomyopathies; and/or if the at least one physiological measurement comprises a high blood pressure, adding to at least one physiological diagnosis score for any of heart failure, congestive heart failure, heart attack, stroke, arterial disease, and aortic aneurysm.

The set of symptom diagnosis rules may comprise adding to a said symptom diagnosis score for a diagnosis of depression if the at least one symptom comprises: tiredness; and/or apathy; and/or lack of enthusiasm; and/or sadness; and/or reduced appetite.

The set of symptom diagnosis rules may comprise: if the at least one symptom comprises tiredness, adding to said symptom diagnosis scores for any of depression and anxiety; and/or if the at least one symptom comprises apathy, adding to said symptom diagnosis scores for any of depression and major depressive disorder; and/or if the at least one symptom comprises lack of enthusiasm, adding to said symptom diagnosis scores for any of depression, major depressive disorder, and suicidal ideation; and/or if the at least one symptom comprises sadness, adding to said symptom diagnosis scores for any of depression, major depressive disorder, and suicidal ideation; and/or if the at least one symptom comprises stress and/or anxiety, adding to said symptom diagnosis scores for anxiety; and/or if the at least one symptom comprises reduced appetite, adding to said symptom diagnosis scores for any of depression, major depressive disorder, anorexia, nutrition disorder.

The set of physiological diagnosis rules may comprise adding to a said physiological diagnosis score for a diagnosis of depression (and anxiety) if the at least one symptom comprises: an irregular heartrate; and/or high GSR fluctuation; and/or high blood pressure; and/or low pH levels; and/or high blood-glucose level fluctuation

According to an embodiment of a third aspect there is disclosed herein a wearable device comprising: at least one processor configured to carry out the method of any of the first or second aspects; at least one sensor configured to obtain the at least one physiological measurement from the target patient; and/or a microphone configured to record (the) speech input from the user and obtain (the) at least one symptom of the target patient.

The wearable device may comprise a memory to store the genogram.

According to an embodiment of a fourth aspect there is disclosed herein a system comprising: an apparatus configured to carry out the method of the first aspect; and the wearable device of the third aspect, further comprising a communication unit configured to receive the genogram from the apparatus, wherein the apparatus comprises a communication unit configured to transmit the genogram to the wearable device.

According to an embodiment of a fifth aspect there is disclosed herein a system comprising: an apparatus configured to carry out the method of the first aspect or the second aspect; and a wearable device comprising: at least one sensor configured to obtain the at least one physiological measurement from the target patient; a microphone configured to record the speech input from the user; and a communication unit configured to transmit the speech input and the at least one physiological measurement to the apparatus.

The at least one senor may comprise at least one of: an electrocardiogram, ECG, sensor; a thermometer; an electromyogram, EMG, sensor; a pacemaker pulse detection sensor; a bioimpedance sensor; a galvanic skin response, GSR, sensor; and an optical sensor configured to measure blood pressure, a pH level of the blood, skin anomalies by light intensity, and/or blood-glucose levels.

The wearable device may comprise a speaker to output the at least one diagnosis.

The apparatus may comprise a server.

According to an embodiment of a sixth aspect there is disclosed herein a computer program which, when run on a computer, causes the computer to carry out a method comprising: using a trained (deep-learning) family medical history, FMH, model trained to use Named Entity Recognition, NER, and Relation Extraction, RE, to extract entities and relations between the entities from unstructured data, extracting information from unstructured (medical history) data for at least one family member of a target patient, the information including: a family member role (keyword) indicating a relationship of the family member with the target patient; a status of the family member (including at least one of alive, dead, and healthy); and at least one observation of the family member; transforming the extracted information into a genogram (comprising a plurality of nodes respectively representing a plurality of people including the target patient and the at least one family member, wherein an edge between two (said) nodes indicates a relationship between the people represented by the two (said) nodes).

According to an embodiment of a seventh aspect there is disclosed herein an information processing apparatus comprising a memory and a processor connected to the memory, wherein the processor is configured to: using a trained (deep-learning) family medical history, FMH, model trained to use Named Entity Recognition, NER, and Relation Extraction, RE, to extract entities and relations between the entities from unstructured data, extract information from unstructured (medical history) data for at least one family member of a target patient, the information including: a family member role (keyword) indicating a relationship of the family member with the target patient; a status of the family member (including at least one of alive, dead, and healthy); and at least one observation of the family member; transform the extracted information into a genogram (comprising a plurality of nodes respectively representing a plurality of people including the target patient and the at least one family member, wherein an edge between two (said) nodes indicates a relationship between the people represented by the two (said) nodes).

Features relating to any aspect/embodiment may be applied to any other aspect/embodiment.

Reference will now be made, by way of example, to the accompanying drawings, in which:

Figure 1 is a diagram illustrating a system;
Figure 2 is a diagram illustrating a method;
Figure 3 is a diagram illustrating a method;
Figure 4 is a diagram illustrating a table;
Figure 5 is a diagram illustrating a genogram;
Figure 6 is a diagram illustrating a GUI window;
Figure 7 is a diagram illustrating a GUI window;
Figure 8 is a diagram illustrating a GUI window;
Figure 9 is a diagram illustrating a method;

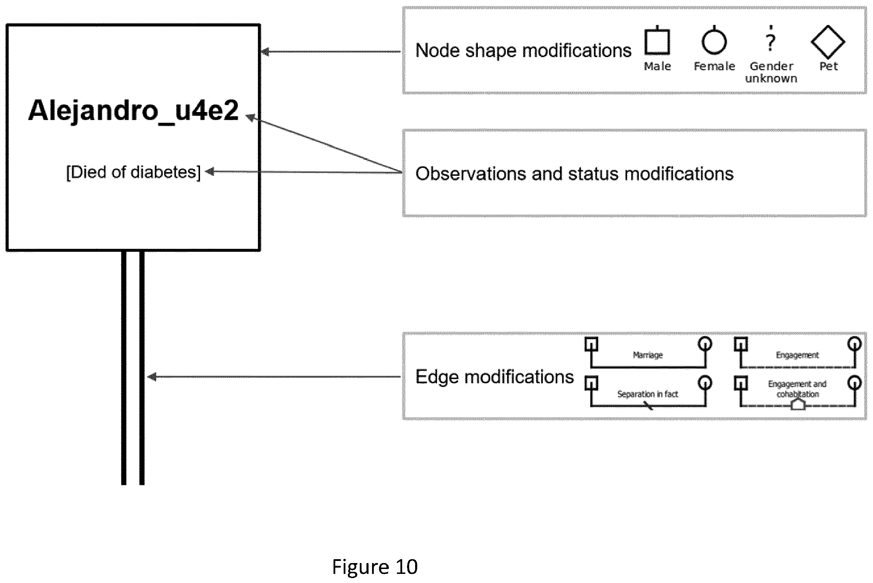

Figure 10 is a diagram illustrating a genogram;
Figure 11 is a diagram illustrating tables;
Figure 12 is a report;
Figure 13 is a diagram illustrating a GUI window;
Figure 14 is a diagram illustrating a table and a genogram;
Figure 15 is a diagram illustrating tables;
Figure 16 is a diagram illustrating tables;
Figure 17 is a diagram illustrating a table;
Figure 18 is a diagram illustrating genograms;
Figure 19 is a diagram illustrating a wearable device;
Figure 20 is a diagram illustrating tables;
Figure 21 is a diagram illustrating a genogram;
Figure 22 is a diagram illustrating tables;
Figure 23 is a diagram illustrating a genogram; and
Figure 24 is a diagram illustrating an apparatus.

The following non-exhaustive and exemplary list describes some technical terms:

- Family Health History: a record of health information about a person and their relatives. A complete record includes information from three generations of relatives, including children, siblings, parents, aunts and uncles, nieces and nephews, grandparents, and cousins.

- Preventive Medicine: a medical specialty which focuses on the health of individuals and communities. The goal of preventive medicine is to promote health and well-being and prevent disease, disability and death.

- Graphical User Interface (GUI): a form of user interface that may allow users to interact with electronic devices through graphical icons and audio indicators.

- Text Mining: the process of deriving high-quality information from text, including e.g. examining large collections of written resources in order to generate new information. The goal of text mining is to discover relevant information in text by transforming the text into data that can be used for further analysis.

- Natural language processing (NLP): a component of Text Mining that performs a special kind of linguistic analysis that essentially helps a machine "read" text. NLP uses a variety of methodologies to decipher the ambiguities in human language. NLP is a field of computer science, artificial intelligence, and computational linguistics concerned with the interactions between computers and human (natural) languages.

- Machine Learning (ML): the subfield of computer science that may be considered to give computers the ability to learn without being explicitly programmed. It explores the study and construction of algorithms that can learn from and make predictions on data.

- Deep Learning (DL): part of a broader family of machine learning methods based on learning data representations, as opposed to task-specific algorithms.

- Artificial Neural Network/Neural Network (ANN/NN): an information processing paradigm that is inspired by the way biological nervous system, such as the brain, process information. It can be used to extract patterns and detect trends that are too complex to be noticed by either humans or other computer techniques.

- Named Entity Recognition (NER): a subtask of information extraction that seeks to locate and classify named entities mentioned in unstructured text into categories such as family members, conditions, etc.

- Relation Extraction (RE): the task of extracting semantic relationships from a text. Extracted relationships usually occur between two or more entities of a certain type (e.g. Person, Organisation, Location) and fall into a number of semantic categories (e.g. married to, employed by, lives in).

- Language model: a language model is a probability distribution over sequences of words belonging to a specific language. Estimating the relative likelihood of different phrases is useful in many natural language processing applications, especially those that generate text as an output. Language modelling is used in speech recognition, machine translation, part-of-speech tagging, parsing, Optical Character Recognition (OCR), handwriting recognition, information retrieval and other applications.

Fine-tuning Language models: the transfer learning of pre-trained language models to solve specific NLP tasks. This process consists of further training the language model using the same algorithm used in the pre-training stage but over the actual dataset and task that one is interested in.

A genetic pedigree or genogram captures relationships and detailed information about the health and medical history of multiple generations in a family. This knowledge is relevant for detecting patterns of inherited medical conditions and may aid in making clinical decisions dependant on such patterns. If the relationships and health information appear in a diagram with standard symbols and terminology, a genogram may be referred to as a pedigree (Bennett, Robin L., et al. "Standardized human pedigree nomenclature: update and assessment of the recommendations of the National Society of Genetic Counselors" Journal of genetic counseling 17.5 (2008): 424-433); (Rich, Eugene C., et al. "Reconsidering the family history in primary care" Journal of general internal medicine 19.3 (2004): 273-280).

Genograms serve as a visual representation of patterns of inheritance and potentially shared environmental risk factors. The interpretation of a genogram may help identify individuals who may be at increased risk of developing of having various health problems. Genograms may be useful in preventive medicine.

Embodiments may provide a system and method for automatic information extraction and generation of genograms and genogram interfaces.

Embodiments may be concerned with the following technical considerations:

- Genogram creation tools: Conventional tools are "drag & drop" tools (requiring e.g. input from a mouse) to build genograms manually from scratch. Embodiments may focus on automatic genogram generation from clinical documents.

- Genogram representation & scalability including different options for defining and updating genograms.

Cross-validation of a genogram with different data, and collaborative tools in genogram creation, e.g. guided-assistance. In some embodiments, cross-validation refers to the comparison of new data (e.g. medical (family history) information from relatives' clinical reports) with the data used to generate the genogram (e.g. the current patient's report) to detect inconsistencies. The new data may be another (newer or older) report for the patient.

Embodiments may relate to a system for automatic generation of genograms from clinical documents.

Example arrangements may include the following features:

  ▪ A module to generate automatically the diagram of genetic pedigree (genogram) with the information extracted automatically from discharge summaries.
  ▪ An interface to edit the genogram (e.g. by a user and/or by healthcare professionals) for example to correct, extend, improve, etc., with automatic recommendations for changes and automatic cross-validation with other data.

- A module For genogram representation (with standard templates or ad-hoc templates).

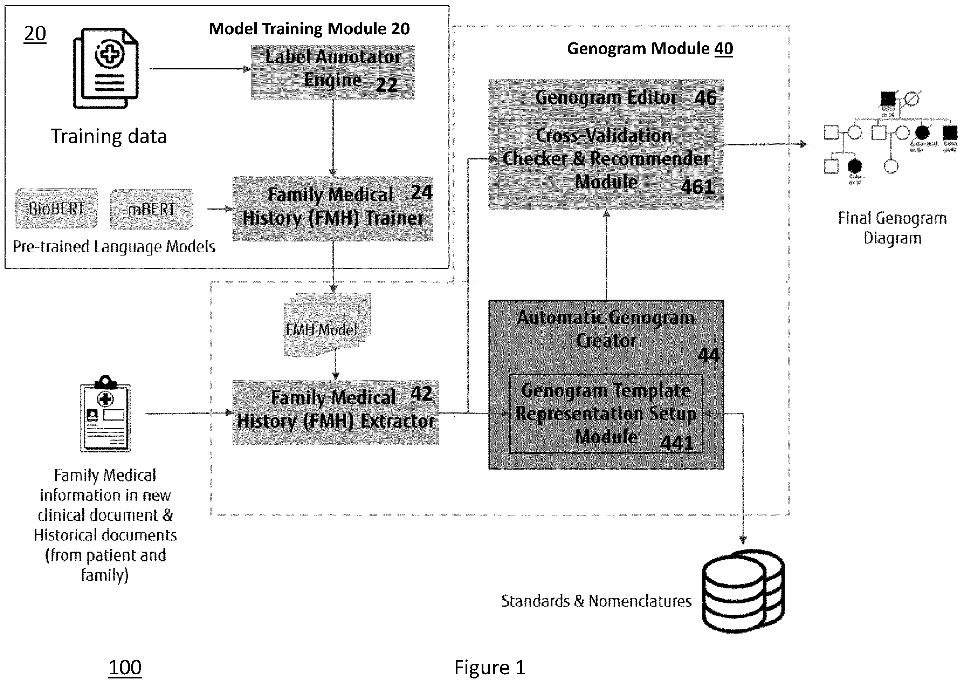

Figure 1 is a diagram illustrating a system 100 for automatic genogram creation. The system 100 comprises a model training module 20 and a genogram module 40.

Model Training Module 20

The model training module 20 may execute its function only once during a workflow. For example, multiple different genograms may be created after the model training module 40 has executed its function once.

The model training module 20 is in charge of the learning process in the creation of a Family Medical History (FMH) model. The FMH model is able to extract relevant entities and their relationships from text fragments of clinical documents, and is described in more detail below.

The model training module 20 receives the following Inputs: unstructured (medical history) data, i.e. text fragments of clinical documents (including family medical information); and pre-trained language models, such as multilingual BERT (Bidirectional Encoder Representations from Transformers), abbreviated as mBERT,, described at https://github.com/google-research/bert/blob/master/multilingual.md, or BioBERT (an implementation of BERT that has been trained on different combinations of biomedical domain corpora; in other words, BioBERT is a domain-specific language representation model pre-trained on large-scale biomedical corpora), described at https://arxiv.org/abs/1901.08746 and https://github.com/naver/biobert-pretrained, for fine-tuning the tasks of NER and Relation Extraction to be performed by the FMH model during a deep learning process (described in relation to the FMH trainer below).

The model training module 20 comprises a label annotator engine 22 and an FMH trainer 24. The label annotator engine 22 implements a rule-based algorithm to extract family medical information and create annotated datasets with this information to be used in the learning process (described in relation to the FMH trainer below). The label annotator engine 22 may be a module.

The FMH trainer 24 is in charge of implementing a deep learning process through state-of-the-art techniques and third-party tools, using the annotated datasets created with the label annotator engine 22 to train the FMH model to generate a trained FMH model.

The output of the FMH trainer 24, and of the model training module 20 in general, is the trained FMH model which is capable of extracting family medical information from new unstructured clinical documents, i.e. the FMH trainer 24 trains the FMH model to use Named Entity Recognition (NER) and Relation Extraction (RE) to extract entities and relations between the entities from unstructured (medical history) data.

Genogram Module 40

The genogram module 40 includes modules concerned with the genogram creation and editing. The genogram module 40 comprises an FMH extractor 42, an automatic genogram creator 44, and a genogram editor 46.

The genogram module 40 receives the following inputs: The FMH Model and unstructured (medical history) data, e.g. a patient clinical document about a target patient including family medical information (medical information about at least one family member of the patient) and/or historical clinical documents with further information about the target patient and/or their family members.

Automatic Genogram creation from unstructured texts

The FMH Extractor 42 exploits/uses the trained FMH Model to extract entities and their relationships from the unstructured (medical history) data, i.e. to extract information from the unstructured data for at least one family member of the target patient. The FMH extractor 42 exports these results (the information) to the other modules of the genogram module 40.

The Automatic Genogram Creator 44 automatically creates the genogram from the textual information extracted by the FMH extractor 42 from e.g. an input clinical report. The automatic genogram creator 44 includes a sub-module for defining the genogram representation: a genogram template representation setup module 441 (may be referred to as template module 441 for convenience).

The genogram template representation setup module 441 is a sub-module that implements functionalities for new definitions of and customization of form-based templates to create standard or ad-hoc genogram visual representations. The genogram template representation setup module 441 and its functionalities are described in more detail below.

The genogram editor 46 edits procedures of the first genogram version created by the automatic genogram creator 44, and outputs an enhanced final version of the genogram. The genogram editor 46 includes a cross-validation checker and recommender module 461 which executes operations for cross-validation of information and recommendations (e.g. via interfaces) for example to maintain scalable and robust updates to the genogram.

The Cross-Validation Checker & Recommender Module 461 implements functionalities to validate data among family members from historical clinical reports automatically through NLP techniques, and to generate recommendations (e.g. via visual recommendation interfaces) to assist the user in the genogram editing. The output of the genogram module 40 is the final genogram. Components of the system 100 are described in more detail below.

Label Annotator Engine 22

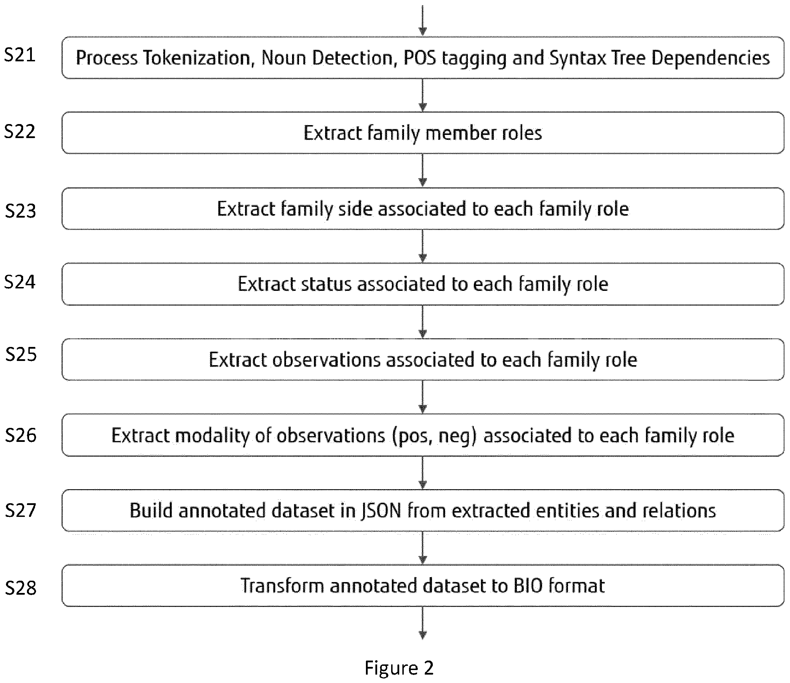

Figure 2 illustrates a method according to a running example executed by the label annotator engine 22. The label annotator engine 22 receives as an input text fragments of training clinical documents with Family Medical information included (unstructured data). As mentioned above, the label annotator engine 22 implements a rule-based algorithm to extract family medical information from unstructured texts and create annotated datasets to be used in training the FMH model. The rule-based algorithm is illustrated in the method of Figure 2.

Step S21

Step S21 comprises receiving unstructured (medical history) data; dividing the unstructured (medical history) data into sentences and tokenizing each sentence to extract tokens, the tokens comprising family member role keywords and/or status keywords; and performing noun detection among the tokens (and part of speech (PoS) extraction on the sentences). Step S21 may also comprise generating syntax tree dependencies for each sentence to analyse relationships between detected nouns/entities.

That is, step S21 comprises Process Tokenization, Noun Detection, POS tagging and Syntax Tree Dependencies. As the unstructured (medical history) data, Family History information in discharge summaries is collected from public repositories. The information is filtered by section names of the document(s) to get the appropriate data. Cleaning and text processing tasks may be carried out on the collected information/data. The training data may relate to a target patient. The target patient may be the same as or different from a target patient to which unstructured data (not for training, described later) relates. That is, a genogram may be created as described later for a target patient which is the same as or different from the target patient of the training data. The training data may relate to a plurality of different families and therefore different target patients.

For the tokenisation, third-party libraries may be used to divide the Family History texts into sentences and tokenize each sentence. Tokenization is a way of separating a piece of text into smaller units called tokens. Here, tokens can be either words, characters, or subwords. Noun detection and Part of Speech (POS) extraction may be carried out over the sentences to get relevant data.

Syntax tree dependencies of sentences are generated to analyse relationships between entities. For the next steps of the method, information processed/extracted in step S21 will aid in the extraction of entities and relations about Family Medical History.

For instance, for the sentence, "Mother and grandmother died of diabetes.", the information extracted by tokenization is, following the notation format of

```
token --> syntax tree tag --> POS tag:
Mother --> nsubj --> NOUN
and --> cc --> CCONJ
grandmother --> conj --> NOUN
died --> ROOT --> VERB
of --> prep --> ADP
diabetes --> pobj --> NOUN
. --> punct --> PUNCT
```

The meanings of the POS tags are as follows: NOUN indicates a noun, VERB indicates a verb, ADP indicates an adposition, PUNCT indicates punctuation, and CCONJ indicates a coordinating conjunction. Further POS tags that may be used may be found at https://universaldependencies.org/u/pos/all.html.

The meanings of the syntax tree tags (grammatical tags of the syntax tree dependencies) are as follows: nsubj=subject (noun), ROOT=main verb, prep=preposition, punct=punctuation, cc=coordination, pobj=object of preposition. Further syntax tree tags that may be used may be found at https://downloads.cs.stanford.edu/nlp/software/dependencies manual.pdf.

Step S21 may be considered to comprise tokenisation optionally followed by noun detection, optionally followed by POS analysis and/or syntax tree tag analysis. Noun detection may be considered to comprise using the POS tags and/or syntax tree tags. Nouns may be detected by filtering tokens to find tokens comprising an element with the POS tag NOUN.

Step S22

Step S22 comprises identifying family member role keywords among the detected nouns by comparing at least some of the detected nouns with a dictionary of family member role keywords.

That is, step S22 comprises extracting family member roles. An initial dictionary may be defined with 31 basic types (keywords) for family roles. Family roles include entities such as Father, Mother, etc. From such initial set, synsets (i.e. a set of one or more synonyms that share a common meaning) may be searched e.g. in the WordNet (http://compling.hss.ntu.edu.sg/omw/) resource (with multilingual capabilities), to construct an extended dictionary of family roles (keywords) for the desired language (e.g. English). Using this particular method, 25 extended entities are obtained, making a total number of 56 family role types (keywords) in the dictionary.

In order to return a list of family roles (keywords) from the Family History texts the nouns detected in the sentences in step S22 may be filtered using several parameters of those nouns, and then the (filtered) nouns are compared with the dictionary to match potential family roles. The resultant entities matched are outputted to the next step.

Step S23

Step S23 comprises extracting a family side associated with each family role. The keywords 'maternal' or 'paternal' are searched for. Such keywords are extracted in the tokenisation together with the associated family role keyword as a (unique) token. Therefore, it is checked whether those keywords (maternal or paternal) are present in each (noun) token including a family role (keyword). If so, the family side keyword will be associated with the specific family role keyword in a list. The complete processed list is outputted to the next step.

Step S24

Step S24 comprises, for any token comprising a family member role keyword and not a status keyword, the family member role keyword being a first family member role keyword, implementing a first status search comprising: analyzing tokens occurring in the unstructured training (medical history) data between the appearance of the first family member role keyword and the next appearance of a family member role keyword to identify a status keyword among those tokens, wherein the group including the first family member role keyword includes the identified status keyword.

Step S24 further comprises, for any token comprising a family member role keyword and not a status keyword which has not been extracted as a group comprising a status keyword after the first status search, implementing a second status search comprising: identifying a second family member role keyword occurring in the unstructured training (medical history) data before the appearance of the first family member role keyword and separated from the first family member role keyword by a conjunctive element (e.g. being a comma or the word "and"), wherein the group comprising the second family member role keyword includes the status keyword which is also included in the group comprising the first family member role keyword of the first status search.

That is, step S24 comprises extracting a status associated with each family role (keyword). A dictionary may be defined with root terms of a status such as died, alive, healthy, live, etc. Such terms may be lemmatized in order to cover all potential verbal forms or variations appearing in the texts. If the status keyword is extracted together with the family role keyword as a (unique) token, the approach in the family side extraction is followed (that is, the status keyword in the token is associated with the family member role keyword). In some implementations, only words with the POS tag of VERB or ADJ (adjective) may be compared with the dictionary of status keywords to identify status keywords.

However, there are cases where this situation does not happen. For such cases, an iterative algorithm capable of analysing text information between appearances of family roles (keywords) in the text may be implemented. This is iterated over all the tokens of the text, and detects two continuous appearances of family roles (keywords). The tokens between such appearances are compared with the dictionary and optionally lemmatizations, looking for status keywords matches. If there is match, the status (keyword) found is associated with the first family role keyword appearing before the status keyword. This part of the iterative algorithm may be referred to as a first status search.

After the first extraction of statuses for the family roles of the list over the whole text (the first status search), another part of the iterative algorithm may be implemented to identify missing statuses due to conjunctive phrases. This may be referred to as a second status search and may be considered a second iteration of the status search. For instance, for the sentence *'His mother, father and brother are alive',* in the first extraction (first status search) the status (keyword) 'alive' is associated only to brother, but in the second iteration steps may be implemented to associate the status (keyword) 'alive with the family members (role keywords) "mother" and "father" as well. The final list of family roles with their associated status is outputted to the next step.

Step S25

Step S25 comprises identifying at least one observation associated with a family member role keyword by, the family member role keyword being a (first) subject family member role keyword: analyzing tokens occurring in the unstructured training (medical history) data between the appearance of the (first) subject family member role keyword and the next appearance of a family member role keyword and identifying at least one token among those tokens that fulfils observation criteria as at least one observation associated with the subject family member role keyword. For example, considering the syntax tree dependencies (and POS tags), to identify observations the method needs to find nouns (POS=NOUN) and filter by the syntax tag of 'pobj' (preposition of object) or 'dobj' (direct object) or 'conj' (conjunct). A token may be considered an observation only if the tag of syntax tree dependency is inside one of those values.

Step S25 may further comprise, for another family member role keyword being a second subject family member role keyword occurring in the unstructured training (medical history) data before the appearance of the first subject family member role keyword and separated from the first subject family member role keyword by a conjunctive element (being a comma or the word "and"), identifying the at least one token which was identified as the at least one observation associated with the first subject family member role keyword as at least one observation associated with the second subject family member role keyword.

That is, step S25 comprises extracting observations associated with each family role. The syntax tree parser is used to identify observations based on their dependency with the nouns detected in the sentence. A search for observations is iterated over all the detected nouns of the text. The approach followed is similar to the first status search and the second status search of step S24. The tokens (or nouns) included between two appearances of family roles (keywords) in the text are analysed. If such tokens/nouns fulfil a set of filters and dependency types (observation criteria), they are marked as observations and associated with the first family role (keyword) appearance (i.e. the family role keyword appearing before the observation). For cases of conjunctive phrases, the same methodology used in the status extraction (second status search) is used in the observation extraction. The updated list of family roles (each family role with the related observations included) is outputted to the next step.

Steps S22 to S25 (and optionally step S26) may be considered to comprise extracting at least one group comprising (or a plurality of groups each comprising) a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword. That is, each of at least one family role keyword may be extracted together with a status keyword and/or at least one observation. They may be extracted as a token.

Step S26

Step S26 comprises extracting the modality of an observation. Step S26 may be considered as an extension of step S25, so that identifying at least one observation associated with a family member role keyword includes identifying a modality of the at least one observation, and wherein the group including the family member role keyword and the at least one observation includes the modality.

That is, step S26 comprises extracting the modality of observations (pos, neg). If a family member (role keyword) in the text is described positively with the condition/disease/symptom of the observation, this may be indicated with a positive tag (pos), and if a family member (role keyword) in the text is described negatively, i.e. without, the condition/disease/symptom of the observation this may be indicated with a negative tag (neg). External models may be used to determine if an observation is affirmative (pos tag) or negative (neg tag). The list of observations may be updated with these tags associated with the observations and outputted to the next step.

Steps S21 to S26 may be considered to comprise extracting information from the unstructured training (medical history) data for at least one family member of the target patient, the information comprising: a family member role (keyword) indicating a relationship of the family member with the target patient; a status of the family member (including at least one of a group comprising alive, dead, and healthy); and at least one observation of the family member.

Steps S21 to S26 may be considered to comprise dividing the unstructured training (medical history) data into sentences and tokenizing each sentence to extract tokens; performing noun detection among the tokens; identifying family member role keywords among the detected nouns (by comparing at least some of the detected nouns with a dictionary of family member role keywords) and storing the family member role keywords as part of an annotation dataset; identifying among the tokens at least one status keyword associated with a family member role keyword (by using a dictionary of status keywords) and storing the at least one status keyword in association with the family member role keyword in the annotation dataset; identifying among the tokens at least one observation associated with a family member role keyword and storing the at least one observation in association with the family member role keyword in the annotation dataset; using the (entities and relations in the) annotation dataset to annotate the unstructured training (medical history) data; and using the annotations in the unstructured training (medical history) data as ground truth information (and using at least one trained language model,) training the family medical history model to use NER and RE to extract as entities, from the unstructured training (medical history) data, the information (entities and relations) included in the annotations.

That is, in some implementations, rather than extracting entities as a group (entities being any of family member role keywords, status keywords, observations, family side keywords, etc.) methods may be considered to comprise extracting entities and storing them in the annotation dataset in association with each other.

Step S27

Step S27 comprises using the extracted groups (annotation dataset) to annotate the unstructured training (medical history) data. That is, step S27 comprises building an annotated dataset in JSON (JavaScript Object Notation) from the extracted entities and relations, i.e. the groups of family member role keywords and statuses/observations etc. Step S27 may comprise, after processing input Family History texts with previous steps, using external libraries for writing the information extracted in JSON format. For instance, in the text example, *"Her father died of leukemia at age 53 and her aunt had leukemia at age 19. Sister died of leukemia, Brother died from HIV/AIDS",* the final output in JSON after previous steps and step S27 is:

```
"{""info"": [{""family_role"": {""name"": ""father""},
""family_status"": {""name"": ""died""},
""family_observation"": [{""name"": ""leukemia"", ""mod"": ""pos""}]}, {""family_role"":
{""name"": ""aunt""}, ""family_observation"": [{""name"": ""leukemia"", ""mod"": ""pos""}]},
{""family_role"": {""name"": ""Sister""}, ""family_status"": {""name"": ""died""},
""family_observation"": [{""name"": ""leukemia"", ""mod"":
""pos""}]}, {""family_role"":
{""name"": ""Brother""}, ""family_status"": {""name"": ""died""}, ""family_observation"":
[{""name"": ""HIV/AIDS"", ""mod"": ""pos""}]}]}"
```

To create an annotated dataset, entities (any of the information extracted above, including family member role keywords, status keywords, observations, family side keywords, observation modalities) in the unstructured training data (family history texts) may be tagged with internal tags in the JSON format (e.g. as above). An annotation tag scheme able to represent the Named Entities and their Relationships at once may be used, so that the FMH model extracts everything (all the required entities and relations) at the same time (in one go).

In the annotation tag scheme, representation of the entities (NER) may be as follows: Family member=FH_ROLE, Family side=FH_SIDE, Family status=FH_STATUS, Family observation=FH_OBS (also including a modality for each observation: FH_OBS_POS, FH_OBS_NEG). A modality may be extracted for status (FH_STATUS_POS, FH_STATUS_NEG) since for example the terms "not alive" or "not healthy" may be used. Modalities may be extracted for any of the extracted terms (family role, family side). In some implementations only a negative modality may be used and the absence of a negative modality therefore indicates a positive modality.

In the annotation tag scheme, representation of the relations (RE) may be as follows: A numeric indicator (relation) may be included at the end of a generic tag. For instance, the first appearance of a family member role keyword in a sentence (FH_ROLE_1) has a '-1' suffix. A '_1' suffix is then included in the related entities to illustrate the relation (e.g. FH_STATUS_1, FH_OBS_1, etc.). Similar suffixes may then be used for second ('_2'), third ('_3') and so on appearances of family member roles in the same sentence. This tagging scheme may also be used for nested relations, i.e. entity relations shared by various family members (e.g. FH_OBS_1_2, FH_SIDE_2_3, etc.). With this kind of representation, in a particular example of training data there are 132 tag labels, but this number may depend on the training data used.

For the text example, *"Mother and grandmother died of diabetes.",* the resultant annotated sentence following the annotation tag scheme is:

```
<FH_ROLE_1 >Mother</FH_ROLE_1> and
<FH_ROLE_2>grandmother</FH_ROLE_2>
<FH_STATUS_1><FH_STATUS_2>died</FH_STATUS_2></FH_STATUS_1> of
<FH_OBS_1><FH_OBS_2>diabetes</FH_OBS_2></FH_OBS_1 >.
```

The annotated dataset following the annotation tag scheme is outputted to the next step.

Step S28

Step S28 comprises transforming the annotated dataset to the BIO (Begin entity, Inside entity, Other) format.

That is, for the training of the FMH model (in particular the fine-tuning of the tasks of NER and Relation Extraction in the creation of the FMH model using the pre-trained language models of mBERT and BioBERT), a specific tag scheme format is required. In the BIO format, every word of an entity is tagged. The previously annotated dataset (in JSON) is transformed to the BIO format through text processing techniques, joining and cleaning terms, and/or combining tags for nested annotations. The result of this transformation on the previous example is:

| Mother | B-FH_ROLE_1 |
|---|---|
| and | O |
| grandmother | B-FH_ROLE_2 |
| died | B-FH_STATUS_1_2 |
| of | O |
| diabetes. | B-FH_OBS_1_2 |

The annotated dataset transformed to this BIO format will be the final output of the Label Annotator Engine in the running example. The BIO / IOB format (I being short for inside, O being short for outside (other), and B being short for beginning) is a tagging format for tagging tokens in a chunking task in computational linguistics. The B- prefix before a tag indicates that the tag is the beginning of a chunk, and an I- prefix before a tag indicates that the tag is inside a chunk. An O tag indicates that a token belongs to no entity/chunk. There are other formats that could be used, e.g. BIOES (BIO is the same + E- prefix before a tag indicates that the tag is the end of a chunk, and an S-prefix before a tag indicates that the chunk is single (single element=one word). BIOES is also called BILOU (L for last, same as the E, and, U for unique, same as the S).

In some implementations/examples, some method steps or parts of method steps may be omitted and/or combined and/or executed in a different order. For example steps S22-S26 may be considered as a single step comprising some or all of those steps, i.e. of extracting a group (of words/tokens/entities) comprising a family member role keyword and at least one of a status keyword associated with the family member role keyword and an observation associated with the family member role keyword, wherein for any token comprising a family member role keyword and a status keyword (according to a dictionary of status keywords), the group including the family member role keyword includes the status keyword.

Family Medical History (FMH) Trainer 24

The FMH trainer 24 trains the FMH model using the annotated unstructured training data output from the label annotator engine 22. That is, the FMH trainer 24 is in charge of implementing a deep learning process, for example through state-of-the-art techniques and third-party tools, using the annotated dataset created with the Label Annotator Engine 22. For example, the external libraries of Tensorflow (https://github.com/tensorflow/tensorflow) and Keras (https://keras.io/) for Python may be used to train the FMH model. For example, techniques described in https://keras.io/examples/nlp/ner_transformers/#build-the-ner-model-class-as-a-kerasmodel-subclass may be used.

The inputs to the FMH trainer 24 are the annotated dataset from the label annotator engine 22 and e.g. the pre-trained language models mBERT and BioBERT.

The FMH trainer 24 implements techniques for fine-tuning NER and Relation Extraction in Family Medial History data (unstructured (medical history) data) using pre-trained language models. The operations of the FMH trainer 24 include methods of preprocessing and preparation of the input annotated training dataset, conversion of texts to numerical representations, design of the neural network architecture and execution of the deep learning activity.

The output of the FMH trainer 24 is the trained Family Medical History (FMH) model which is capable of extracting family medical information from new unstructured clinical documents. This model will be used in the automatic creation of genograms.

Automatic Genogram Creator 44

The automatic genogram creator 44 is in charge of creating automatically the genogram from the textual information extracted from the unstructured data about the target patient and their family members input to the system (e.g. an input clinical report). The automatic genogram creator 44 includes the genogram template representation setup module 441.

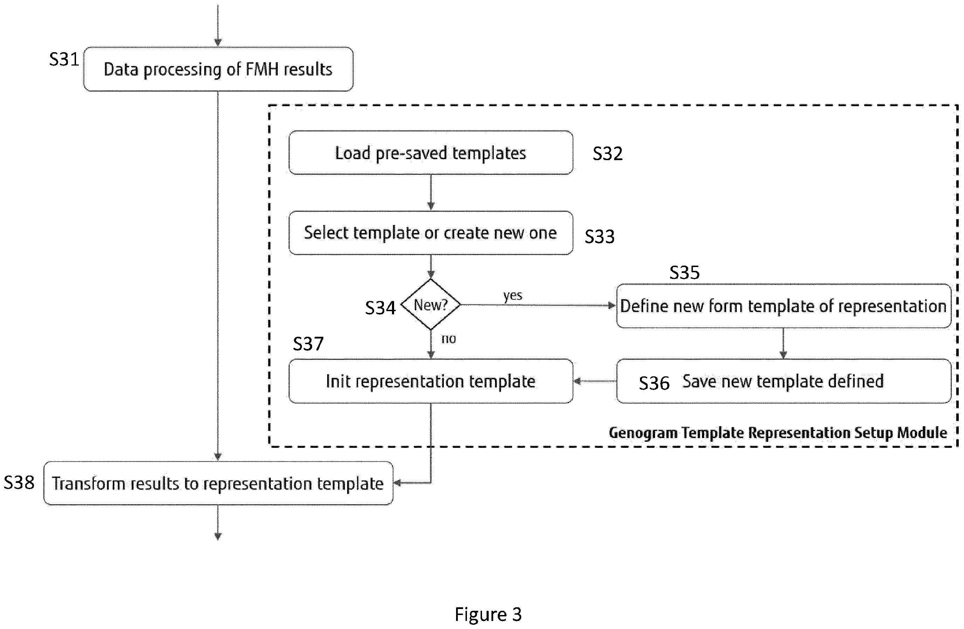

Figure 3 illustrates a method implemented by the Automatic Genogram Creator 44 and the FMH extractor 42 according to a running example. The steps performed by the genogram template representation setup module 441 are outlined in a dashed box.

Step S31

Step S31 is performed by the FMH extractor 42. Step S31 comprises converting the input data containing family history of the target patient into a general data structure which may be transformed into a Genogram.

The Family Medical History (FMH) Extractor 42 processes the input data, e.g. the target patient's clinical Family Medical report, using the trained FMH model (trained previously). The trained FMH model may be considered to perform a prediction over the patient's data, returning the results in the BIO format defined during the training of the FMH model. Next, the FMH Extractor 42 transforms the data from the BIO format/scheme to a JSON data structure containing for each identified family member of the patient:

1. Family member role: The role of the family member in relation to the target/current patient. Also, in this step, the extracted family member roles may be analysed to identify inner-relations between them - although the Genogram is based on one main patient (the target patient), it may include relations between other family members.
2. Family member status: Current status of the family member (e.g. alive, dead, healthy, etc.)
3. Family member side: Side of the family member (maternal or paternal)
4. Family member observations: A list containing details of the observation(s) (diseases, affections, conditions, symptoms, etc.) of the family member
5. Family member observation modality: Tagging the observation as positive (family member has the disease, affection, condition, symptom, etc), or negative (family member does not have the disease, affection, condition, symptom, etc).

This is a generic example of the output of the FMH Extractor 42:

```
{'info': [{'family_role': {'name': 'father', 'mod': 'pos'},
'family_status': {'name': 'died', 'mod':
'pos'}, 'family_observation': [{'name': 'a brain tumor', 'mod': 'pos'}]}, {'family_role':
{'name': 'mother', 'mod': 'pos'}, 'family_status': {'name': 'died', 'mod': 'pos'},
'family_observation': [{'name': 'diabetes complications', 'mod': 'pos'}]}], 'text': 'Father
died of a brain tumor. Mother died of diabetes complications.'}
```

This JSON output is then processed in the step S31. The family member roles are analysed to obtain inner-relations between them using a rule-based approach over known family relations. Then, the data is for example included in a table with headings: "family member 1"; "relationship"; "family member 2"; "status"; "observation".

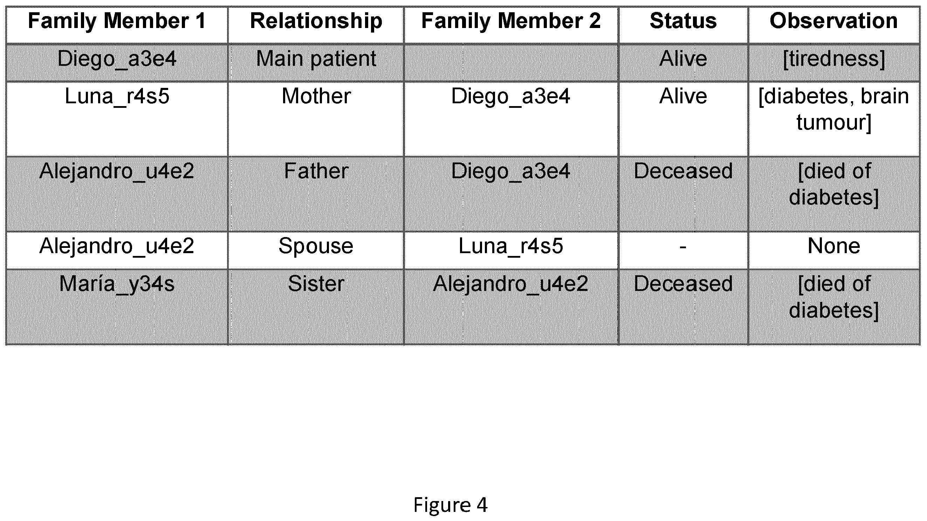

To populate this table, the JSON output is processed and special identifiers are added to names of family members to make them unique in the data to avoid different-person and same-name ambiguities. The names (Diego, Luna, etc.) included in the identifiers in Figure 4 are used to aid description. Actual names such as Diego and Luna may not be extracted from data and instead generic identifiers may be used such as "subject_1, subject_2, subject_3, etc., or person_1, person_2, person_3, etc.). The table resulting from this process is transformed into a Genogram in a subsequent step.

Figure 4 illustrates an example of a table output from step S31.

Step S38

Step S38 is carried out by the automatic genogram creator 44 and comprises transforming the extracted data (in the table format output from step S31) into a genogram. A genogram template (or genogram representation template) is used in the running example.

That is, in step S38 the table output from step S31 is transformed into the genogram. The usual graph standard may be used to define a genogram using the *graphviz* python library (https://pypi.org/project/graphviz/). In the genogram, each family member is represented by a node. The target patient is also represented by a node. Relationships between family members (and the target patient) are defined by edges between nodes. Nodes are data structures comprising any of a family member identifier, a status, a family side, and any observation(s) extracted from the unstructured data input to the genogram module 40.

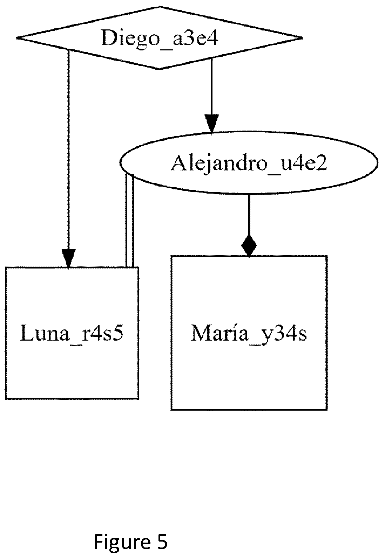

Figure 5 illustrates an example of a genogram generated based on the table in Figure 4. The information included in each node is not necessarily displayed, as shown in Figure 4.

Further visualization specifications can be defined in a JSON file containing a standard template or a manual definition of each element in the visualization. Visualization specifications are described in more detail below.

Below is an example of possible JSON element settings used to produce the genogram in Figure 5.

```
"edges":{
        ["relationship": "sibling",
        "shape": "diamond arrow"],
        ["relationship": "spouse",
        "shape": "double line"]
        },
        "nodes":
        {
        ["member": "main patient",
        "shape": "diamond"],
        ["member": "is_male",
        "shape": "ellipse"],
        ["member": "is_female",
        "shape": "square"]
        }
```

Shapes of edges and nodes can be specified and can be specified as depending on the status or observations of each family member. In this example male family members are depicted as ellipse nodes and females as squared nodes. Also in this example spouse relationships are indicated with a double line, sibling relationships with a diamond arrow edge, and mother/father relationships with a normal arrow.

Genogram Template Representation Setup Module 441 (or template module 441)

The template module 441 implements operations for definition of new templates and customization of existing templates to create standard or ad-hoc genograms. Pre-defined templates according to recognized standards may be provided e.g. for users to load directly.

As mentioned above, the dashed box in Figure 3 illustrates steps carried out by the template module 441.

Step S32

Step S32 comprises loading pre-saved templates. The pre-saved templates may be a set of standard templates. In other steps, the user may create new templates and may save them in database, for example the database in which the pre-saved templates are stored. The templates (standard, newly created, and edited (ad-hoc)) may be stored in a physical database or server or using cloud-based storage. In a running example the template information saved and processed using JSON configuration files and properties files. Each JSON configuration file is of an attribute-value type scheme. Below is an excerpt of a JSON configuration file for an example template:

```
{"template_name"="x","female_basic_form"="circle",
"female_deceased_form"="fill-black",
"female_carrier_form"="middle_point", "male_basic_form"="square",
"relation_line"="basic_line", "text_alignment_node"="bottom_form", ...}
```

Each properties file links the names of the forms in the template (i.e. the names given to the form or style of elements (nodes, edges) in the genogram) with the physical location (or address, and not necessarily physical) of the file with those shapes stored. An example of a properties file is shown below:

```
circle=/genogram/circle.eps
circle_fill-black=/genogram/circle_fill-black.eps
square_square_bracket=/genogram/square_square_bracket.eps
```

Steps S33 and S34 comprise receiving user input to select a (pre-saved/stored) template or create a new template. When the user input indicates instructions to create a new template the method proceeds to step S35. Step S35 comprises defining a new template. Step S36 comprises saving the new template. When the user input in step S34 indicates instructions not to create a new template (i.e. to select a (pre-saved/stored) template), the method proceeds to step S37. The method also proceeds to step S37 after step S36. Step S37 comprises initialising the representation template (either the selected template ("no" after step S34) or the newly created template (from step S36 and after "yes" at step S34)).

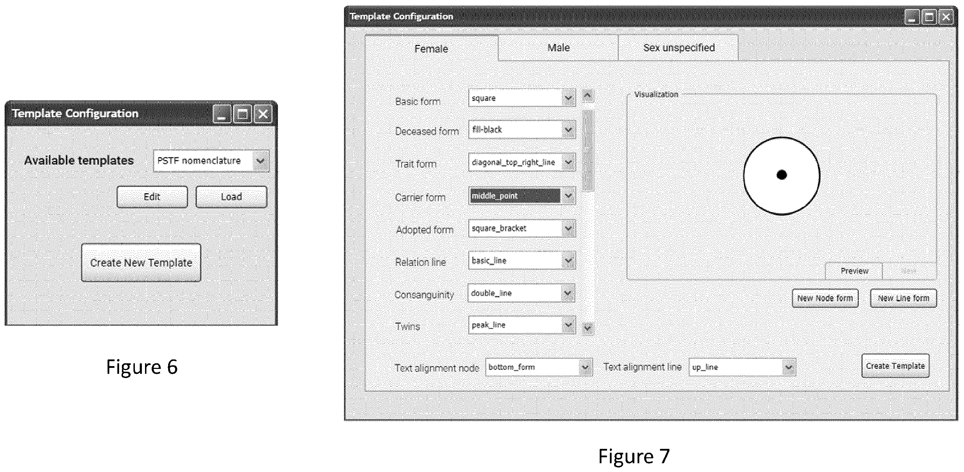

User input may be received via a graphical user interface (GUI). Figures 6 and 7 illustrate example windows that may be displayed and used to receive user input as part of the GUI in the method implemented by the template module in a running example.

The steps implemented by the template module 441 are described in more detail below according to a running example employing the GUI.

Figure 6 illustrates an example window of the GUI. The GUI and/or the template module may be considered a tool to deal with genogram representation. In steps S32 and S33 the templates' names are loaded and a combo-box/drop-down box or menu of pre-saved templates is presented to the user for them to select one of the templates by interacting with the GUI and entering user input. That is, for all JSON configuration files (each one representing one template), the attributes are loaded for each one and the name attribute of each is extracted so that it can be included in the combo-box, and so that any of the templates can be selected by the user. In Figure 6, in the combo box of 'Available templates', if user expands the list, they will see possible templates represented by the name attribute/value ({"template_name"="x"} The window illustrated in Figure 6 may be referred to as an init template configuration window.

In step S33, in the window of Figure 6 the user may select a pre-saved template in the combo-box. Then, optionally, the user may edit existing templates including creating new attributes and/or deleting existent attributes, which is described below. The user may load the selected template (by selecting "Load") and the method will proceed directly to step S37 and the selected template will be initialised. The user may create a new (ad-hoc) template by selecting the button Create New Template', which corresponds to "yes" at step S34. In this case a new GUI window may be presented to the user for implementing the step S35.

A template may be stored in an apparatus implementing the method of Figure 3 or another apparatus. A template may be stored in a server (physical or cloud-based).

Figure 7 is a diagram illustrating an example window (which may be referred to as a configuration window) to be presented to the user to receive user input to carry out step S35. The information received from the user in this window may be transcribed to a new JSON configuration file for the new template. If new shapes are created, a properties file will be also updated accordingly. In addition, the user may create new attributes or delete some of them.

Figure 7 shows an example window for the user to interact with to enter the user input to create the new template, i.e. to define the new form template of representation (step S35). The window in Figure 7 may be considered a form-based GUI to capture the user input for step S35.

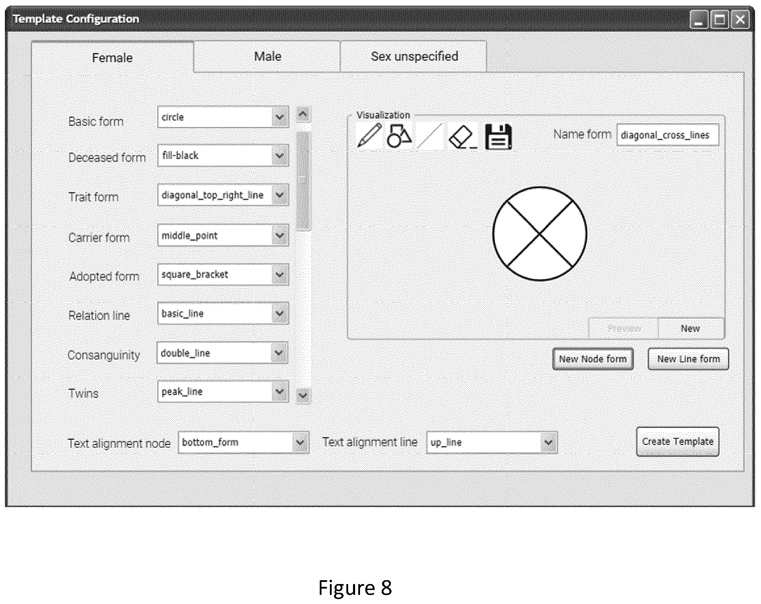

In the window/interface of Figure 7, there are combo/drop-down boxes or menus for the user to select the different forms for nodes and relations for example depending on gender (female, male, sex unspecified). The defined forms (e.g. shapes) of such nodes and relations will be the ones used in the genogram when it is created according to the new template. When the user provisionally selects an attribute/form (i.e. using the combo/drop-down boxes but before selecting the "create new template" button), a preview of the provisionally selected node(s) and/or edge(s) is displayed to the user in the Figure 7 window. The user may create new forms for nodes and edges rather than selecting from the drop-down boxes, e.g. by changing to another panel/tab ('New') when the buttons 'New Node form' or 'New Line form' are selected. The new form panel is illustrated in Figure 8.

Figure 8 illustrates an example window of the GUI for the user to interact with to create a new node form (i.e. new node form creation in the form-based GUI).

The user may draw using a "pencil" tool, input pre-defined shapes, or input an image from a file to create the new node form. The new node form is saved for example when the user selects the save button, or automatically. The shape/form will be stored in an .eps file and a properties file will be updated accordingly. The combo boxes of the interface may be updated to include the new shape/form. In the case of the shape/form created in the Figure 8 example window, a properties file may include the following:

circle_diagonal_cross_lines=/genogram/circle_diagonal_cross_lines.eps.

Several potential values of the attributes that affect and that may be selected by the user in the Figure 8 example window of the GUI are collected in the lists below:

- list_of_predefined_basic_forms= {square, rectangle, circle, triangle}
- list_of_extended_forms={diagonal_top_right_line, fill_black, middle_point, middle_vertical_line, square_bracket}
- list_of_lines= {basic_line, double_line, peak_line, discontinuous_line, triple_line, directed_line, separated_line, uneven_line}
- list_of_text_alignment_node= {bottom_form, up_form, left_form, right_form, inside_form}
- list_of_text_alignment_line= {boftom_line, up_line}

When the user selects the button Create Template', the method proceeds to the next step, step S36.

Step S36 comprises saving the new template that has been created. The data input in the form-based interface (the received user input) is processed and written in a new JSON configuration file in a running example, following the scheme of attribute-value pairs. This configuration template file may then be added to the database of templates (pre-saved templates).

Step S37 comprises initialising the template (or representation template). Using either the selected template or the newly created template, the corresponding JSON configuration file is read, linking the attribute values with corresponding paths of the properties file. In this way, the correspondent figures associated with each attribute defined in the template are obtained. The JSON configuration file and the associated figures will be sent output to the next step in the Automatic Genogram Creator 44.

In step S38, the automatic genogram creator 44 uses the template (i.e. the JSON configuration file and the associated figures) and transforms the extracted information into a genogram based on the template.

Genogram Editor 46

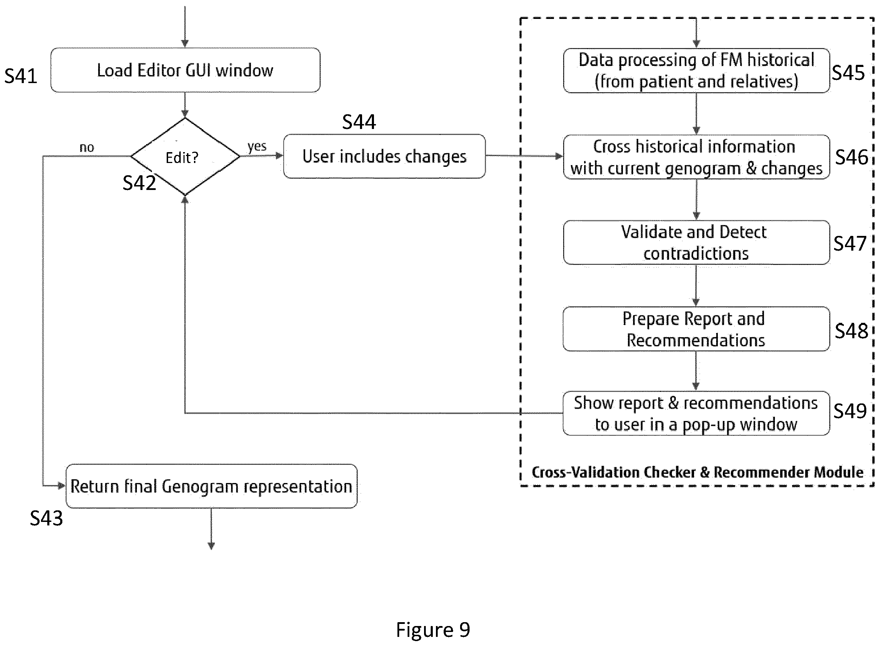

Figure 9 illustrates a method that is carried out by the genogram editor 46 according to a running example. The steps carried out by the Cross-Validation Checker & Recommender Module 461 are indicated with a dashed box. The cross-validation checker and recommender module 461 may be referred to as the checker module 461.

Step S41 comprises loading an editor GUI window. A window with the preview of the Genogram generated as described above is displayed to the user. The genogram editor enables editing of the Genogram e.g. via a GUI. The genogram may be edited/modified on two levels. The first level allows for manual modification of data and shapes/forms of edges and nodes in the graph. The second level allows for modification through recommendations and validations powered by NLP technology, implemented by the checker module 461.

Step S42 comprises allowing user input to be entered to edit the genogram.

If the user changes the genogram the method proceeds to step S44 in which user input is received to edit the genogram. At this stage, editable elements in the genogram are: Nodes, Edges and text from observations and statuses. In a GUI window, possible edge and node elements to be selected may be displayed upon the user selecting a node or an edge. This list of options may be taken from the previously defined template. Text elements may be modified within a text box for example when the user selects the text to modify.

Figure 9 illustrates possible ways in which a node and an edge may be edited by the user (in step S44).

Cross-Validation Checker & Recommender Module 461 (or checker module 461)

This (sub-)module operates to validate data among family members from historical clinical reports automatically through NLP techniques and may generate and display recommendations via interfaces (a GUI) to assist users in the genogram editing.

As mentioned above, the dashed box in Figure 7 indicates the steps carried out by the checker module 461.

Step S45 comprises data processing of further unstructured (medical history) data, which may for example be referred to as FM historical (from patient and relatives). Step S45 is the same as step S31, but using further unstructured data rather than the original unstructured data.

The original data may be for example data focused on the target patient, e.g. clinical reports and/or medical history with the target patient as the subject patient of the data. The further data may be for example data focused on at least one of the target patient's family members, e.g. clinical reports and/or medical history with the at least one family member as at least one subject patient of the data. The original data may include data focused on the at least one family member. The further data may be new data, e.g. recently produced/located clinical reports. The further data may be historical data, e.g. more medical history data about the target patient and/or at least one family member.

Considering the example in which the original unstructured data is a clinical report focused on the target patient as the subject patient and the further unstructured data is a plurality of clinical reports/medical history focused on a plurality of family members as the subject patient of each piece of further data, and/or a plurality of clinical reports focused on the target patient at different times, a difference between steps S31 and S45 is that instead having only a single table corresponding to the target patient's clinical report output from step S31, output from step S45 are multiple tables (e.g. one table for each clinical report/piece of further data analysed - the number will depend on the number of patient's clinical reports available and the number of relatives' clinical reports available). Each table output from step S45 may be marked with a unique identifier corresponding to the clinical report upon which it is based.

Step S46 comprises crossing the historical information with the current genogram including the user's changes (from step S44). "Historical information" is used in place of "further data" because step S46 in Figure 9 considers the running example in which the data and further data are defined as above.

In step S46, the information contained in the genogram (i.e. the data of the target patient and family members) is transformed into a table e.g. with headings: "family member 1"; "relationship"; "family member 2"; "status"; "observation". Alternatively, the information in the table format may be received from the output of step S31 (though if the user has changed the data in step S44 these changes would be ignored, or may need to be included).

Step S47 comprises validating the original data using the further data and detecting inconsistencies and/or contradictions. The information extracted from the data and the further data in the table format is analysed to find inconsistencies. An algorithm iterates over all tables comparing for each individual (target patient and family members) their appearances in the data and further data. If contradictions or partial information are detected, such inconsistencies are stored in a new table. The solutions to address contradictions/inconsistencies are replacement of values, joining of values or deletion of values, depending on the specific inconsistency. The table for storing inconsistency data may have the headings: ID of a first table, ID of a second table containing information that is inconsistent with the information in the first table, the family member ID for whom a contradiction/inconsistency has been found, the information field of the contradiction/inconsistency (i.e. Status or Observations), the first value of the information from the first table (i.e. the entity from the first table), and the second value of the information from the second table (i.e. the entity from the second table that contradicts/is inconsistent with the entity from the first table).

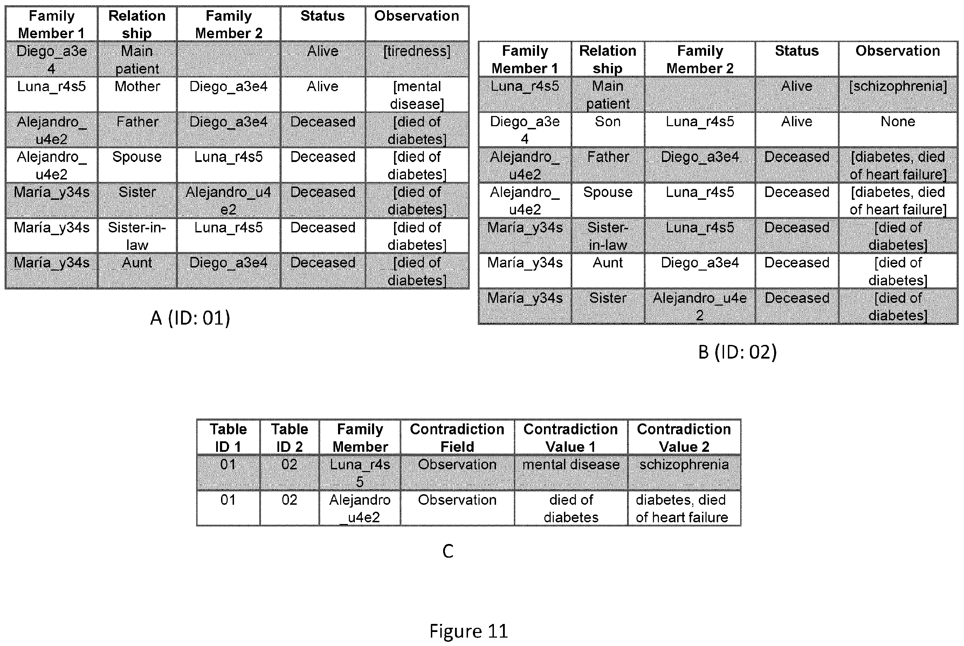

Figure 11 illustrates a first table A having an ID of 01 (for example including information extracted from the original unstructured data and therefore currently included in the genogram), a second table B having an ID of 02 (for example including information extracted from the further unstructured data), and a table of inconsistencies, C.

Step S48 comprises preparing recommendations for example in the form of a report. That is, in a running example a report is prepared highlighting the contradictions found (if any) and recommending changes that may be made in the information and so in the genogram. To prepare the report, third-party tools of text processing and rule-based algorithms may be used to retrieve the inconsistency information from the table of inconsistencies and generate automatically a natural written-style report in a Word document format. The template of the example report may include the sections:

- Identification information of current clinical report analysed (data such as the ID of the report and basic information from the target patient)
- The table associated with the genogram generated (i.e. the information extracted from the original unstructured information in the table format
- Table of inconsistencies generated
- Textual descriptions of recommendations based on the table of inconsistencies, with explanations for such recommendations

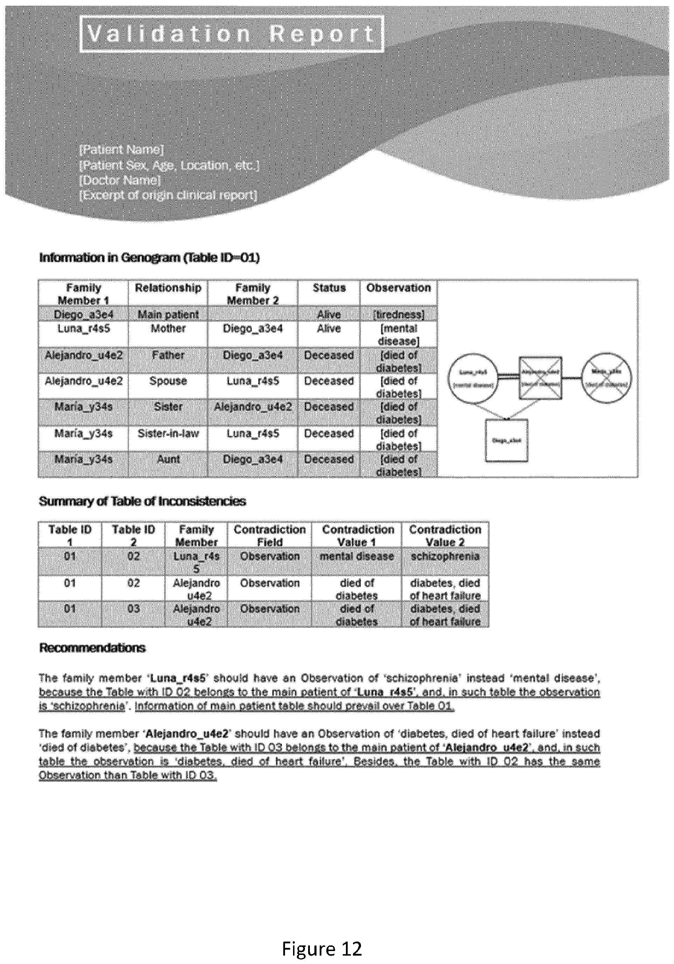

Figure 12 illustrates an example of a report. Explanations are included for recommendations.

Step S49 comprises displaying the recommendations to the user, for example with the report and in a pop-up window of the GUI. The report may be available for downloading by the user.

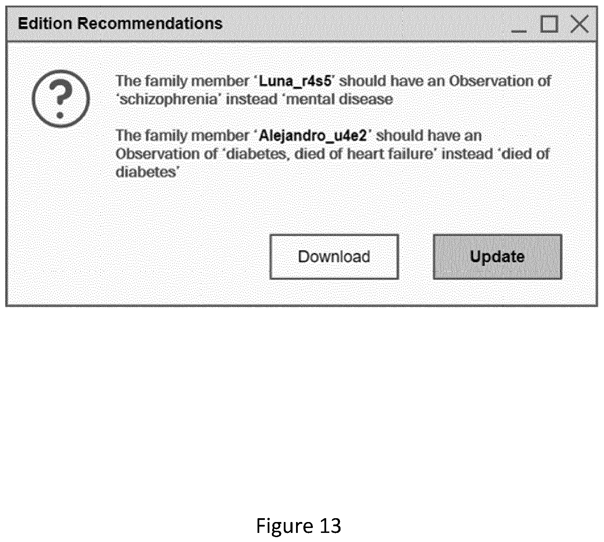

Figure 13 illustrates an example of a pop-up window to be displayed to the user including two recommendations for changes in the genogram due to inconsistencies. There are two buttons in the example window of Figure 13: A button for downloading the report file (Download); and a button for automatic generation of an updated genogram following the recommendations (Update).

Step S49 also comprises updating the genogram if user input indicates instructions to update the genogram, for example with the suggested recommendations. The update of the genogram with the new information may follow the same operations as step S38 of Figure 3, transforming the extracted information into a genogram based on the template.

After step S49, the method proceeds to step S42 in which the user can select to make further changes, or not. If the user selects not to make further changes, the method proceeds to step S43.

Step S43 comprises returning the updated (final) genogram. The genogram editor 46 outputs the final genogram.

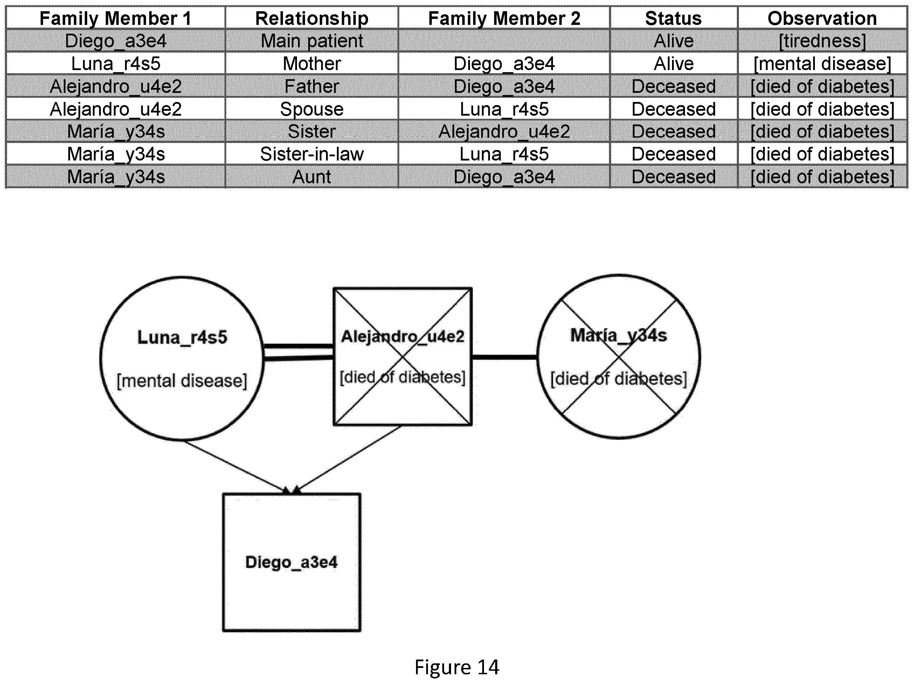

Figure 14 illustrates a table that has been updated based on recommendations from the checker module 461, and a final genogram based on the table.

Worked Example

A worked example of implementing the method according to some embodiments will be described.

Step S21 of Figure 2 comprises, in the worked example, Process Tokenization, Noun Detection, POS tagging, and Syntax Tree Dependencies identification based on Family History information in discharge summaries. A portion of the dataset of family history information is:

Mother deceased 94 DM/CAD/MI. Father deceased 81 DM/CAD. Sister deceased Breast CA/DM. Sister deceased during child birth/bleed
The patient does not report anything.
Mother with rheumatoid arthritis
Father: diabetes, stroke and MI
Positive for kidney tumor in his father, which was apparently benign. 2 healthy children without significant medical problems.
mother died of MU in 80's, had HTN and t2DM. Father died in 70's.
No family hx of sz.
Father CVA at 70 year old

pt reports asthma, HTN, HL in the family
Father died in 50's from Kidney failure, Mother died of cerebral hemorrhage in 60's Mother - CAD. Father - had stroke
Father died at 54 of MI. 5 siblings with mitral.

Each row represents one text fragment of family history information. Cleaning and processing tasks of the texts are carried out. Sentence detection, tokenization (by word-level), and noun detection are carried out. Examples of nouns in some of the texts include: Mother, Father, DM/CAD/MI, kidney tumor, 2 healthy children, cerebral haemorrhage, etc. Part of Speech (POS) tagging is carried out (e.g. Mother - NOUN, deceased - VERB, during - ADP, . - PUNCT, and - CCONJ, etc.). Syntax tree dependencies extraction is carried out (e.g. Mother - nsubj, deceased - ROOT, during - prep, . - punct, and - cc, cerebral haemorrhage - pobj, etc.).

Step S22 comprises extracting family member roles. The dictionary with an initial set of 31 basic types for family roles is defined:

```
family_roles = [father, mother, sister, parent, brother, grandmother, grandfather,
grandparent, daughter, son, ...]
```

Synsets in WordNet are searched through Python library. For instance, for 'father' the following code fragment is used to search synsets:

```
from nltk.corpus import wordnet as wn
print(wn.synsets('father'))
syn = wn.synsets('father') [1]
print ("Synset abstract term : ", syn.hypernyms ())
print ("Synset specific term : ", syn.hypernyms() [0].hyponyms())
#print ("Synset root hypernerm : ", syn.root_hypernyms())
```

The results of this search for 'father' are:
[Synset('father. n.01'), Synset('forefather.n.01'), Synset('father.n.03'), Synset('church_father.n.01'), Synset('father.n.05'), Synset('father.n.06'), Synset('founder.n.02'), Synset('don.n.03'), Synset('beget.v.01')]

Synset abstract term :
[Synset('ancestor.n.01')]

Synset specific term :
[Synset('ancestress.n.01'), Synset('forebear.n.01'), Synset('forefather.n.01'), Synset('foremother.n.01'), Synset('progenitor.n.01')]

This search is carried out for every basic type of family role and resultant terms are added to the dictionary list of family roles.

A search is iterated over all Family History texts, comparing the nouns detected with the dictionary list of family roles. For matches, the family role will be added to the output of this step in JSON format. For example, for one of the texts in the above dataset, *"Father died in 50s from Kidney failure, Mother died of cerebral haemorrhage in 60s"*, the output will be: {info: [{family_role: {name: 'Father'} }, {family_role: {name: 'Mother'} }]}".

Step S23 comprises extracting the family side associated with each family role. A similar approach as above will be followed to obtain the family side, which is either 'paternal' or 'maternal'. The detected nouns are searched and identified family side are added to the associated family role in the JSON output file of this step.

Step S24: Extract status associated with each family role: A dictionary with root terms is defined, e.g.:

$$family\_status = [\text{"died", "alive", "healthy", "living", etc.}]$$

This dictionary is extended with lemmatization and other verbal forms or variations.

To extract the status from Family Medical texts, a search is iterated over the tokens of each text. Two continuous appearances of family roles are detected. The tokens between family roles are compared with the dictionary. If there is match, the status is associated with the family role appearing before the matching token. Following the previous example, the output will be: {info: [{ family_role: {name: 'Father'}, family_status: {name: 'died'} }, {family_role: {name: 'Mother'}, family_status: {name: 'died'} }]}".

After the first extraction of statuses for the family roles of the list over the whole text, another iteration is implemented (second status search) to deal with missing statuses because of conjunctive phrases. For instance, for the sentence *'His mother, father and brother are alive',* in the first extraction the status *'alive'* will be associated only with *brother,* but in the second iteration the status is associated with *mother* and *father* as well.

Step S25: Extract observations associated to each family role. Syntax tree dependencies information is used to filter observations. A search iterates over the extracted nouns and the nouns are checked to see if they contain specific dependency tags, such as 'pobj' or 'dobj'.

Nouns appearing in the data between two appearances of family roles are analysed, similar to the previous step. Following the previous example, the output of this step in the JSON format will be: {info: [ { family_role: {name: 'Father'}, family_status: {name: 'died'}, family_observation: [ {name: kidney failure'} ] } , { family_role: {name: 'Mother'}, family_status: {name: 'died'}, family_observation: [ {name: 'cerebral haemorrhage'} ] } ]}".

Step S26: Extract modality of observations (pos, neg) associated with each family role. A positive tag (pos) is included in the extracted information if in the text the family member is described as having the observation, or, with a negative tag (neg) if in the text the family member is described as not having the observation. These pos/neg tags may also be expressed for other entities. External models may be used to include these tags.

Step S27: Build annotated dataset in JSON from extracted entities and relations. The final output in JSON format following the previous example will be (if pos/neg tags are included for all entities):

```
{info: [ { family_role: {name: 'Father', mod: 'pos'}, family_status: {name: 'died', mod:
'pos'}, family_observation: [ {name: kidney failure', mod: 'pos'} ] } , {family_role: {name:
'Mother', mod: 'pos'}, family_status: {name: 'died', mod: 'pos'}, family_observation: [
{name: 'cerebral haemorrhage', mod: 'pos'} ] } ]}".
```

In the creation of the annotated datasets, the Family History texts in the JSON format are transformed to a format including internal tags within the texts, following the definition of a tag scheme described above. The tagging text for the previous example will be:

```
<FH_ROLE_1>Father</FH_ROLE_1> <FH_STATUS_1>died</FH_STATUS_1> in 50s
from <FH_OBS_1 >Kidney failure</FH_OBS_1 >, FH_ROLE_2>Mother</FH_ROLE_2>
<FH_STATUS_2>died</FH_STATUS_2> of <FH_OBS_2>cerebral
haemorrhage</FH_OBS_2> in 60s
```

Step S28: Transform annotated dataset to BIO format. Following the previous example, the annotation in the BIO format is:

| | |
|---|---|
| Father | B-FH_ROLE_1 |
| died | B-FH_STATUS_1 |
| in | O |
| 50s | O |
| from | O |
| Kidney failure | B-FH_OBS_1 |
| , | O |
| Mother | B-FH_ROLE_2 |
| died | B-FH_STATUS_2 |
| of | O |
| cerebral haemorrhage | B-FH_OBS_2 |
| in | O |
| 60s | O |

The annotated dataset in the BIO format is input to the FMH trainer 24 and, the FMH trainer trains the FMH model using deep learning techniques and third-party libraries, to output trained FMH model.

Automatic Genogram Creator 44

For this component and the method it carries out in combination with the FMH extractor 42 (illustrated in Figure 3), the new text fragment: *"His mother is alive but has a mental disease. His father and paternal aunt died of diabetes."* will be used as an example of the unstructured data to describe the implementation of the method in the worked example.

Step S31: Data processing of FMH results. The example text fragment is input into the FMH extractor 42 which executes the FMH model to obtain results in the BIO format. The results are then transformed to the JSON format and output to the next step:

```
{info: [ { family_role: {name: 'mother', mod: 'pos'}, family_status: {name: 'alive', mod:
'pos'}, family_observation: [{name: 'mental disease', mod: 'pos'}]} , {family_role: {name:
'father', mod: 'pos'}, family_status: {name: 'died', mod: 'pos'}, family_observation: [
{name: 'diabetes', mod: 'pos'} ] }, , { family_role: {name: 'aunt', mod: 'pos'}, family_side:
{name: 'paternal', mod: 'pos'}, family_status: {name: 'died', mod: 'pos'},
family_observation: [{name: 'diabetes', mod: 'pos'} ] } ]}".
```

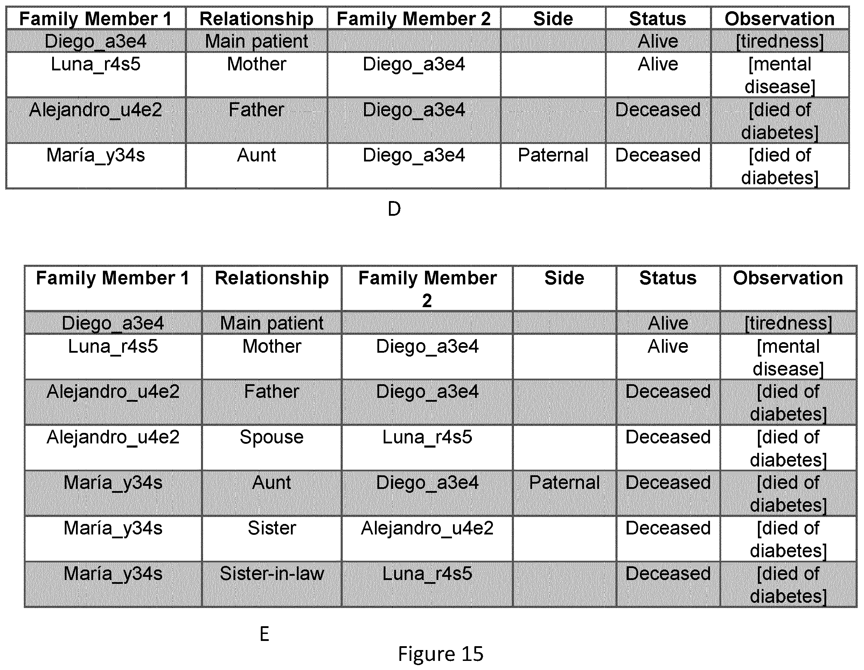

This information (directly extracted from FMH model) is then used to populate a table to obtain the table D illustrated in Figure 15.

The information in the table (in particular the relationships) is analysed to obtain inner-relations between family members using a rule-based based on known family relation rules. For instance, in table E the process begins with 'Mother' and is iterated over the rest of members, and then the process begins with the next relationship, "Father", and is iterated over the other family members, and so on. For example, *María_y34s* has the relationship "Aunt" and family side "Paternal" with *Diego_a3e4* and *Alejandro_u4e2* has the relationship "Father" with *Diego_a3e4,* therefore using a rule-based approach including a rule indicating that a person's paternal aunt is their father's sister, it is derived that the relationship for *María_y34s* with *Alejandro_u4e2* is "Sister". After applying this algorithm over the initial table D, the table E in Figure 15 is generated including the inner-relations between family members.

Step S38: Transform results to representation template. Next, the information in the table E is transformed to a graphic representation using the *graphviz* python library. For this transformation a genogram template has to be determined first, by the steps carried out by the template module 441 and indicated with the dashed box in Figure 3.

Step S32: Load pre-saved templates. A pre-defined set of standard templates stored in a database using JSON configuration files and properties files is provided and loaded. Each JSON configuration file is of attribute-value type scheme. The following is an excerpt of a JSON configuration file for an example template:

```
{"template_name"="x","female_basic_form"="circle",
"female_deceased_form"="fill-
black", "female_carrier_form"="middle_point", "male_basic_form"="square",
"relation_line"="basic_line", "text_alignment_node"="bottom_form", ...}
```

The properties file links the name of the forms/shapes in the template with the physical location of the file with the shape stored. An example of a properties file is:

```
circle=/genogram/circle.eps
circle_fill-black=/genogram/circle_fill-black.eps
square_square_bracket=/genogram/square_square_bracket.eps
```

Figure 6 describes above shows the GUI of the first window of what may be considered a genogram creation tool where all standard templates stored are provided to the user in a combo-box to be selected.

Several examples of known standards included may be:

- Proposed standard by the National Society of Genetic Counsellors, Pedigree Standardization Task Force (PSTF) (Bennett, Robin L., et al. "Standardized human pedigree nomenclature: update and assessment of the recommendations of the National Society of Genetic Counselors." Journal of genetic counseling 17.5 (2008): 424-433)
- https://onlinelibrary.wiley.com/doi/epdf/10.1007/s10897-008-9169-9
- Adaptations from the previous standards:https://precisionmedicine.duke.edu/sites/precisionmedicine.duke.edu/files/field/attachments/Standard_Pedigree_Symbols.pdf
- Or based in Bennet's standard: https://d3i71xaburhd42.cloudfront.net/7c061fc841ef5f16c60f83a4bf265c7ec842c5e3/7-Figure8-1.png

Steps S33 to S37 are described above.

An example configuration file for a template is as follows:

```
{"template_name"="genogram_1", "female_basic_form"="circle",
"male_basic_form"="square", "deceased_form"="diagonal_cross_lines",
"carrier_form"="middle_point", "relation_line_sibling"="basic_line",
"relation_line_spouse"="double_line", "relation_line_parent"="directed_arrow_line",
"text_alignment_node"="bottom_form", ...}
```

For the transformation to visual representation in step S38, the JSON template file is processed and formatted to a data structure required by the library. For instance, following the above example JSON configuration file, the data structure is:

```
 "edges":

          {
          ["relationship": "sibling",
          "shape": "basic line"],
          ["relationship": "spouse",
          "shape": "double line"],
          ["relationship": "parent",
          "shape": "directed arrow line"]
          ...
          },
          "nodes":
          {
          ["member": "is_male",
          "shape": "square"],
          ["member": "is_female",
          "shape": "circle"],
          ["member": "is_deceased",
          "shape": "diagonal_cross_lines"
          ...]
          }
```

The resultant genogram created automatically is that illustrated in Figure 14 described above.

The description of the worked example continues with the method in Figure 9.

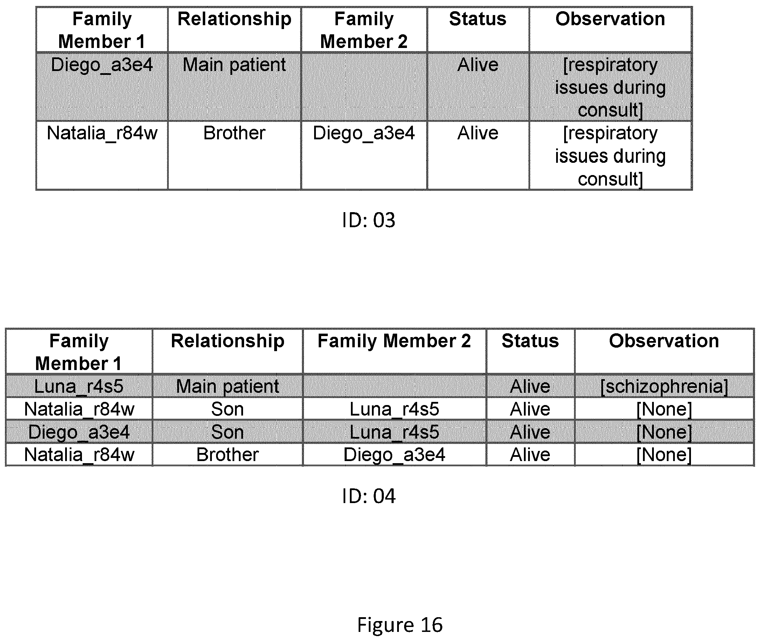

Step S45: Data processing of FM historical (from patient and relatives). In this step, two new reports are processed for this example: a previous medical report on the target patient (with the target patient as the subject patient of the report) and a medical report on his mother (i.e. with the mother as the subject patient of the report). These reports are processed by the FMH Extractor 42 and information is extracted. The information populates new tables illustrated in Figure 16 with IDs 03 and 04.

Step S46: Cross historical information with current genogram & changes. At this step the two new tables generated in the previous step are "crossed" with the initial genogram for validation and inconsistency detection. The initial genogram table used to represent the information in the genogram is table E of Figure 15. The tables 02 and 03 generated in this example show a common mistake due to sex ambiguity. "Natalia_r84w" is stated to be the brother of "Diego_a3e4" and son of "Luna_r4s5", and because of this is tagged as male. This error is expected to be corrected in later steps.

Step S47: Validate and Detect contradictions. In this step, the data in the initial genogram table E and the tables 03 and 04 is analysed to find inconsistencies.

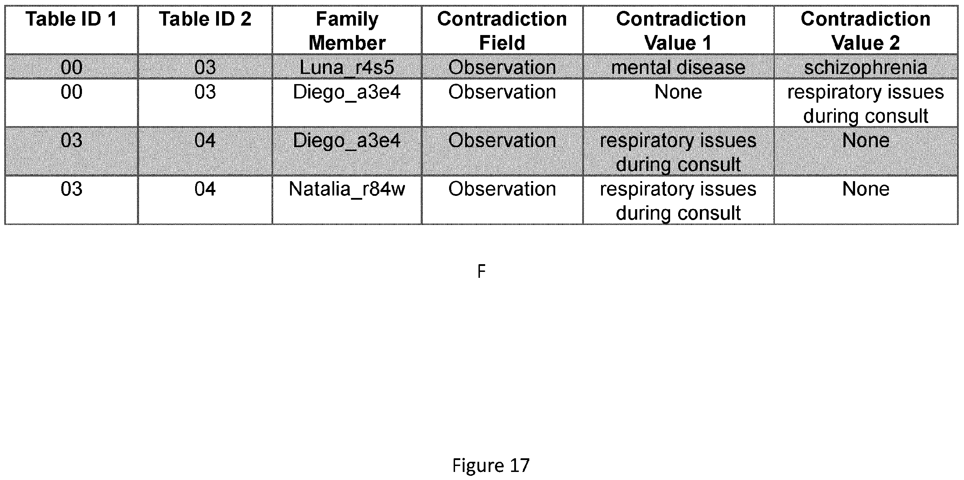

After iterations over each individual (family member) in the table a new table of inconsistencies is obtained (table F in Figure 17), the table containing possible inconsistencies and contradictions.

Step S48: Prepare Report and Recommendations. The prepared report is prepared and may be presented to the user with recommendations based on the inconsistencies encountered.

Step S49: Show report & recommendations to user. In this step the user may address each contradiction through a pop-up window (of the GUI) for each contradiction as illustrated in Figure 13.

The first inconsistency of table F is resolved by a merge of values since "schizophrenia" is complementary to "mental disease". In the same way, the second and third inconsistencies are omitted and deleted since "respiratory issues during consult" is not part of the diseases of interest for the target patient.

At this stage, the only error in the genogram to be fixed is "Natalia_r84w" sex, since the correct sex was not stated in the medical report and it was not corrected by the checker in this module.

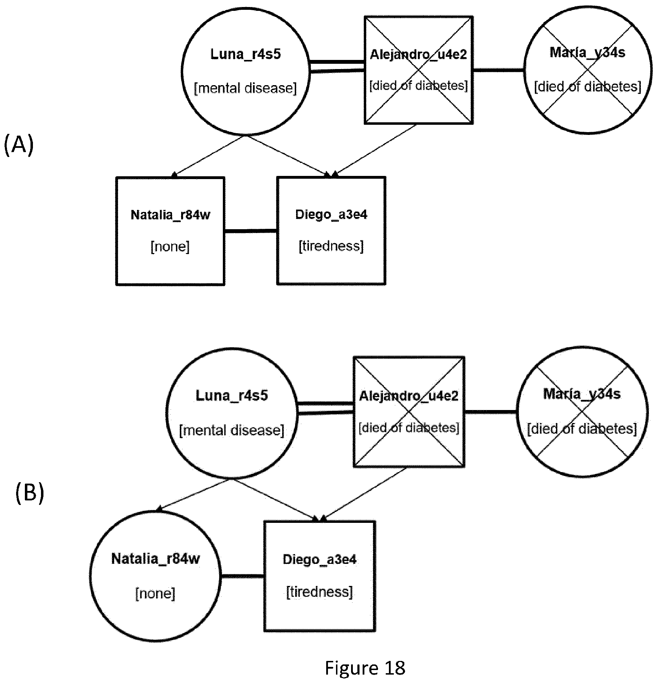

Step S41: Load Editor GUI window. Before the final Genogram is generated, it is useful to validate the preview genogram. In this case the new individual "Natalia_r84w" is presented with a square shape since there was no information regarding her sex in previous reports. The preview genogram that is displayed to the user in this example is illustrated in (A) of Figure 18. Using the GUI, the user clicks on the shape of the "Natalia_r84w" node and manually modifies the shape to a circle. The final Genogram is then outputted, which is illustrated in (B) of Figure 18.

Some benefits of embodiments may be:

- Reduced time of Genogram creation (e.g. from an average of 30 minutes to seconds, due to the automatic generation)

- Cross-validation to update genograms with newly available data (from different times and/or different patients)
- No need for a user to have deep knowledge of Genogram representations, since there is no need for the user to create the genogram from scratch (due to automatic generation and templates loading)
- Support in Genogram editing with recommendations
- Multi-language capabilities

This technology could be integrated as a plugin inside existing current frameworks or integrated within a bigger clinical decision support system for healthcare applications.

Embodiments may include an automated genogram generation system that processes the information of family medical history automatically from unstructured clinical reports in textual format with natural language processing and deep learning techniques. The system may comprise:

- a learning process engine that creates models capable of processing textual documents that contain family medical information to extract structured relevant data from such information through rule-based, natural language processing and deep learning approaches;
- an automatic genogram creator engine to receive data from textual clinical reports and transform such data to generate the genogram visual representation without human intervention;
- a genogram editor engine to allow modifications of the genogram generated with automatic cross-validation mechanisms among family members and reports along time to maintain consistency in the genogram, and, recommendations for consistent editions based on such cross-validations presented with explainable references in natural text-style.

The learning process engine may be configured with text processing rule-based methods to generate automatically annotated family medical history input datasets from textual documents to be used during the learning workflow for creating the models. Models created by the learning process engine may be exploited by the automatic genogram creator engine to extract family medical data from texts without human intervention.

Genogram generation may be performed based on the support of genogram template representation setup module that defines novel interfaces and implementations of form-based configuration templates to load or create ad-hoc types of visual genogram representations. The types of visual genogram representations may be created through form-based templates in graphical user interfaces, and, such form-based templates may be transformed to configuration scheme files maintained in persistence data-storage system. Automatic processing may be performed of inner-relations between family members in the textual clinical reports using natural language processing and rule-based techniques over known family relations, to be appended to the initial data received from such textual clinical reports.

The automatic genogram generated may be inputted to the genogram editor engine to be enhanced by editable information that is checked via cross-validation of family members' textual clinical reports and historical reports. The inconsistencies found in the cross-validation checking may be supported by recommendations with explainable references in natural text-style.

Automatic updates of the genogram following recommendations may be performed. The final genogram outputted may be integrated in a clinical decision support system that manages textual clinical reports for monitoring patients at increased risk of developing a particular condition that runs in their family.

Embodiments may comprise outputting a diagnosis for the target patient based on the genogram. Embodiments may comprise prediction and monitoring of potential diagnosis and future risks for patients by the automated analysis of the joint features of symptoms, family medical history and physiological measurements e.g. collected through wearable devices. That is, embodiments may include outputting a diagnosis based on any of a genogram (e.g. as created above), at least one symptom of the target patient, and at least one physiological measurement of the target patient. A wearable device may be used to collect any of the at least one symptom, the at least one physiological measurement, and family medical history data.

Embodiments may comprise a wearable device for collection and monitoring of patients' information regarding vital signs (e.g., temperature, pulse rate, blood pressure, etc.) and other data provided by patients. The nature of collected information is of two different types:

1. Implicit data collected automatically by the device: Vital signs (physiological measurements)
2. Explicit data provided voluntarily by the patients and shared via voice from the patient to the device, which may include either of:

- Symptoms in real time, described by speech from patient to device microphone
- Family Medical History information, shared by speech from patient to device microphone

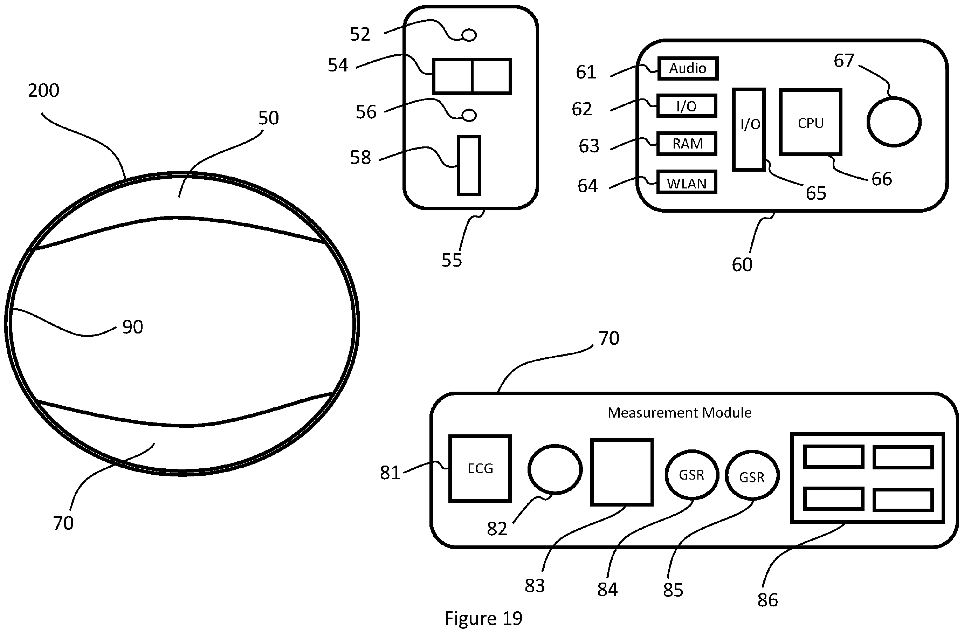

The wearable device may be a bracelet. Figure 19 is a diagram of such a bracelet 200. The bracelet 200 comprises a bracelet portion 90, a system module 50, and a measurement module 70.

The system module 50 comprises an upside 55 which comprises components for communication purposes between a patient and the bracelet 200. The upside 55 comprises a microphone 58, a speaker 56, buttons 54, and a light 52. The microphone 58 is used to collect information from the patient by speech to the device 200. The speaker is configured to output diagnosis/risks predictions and preventive guidelines from the device 200 to the patient. The patient-device communications will be carried out in natural speech. The buttons 54 comprise a 'Record Button' (when the patient clicks on this button the audio recording will start and the patient can talk into the microphone 58) and a 'Play Button' (when the patient clicks on this button any pending message generated by the device 200 or sent from the doctor will be transmitted via the speaker 56). The light 52 is a 'Message Alert Light' and alerts the user if there are any pending messages. For example, the light 52 turning red indicates that a new message came in.

The system module 50 also comprises a Down-Inside 60 or a Motherboard Module 60. The motherboard module comprises a power battery 67, a CPU 66 for processing tasks, a BUS 65 to connect and facilitate communicate between components of the motherboard module 60, random access memory (RAM) 63 to save in memory permanent data such as the Family Medical History information or software apps, a wireless local area network (WLAN) 64 for network connections, and Audio and I/O hardware 61, 62 to manage the functionality of the microphone 58 and the speaker 56.

The Measurement Module 70 includes sensor units to collect physiological data (i.e., the vital signs). The measurement module comprises:

- Electrocardiogram (ECG) sensor 81 to collect electrical signals generated by the heart;
- Thermometer 82 to measure the current temperature of the patient;
- Other Biopotential sensors 83 such as electromyogram (EMG) sensor to measure the health of the muscles or pacemaker pulse detection sensors or bioimpedance sensors to monitor the health of tissues.
- Galvanic Skin Response (GSR) sensors 84, 85 to collect measurements about the electrical conductance of the skin to study the emotional state of the patient. Perturbations in the sweat gland activity are directly connected with the intensity of a person's emotional state.
- A set of Optical sensors 86 to measure different physiological information such as blood pressure, pH analysis of the blood (i.e. pH levels), skin anomalies by light intensity, glucose level of the blood (blood-glucose level), etc. Some information on such measurements may be found at

  https://www.webmd.com/lung/what-to-know-about-acid-base-balance#:~:text=The%20acidity%20and%20alkalinity%20of,usually%20betwee n%207.35%20to%207.45,
  https://www.researchgate.net/publication/344375632_Wireless_wearable_wrist
  band_for_continuous_sweat_pH_monitoring, and
  https://www.healthline.com/diabetesmine/non-invasive-diabetes-technology#how-does-it-work.

The implicit data (physiological measurements) is collected by the Measurement Module 70 and processed by the Motherboard Module 60, and the explicit data is collected by the microphone 58 and processed by Motherboard Module 60.

The bracelet 200 may output two types of outputs:

- Diagnoses, or predictions on potential diagnosis/risks and preventive recommendations, communicated from the device to the patient by the speaker in natural language speech.
- Predictions and automated information generated (such as Family History data extracted and Genogram), sent to central server in medical institution and merged in the Electronic Health Record (EHR) database of the patient from the device 200 via network connection.

A workflow of the bracelet 200 will be described according to an example. The bracelet has stored therein (e.g. in the RAM 63) the FMH model which has been trained as described above, as well as other software to carry out the genogram creation.

Collection of information

Implicit data: physiological measurements (i.e., vital signs) are collected in real-time from the data extracted with the sensors of the wearable device 200.

Explicit data

- Symptoms in real-time: The patient clicks on the 'Record Button' and talks into the microphone 58, describing their symptoms. For instance, the patient may express the following message by voice to the microphone 58: *"I feel tachycardia and respiratory distress. Besides, I have dry cough and fever episodes".*

- Family Medical History information: When sharing this kind of information for the first time, the patient clicks on the 'Record Button' and talks into the microphone 58, describing their relatives' health history. This information is stored in a database e.g. in the RAM 63. The patient can update this information by sharing further family medical history information in the same way by talking into the microphone 58. The Motherboard module 60 saves the new information in a/the database e.g. in the RAM 63. For instance, the patient could add the following further information: *"I have two brothers and two sisters, one of which died of old age. My living siblings have dementia, hypertension, diabetes mellitus and a stroke. Five children, one with asthma, three with hypertension".*

Receive/Transformation of information

The implicit data (i.e., physiological measurements) are sent internally in the device 200 from the sensors to Motherboard Module 60. The I/O connectors 62 of the motherboard module 60 facilitate the communication of such data from sensors to the CPU 66 and memory 63. The CPU 66 handles data with installed software in memory for data manipulation to deal with the categorical and number values depending on the origin sensor. Each sensor, depending on its nature, will provide values in different formats (numbers, category values, chart lines). For instance, pacemaker pulse detection sensors, thermometers, pressure sensors, or pH levels sensors may give numerical values; ECG or EMG sensors may give a chart line of numerical time series; and GSR results may be categorized as a positive or a negative emotional state depending on the intensity of the sweat gland activity.

The explicit data (i.e., symptoms in real-time and Family Medical History) are sent internally from the microphone 59 to the CPU 66 and memory 63 via I/O connectors 62, and the Audio and BUS component 61. The CPU 66 handles the information through installed software in memory of Speech-to-Text Recognition. This software may comprise third party tools. For example the library of SpeechRecognition (https://pypi.org/project/SpeechRecognition/) in Python may be utilised. This tool acts as a wrapper for popular speech APIs, and is flexible and easy to use. It provides support for handling audio data directly from microphone inputs. Several speech APIs supported in this tool are Google Cloud Speech API (https://cloud.google.com/speech/), Microsoft Bing Voice Recognition (https://www.microsoft.com/cognitive-services/en-us/speech-api), or IBM Speech to Text (http://www.ibm.com/smarterplanet/us/en/ibmwatson/developercloud/speech-to-text.html) among others. Using such software the audio data is transformed into a textual format.

At the end of this step, the device has obtained real-time values of physiological measurements by the sensors including type of value (numerical, time-series, categorical), and real-time symptoms and the Family Medical History in a natural language text format.

Processing information of Explicit data

- Real-time symptoms: To extract the symptom entities in the text, Named Entity Recognition (NER) will be used. Proprietary in-house software or from third party tools such as SpaCy (https://spacy.io/) or NLTK (https://www.nltk.org/) may be utilised. After this processing, a list of entities of the type "Symptom" will have been obtained. In the previous message (*"I feel tachycardia and respiratory distress. Besides, I have dry cough and fever episodes"*), the extracted entities would be [tachycardia, respiratory distress, dry cough, fever episodes].
- Family Medical History information: To extract the entities and relations, the methods above for extracting information from unstructured (medical history) for genogram creation are used.

Predict potential diagnoses/risks

The physiological measurements (e.g. vital signs) and the current symptoms are considered together by software e.g. in the CPU 66 and an estimation decision of current diagnoses is provided following clinical rule-based algorithms. Such algorithms involve a set of premises (the vital signs and symptoms), analyse a set of logical rules with such premises, and return a conclusion based on the output of the logical rules. The set of logical rules may be defined by physicians and healthcare professionals. A simple example is: *if Symptom(nasal congestion) and Symptom(headache) and Thermometer(value > 38°C) -> Diagnosis(Flu, cold).*

The outputted potential diagnoses are corroborated through the analysis of the patient's family medical history. In light of this family medical history data the most suitable diagnosis among various options may be confirmed, new potential diagnoses may be included, and some potential diagnoses discarded.

Automated generation of the genogram as described above will be carried out by the device 200 to analyze inheritance patterns in the family. Rule-based approaches are followed by software e.g. in the CPU 66 to analyze the genogram, including logical rules to express inheritance traits of diagnoses, and may be defined by physicians and/or healthcare professionals. The result of analyzing the genogram is at least one potential diagnosis.

The at least one diagnosis resulting from the analysis of the symptoms and physiological measurements is compared with the at least one diagnosis resulting from the analysis of the genogram, for example by software in the CPU 66. Below, three main cases are considered:

1. If at least one diagnosis appears in the results from both analyses, it is marked as a primary diagnosis, and the rest of the diagnoses are extracted as secondary or less relevant.
2. If there is no diagnosis appearing in the results from both analyses, all diagnoses extracted will be considered of the same importance.
3. If there are no diagnoses output from the analysis of the genogram (i.e. no relevant inheritance conditions found), only the diagnoses output by the analysis of vital signs and symptoms will be taken into consideration.

Merge results with EHR and review information

The WLAN component 64 may provide network connection to link the device 200 with a Healthcare system and thus an Electronic Health Record (EHR) of the patient. In this way, the data collected and the results of the analysis may be transmitted to the patient's EHR. Then, merging tasks may be carried out to update the data of the current records with the new information and avoiding any inconsistency or redundancy issues. In the storing of the information, snapshots of temporal data may be maintained such as the current vital signs and symptoms, including datelines of such episodes. Permanent data will also be stored such as the (potential current/future) diagnoses, family history and genogram, for which updates will be required less frequently.

A physician may examine the information stored in the patient's EHR to improve it or correct possible mistakes. The methods described above (e.g. in Figure 9) may be used to edit the genogram. The data improved/corrected by the physician may be saved in the patient's EHR. Recommendations (e.g. to see a doctor or how to prevent particular diseases from occurring) may be sent to the patient, for example to the device 200 to be output by the speaker 56.

In a first round, to avoid communication delay to the patient due the physician reviewing the data and possible making changes, initial recommendations may be output to the patient based on the preliminary predictions. In a second round, after a physician has checked the data (validating the information or including modifications), a new message may be sent to the patient to corroborate previous recommendations or provide different ones. The message in the first round may be produced in seconds. For the second round, the data updated or validated by the physician is sent from the healthcare system maintaining the EHR record to the device 200, and the device 200 produces the new message accordingly. In both cases the generation of recommendations may be carried out by software installed in the RAM 63 and processed by the CPU 66. Such recommendations and prevention tips may be generated by rule-based approaches following clinical guidelines and physicians' expertise. For instance, if a patient presented symptoms like irritation of breast skin and new lump in the breast and has a family history of cancer conditions, the methods described above may predict as a primary diagnosis *breast cancer,* and may predict secondary diagnoses such as *skin cancer, unknown cancer.* Therefore, the logical rules implemented following clinical guidelines may generate recommendations of visiting an oncology specialist, having a mammogram, and monitor/check the progress of the irritation and the lump.

Once the recommendations are generated in natural language text, Text-to-Speech techniques may be used to produce the audio message in natural language speech. Like techniques for Speech-to-Text described previously publicly available third-party tools may be used. For Python implementations, two possible libraries may be used among others: gTTS (https://pypi.org/project/gTTS/) (Google Text-to-Speech) or pyttsx3 (https://pypi.org/project/pyttsx3/).

When the message is created the 'Message Alert Light' 52 turns red, for example (it may instead be another colour(s) and/or may flash), then the patient presses the 'Play Button' and the audio message is output by the speaker 56. The message output may include the predictions of potential current/future diagnoses and the recommendations.

In the example described above the wearable device 200 is a bracelet 200. In other examples the wearable device 200 may be a different item, for example a ring, a watch, a necklace, a pad/patch, a headband, an armband, or a band, etc.

In the example described above the wearable device 200 carries out all of the operations. In other examples the device 200 is part of a system comprising the wearable device 200 and an apparatus, and the operations may be shared in various ways between the device 200 and the apparatus. For example, the device 200 may collect at least one physiological measurement and/or at least one symptom and/or unstructured (medical history) data as described above and may then transmit this information to the apparatus. The apparatus may carry out operations described above to create the genogram and allow it to be edited. The apparatus or the device 200 may carry out the diagnosis prediction. In some examples the unstructured (medical history) information and/or the at least one symptom and/or the at least one physiological measurement may be provided to the apparatus in the form of textual data and/or speech input instead of to the device 200. The apparatus may implement all of the methods above and the system may not comprise the device 200 in some examples. Embodiments include a method for creating the genogram and outputting at least one diagnosis as described above and below with or without a device 200 - that is, the information collected by the device 200 in the above example may be provided to a system/apparatus which implements the method.

In an example, a method (executed by the device 200 or by another apparatus comprised in a system including the device) of outputting the at least one diagnosis for the target patient comprises

> A. predicting at least one genogram diagnosis, the at least one genogram diagnosis being at least one diagnosis of the target patient based on the genogram;
> B. predicting at least one symptom diagnosis, the at least one symptom diagnosis being at least one diagnosis of the target patient based on the at least one symptom of the target patient;
> C. predicting at least one measurement diagnosis, the at least one measurement diagnosis being at least one diagnosis of the target patient based on the at least one measurement of the target patient, and
> D. comparing the at least one genogram diagnosis, the at least one symptom diagnosis, and the at least one measurement diagnosis and, when at least one diagnosis among the at least one genogram diagnosis is the same as a diagnosis among the at least one symptom diagnosis and at least one diagnosis among the at least one measurement diagnosis, outputting the at least one diagnosis as at least one final diagnosis.

The feature described above of corroborating a diagnosis with the medical history data may be considered accomplished by feature D.

Predicting the at least one genogram diagnosis may comprise: maintaining a genogram diagnosis score for each of a plurality of possible diagnoses; adjusting at least one of the genogram diagnosis scores based on a set of genogram diagnosis rules and based on the genogram; and determining at least one of the possible diagnoses having the highest genogram diagnosis score as the at least one genogram diagnosis.

Each genogram diagnosis score may comprise: a primary score based on information in the genogram about at least one sibling of the target patient; a secondary score based on information in the genogram about at least one parent of the target patient; and a tertiary score based on information in the genogram about at least one grandparent of the target patient and/or at least one sibling of a parent of the target patient and/or at least one cousin of the target patient.

For each genogram diagnosis score, the primary, secondary, and tertiary scores may be summed together with primary, secondary, and tertiary weightings, respectively, to generate the genogram diagnosis score.

Predicting the at least one symptom diagnosis may comprise: maintaining a symptom diagnosis score for each of a plurality of possible diagnoses; adjusting at least one of the symptom diagnosis scores based on a set of symptom diagnosis rules and based on the at least one symptom of the target patient; and determining at least one of the possible diagnoses having the highest symptom diagnosis score as the at least one symptom diagnosis.

Predicting the at least one physiological diagnosis may comprise: maintaining a physiological diagnosis score for each of a plurality of possible diagnoses; adjusting at least one of the physiological diagnosis scores based on a set of physiological diagnosis rules and based on the at least one physiological measurement of the target patient; and determining at least one of the possible diagnoses having the highest physiological diagnosis score as the at least one physiological diagnosis.

Determining the at least one genogram diagnosis may comprise disregarding a diagnosis if its genogram diagnosis score is below a genogram diagnosis score threshold. Determining the at least one symptom diagnosis may comprise disregarding a diagnosis if its symptom diagnosis score is below a symptom diagnosis score threshold. Determining the at least one physiological diagnosis may comprise disregarding a diagnosis if its physiological diagnosis score is below a physiological diagnosis score threshold.

The diagnosis scores may be calculated following a rule-based approach defining an amount to be added or subtracted to a score in view of one or more particular symptoms or physiological measurements within particular ranges or particular family medical history data. The particular family medical history data may include family members having particular diseases or conditions or afflictions.

Example - diagnosing 'heart disease'

Described below is an example following embodiments to diagnose heart disease (in this specific case a diagnosis of Heart Failure is output).

Collection of information

Implicit data: Physiological measurements (i.e., vital signs) are collected e.g. in real-time from the data extracted with the sensors of the wearable device 200. In this example the following physiological measurement values are collected (these measurements are relevant in the context of Heart Disease):

- ECG sensor: Time-series values of the last 15 minutes. Very irregular values and abnormalities present in the electrocardiogram waveforms. The irregularities and abnormalities may be identified/detected by comparing, using a computer, the measurements with other ECG measurements of healthy and of unhealthy hearts.

- Thermometer: Temperature of 38.6° measured
- EMG sensor: Time-series values of the last 15 minutes. Abnormal values and low electromyographic activity measured. The abnormalities may be identified/detected and the electromyographic activity classified (e.g. as low) by comparing, using a computer, the measurements with other EMG measurements of healthy and of unhealthy hearts.
- Pacemaker pulse detection: Low breathing rate and high and enlarged heart rate measured. The breathing and heart rate may be classified (e.g. as low/high and enlarged) by comparing, using a computer, the measurements with other pacemaker pulse detection measurements of healthy and of unhealthy hearts.
- GSR sensors: Increased skin conductivity with low GSR activity values. The skin conductivity and GSR activity values may be classified (e.g. as increased and low) by comparing, using a computer, the measurements with other GSR measurements of people with healthy and unhealthy hearts
- Other measurements from Optical sensors:

  • High blood pressure
  • pH analysis: acid-base balance
  • Skin light intensity: Values in normal range
  • Blood-glucose levels: values in the normal range

Physiological measurements may be classified e.g. as "normal", "low", "healthy", "abnormal", etc., by comparing (using a computer) those measurements with corresponding measurements of other people (e.g. with healthy and unhealthy hearts, in this case).

The symptoms and family medical history information are collected/acquired according to any of the examples described above (e.g. by the device 200 or from storage or a server or apparatus, etc.).

Receive/Transformation of information

Some useful definitions are below:

• P-wave axis: a measure calculated from an ECG of the net direction of atrial depolarization. It is determined by measuring net positive or negative P-wave deflections on all six limb leads and calculating the net direction of electrical activity using the hexaxial reference system. Abnormal P-wave axis may be defined as any value outside 0-75°. The P wave is an integral part of an ECG. It represents the electrical depolarization of the atria of the heart. It is typically a small positive deflection from the isoelectric baseline that occurs just before the QRS complex.

• QRS duration: A combination of the Q wave, R wave and S wave, the "QRS complex" represents ventricular depolarization. The normal duration (interval) of the QRS complex may be considered to be between 0.08 and 0.10 seconds - that is, 80 and 100 milliseconds. When the duration is between 0.10 and 0.12 seconds, it may be considered intermediate or slightly prolonged. A QRS duration of greater than 0.12 seconds may be considered abnormal.

• QRS axis: The QRS axis represents the major vector of ventricular activation, which is the overall direction of electrical activity.

• General definition of axis in the context of the heart: When all electrical signals from the heart are averaged, the direction of the average electrical depolarization can be indicated with an arrow (vector). This is the heart axis. A change of the heart axis or an extreme deviation can be an indication of pathology. To determine the heart axis the extremity leads only (not V1-V6) are analysed. If focus is especially given to leads I, II, and AVF a good estimate of the heart axis can be made. An important concept in determining the heart axis is the fact that electricity going towards a lead yields a positive deflection in the electric recording of that lead. It can be useful to imagine the leads as cameras looking at the heart. Lead I looks horizontally from the left side. Lead II looks from the left leg. Lead III from the right leg and lead AVF from below towards the heart. A positive deflection here is defined as the QRS having a larger 'area under the curve' above the baseline than below the baseline.

The implicit data, i.e. physiological measurements, are sent to the Motherboard Module 60 and information is extracted from the data so that the physiological measurements may be processed according to the methods described above. Following the physiological measurements and their values described above for this example, the following information is extracted in this example:

- ECG sensor. Transform the (15 minutes worth of) time-series to relevant categorical and numerical values. Specific values in this example include (with associated rules indicated inside the parentheses {}): P-wave axis 99° (enlarged) {normal range between 0°-75°}, QRS duration = 0.17 (prolonged duration) {normal duration between 0.08 and 0.10}, Time to ID=62ms (abnormal) {values > 50ms are considered abnormal}, QRS axis=-45° (left axis deviation) {left axis deviation if QRS axis between -30° and -90°}. Therefore the categorical information extracted from the ECG sensor measurement includes: P-wave axis is 'enlarged'; QRS duration is 'prolonged'; time to ID is abnormal; left axis deviation=True.

- Numerical value of temperature=38.6°.
- EMG sensor. Transform the (15 minutes worth of) time-series to relevant categorical and numerical values. Specific values in this example include (with associated rules indicated inside the parentheses {}): Cross-Sectional Area (CSA) muscle ~ 134 cm$^2$ (reduction) {normal values 165.2 $\pm$ 7.4 cm$^2$, $P$=0.002}, muscle strength ~ 227 N (reduction) {normal values 286.9 $\pm$ 17.1 N, P<0.05}, muscular fatigability ~ -2.10 (high) {normal values - 0.54 $\pm$ 0.20 N/s, P<0.01}, submaximal contraction ~ 68 % (low) {normal values 114 $\pm$ 36%; P<0.05}. Therefore the categorical information extracted from the ECG sensor measurement includes: CSA muscle as 'reduction', muscle strength as 'reduction', muscular fatigability as 'high', submaximal contraction as 'low'. In the above measurements, P is the p-value. In statistics, the p-value is the probability of obtaining results at least as extreme as the observed results of a statistical hypothesis test, assuming that the null hypothesis is correct. A statistically significant test result (P $\leq$ 0.05) means that the test hypothesis is false or should be rejected. A P value greater than 0.05 means that no effect was observed.
- Pacemaker pulse detection. The categorical information extracted from the pacemaker pulse detection measurement includes: breathing rate='low', heart rate='high', heart rate='enlarged'.
- GSR sensors (skin conductivity measurement): Categorical information extracted: Values around less than 15kohms (low values indicating "negative" emotional states such as "sad", "fear", "angry", etc.)
- Other measurements from Optical sensors. Categorical values:

  • Blood pressure=high
  • pH analysis: acid-base balance
  • Skin light intensity: normal
  • Blood-glucose levels: normal

In the above example measurements, CSA muscle is the area of the cross section of a muscle perpendicular to its fibres, generally at its largest point (it is typically used to describe the contraction properties of pennate muscles); muscle strength may be defined as the ability to exert force on an external resistance; muscular fatigability may be is defined as a decrease in maximal force or power production in response to contractile activity; submaximal contraction may be defined as e.g. a number of all contractions without maximal effort. Further information on EMGs and particular quantities that may be measured may be found at https://www.ncbi.nlm.nih.gov/pmc/articles/PMC3821366/.

An example of the symptom information in this example, for example recorded by the target patient using the microphone 58 and converted to textual data is: *"I feel tired and weak, and I have chest pain and difficulty breathing. Sometimes I experience tachycardia, nausea and accelerated heartbeat. I have had a persistent cough for several days"*.

An example of the family medical history data in this example, for example recorded by the target patient using the microphone 58 and converted to textual data is: *"My mother is healthy. My father died of a heart valve problem at age 52 and 4 of my siblings had heart problems (though not valvular disease). My paternal grandmother died of a heart attack. My paternal grandparent has carotid artery disease. My paternal uncle died of a heart attack too. My maternal grandmother has diabetes and hypertension"*.

Processing information of Explicit data (symptoms and family medical history)

Real-time symptoms: NER techniques are used to extract the following entities from the textual data relating to symptoms: [tired, weak, chest pain, difficulty breathing, tachycardia, nausea, accelerated heartbeat, persistent cough]

Family Medical History data: The methods described above are used to extract information from the family medical history data (e.g. step S31 using the trained FMH model). The FMH model may be trained specifically on training data including a family history of heart disease.

The following JSON description ('mod' attribute is the modality of entity; pos=positively present, neg=absence) is extracted from the family medical history data above:

```
[ {
"family_role": {"name": "mother","mod": "pos"},
"family_status": {"name": "healthy","mod": "pos"} },
{
```

```
"family_role": {"name": "father","mod": "pos"},
"family_status": {"name": "died","mod": "pos"},
"family_observation": [{"name": "a heart valve problem","mod": "pos"}]},
{
"family_role": {"name": "siblings","mod": "pos"},
"family_observation": [{"name": "heart problems","mod": "pos"},{"name": "valvular
disease","mod": "neg"}]},
{
"family_role": {"name": "grandmother","mod": "pos"},
"family_side": {"name": "paternal","mod": "pos"},
"family_status": {"name": "died","mod": "pos"},
"family_observation": [{"name": "heart attack","mod": "pos"}]},
{
"family_role": {"name": "grandparent","mod": "pos"},
"family_side": {"name": "paternal","mod": "pos"},
"family_observation": [{"name": "carotid artery disease","mod": "pos"}]},
{
"family_role": {"name": "uncle","mod": "pos"},
"family_side": {"name": "Paternal","mod": "pos"},
"family_status": {"name": "died","mod": "pos"},
"family_observation": [{"name": "heart attack","mod": "pos"}]},
{
"family_role": {"name": "grandmother","mod": "pos"},
"family_side": {"name": "maternal","mod": "pos"},
"family_observation": [{"name": "diabetes","mod": "pos"},{"name": "hypertension","mod":
"pos"}]}]
```

The extracted family medical history data may be stored in the database of the device 200 or elsewhere in a system comprising the device 200.

Determining Diagnoses

A rule-based approach is followed to determine physiological diagnosis scores for a number of heart diseases (and other diseases/conditions) based on the physiological measurements above in this example. Initially, the physiological diagnosis scores start at 0. A change in a physiological diagnosis score is indicated as follows: a physiological measurement resulting in adding 1 to a diagnosis score is shown below by "Diagnosis([condition], +1)".

If *P-wave*=='enlarged':

Diagnosis(atrial enlargement, +1), Diagnosis(chronic respiratory disease, +1), Diagnosis(heart failure, +1), Diagnosis(congestive heart failure, +1), Diagnosis(cardiomyopathies, +1), Diagnosis(congenital heart defects, +1), Diagnosis(valvular heart disease, +1);

If *QRS_duration*=='prolonged':

Diagnosis(heart failure, +1), Diagnosis(congestive heart failure, +1), Diagnosis(atrial fibrillation, +1), Diagnosis(coronary disease, +1), Diagnosis(arrhythmia, +1), Diagnosis(ischemic heart disease, +1), Diagnosis(valvular heart disease, +1);

If *left_axis_deviation*==True:

Diagnosis(ischemic heart disease, +1), Diagnosis(heart failure, +1), Diagnosis(congestive heart failure, +1), Diagnosis(congenital heart defects, +1);

If *time_to_ID* > 50:

    Diagnosis(heart failure, +1), Diagnosis(coronary heart disease, +1);

If *CSA_muscle*=='reduction':

Diagnosis(heart failure, +1), Diagnosis(congestive heart failure, +1), Diagnosis(skeletal muscle atrophy, +1), etc.

If *muscle_strength*=='reduction':

Diagnosis(heart failure, +1), Diagnosis(congestive heart failure, +1), Diagnosis(skeletal muscle atrophy, +1), Diagnosis(muscular dystrophy, +1), etc.

If *muscle_fatigability*=='high':

Diagnosis(heart failure, +1), Diagnosis(congestive heart failure, +1), Diagnosis(skeletal muscle atrophy, +1), Diagnosis(muscular dystrophy, +1), etc.

If *submaximal_contraction*=='low':

Diagnosis(heart failure, +1), Diagnosis(congestive heart failure, +1), Diagnosis(skeletal muscle atrophy, +1), Diagnosis(muscular dystrophy, +1), Diagnosis(neurological disorders, +1), etc.

If *breathing_rate*=='low':

Diagnosis(heart failure, +1), Diagnosis(congestive heart failure, +1), Diagnosis(bradypnea, +1), Diagnosis(lung disorders, +1), Diagnosis(chronic bronchitis, +1), Diagnosis(pneumonia, +1), etc.

If *heart rate*=='high':

Diagnosis(heart failure, +1), Diagnosis(congestive heart failure, +1), Diagnosis(valvular heart disease, +1), Diagnosis(coronary heart disease, +1), Diagnosis(cardiomyopathies, +1), etc.

If *heart rate*=='enlarged':

Diagnosis(heart failure, +1), Diagnosis(congestive heart failure, +1), Diagnosis(stroke, +1), Diagnosis(cardiomyopathies, +1);

If *GSR* < 15: //low values -> negative -> associated to emotional state of sad, anxiety or anger.

Diagnosis(depression, +1), Diagnosis(anxiety, +1), etc.;

If *blood_pressure_fluid*=='high':

Diagnosis(heart failure, +1), Diagnosis(congestive heart failure, +1), Diagnosis(heart attack, +1), Diagnosis(stroke, +1), Diagnosis(arterial disease, +1), Diagnosis(aortic aneurysm, +1), etc.

There may of course be other diagnosis scores not considered above and in places these are indicated by "etc.". In another example focused on heart disease, only diagnosis scores relating to heart diseases may be included and diagnosis scores related to e.g. depression and anxiety may not be included/considered.

After summing each diagnosis score and selecting the highest four, the physiological diagnoses are:

- Heart failure with score=12
- Congestive heart failure with score=11
- Cardiomyopathies with score=3
- Valvular heart disease with score=3

A rule-based approach is followed to determine symptom diagnosis scores for a number of heart diseases based on the symptoms above in this example. Initially, the symptom diagnosis scores start at 0. Some comments about the rules are given inside "// //" below.

If *tired_weak*==True:

//This symptom may imply many diseases besides 'heart diseases'. Therefore, addition to score may be less than 1 in other examples//.

Diagnosis(heart failure, +1), Diagnosis(congestive heart failure, +1), etc.

If *chest_pain*==True:

//This symptom could be caused by many other diseases, but, if physiological measures and family history support case of 'heart disease', it would be determinant.//

Diagnosis(heart failure, +1), Diagnosis(congestive heart failure, +1), Diagnosis(muscle strain, +1), Diagnosis(GERD, +1), Diagnosis(asthma, +1), Diagnosis(costochondritis, +1), Diagnosis(valvular heart disease, +1), etc.

If *bad_breath*==True:

//Same comment as previous rule//

Diagnosis(heart failure, +1), Diagnosis(congestive heart failure, +1), Diagnosis(pneumonia, +1), Diagnosis(coronavirus, +1), Diagnosis(asthma, +1), Diagnosis(emphysema, +1), Diagnosis(valvular heart disease, +1), etc.

If *tachycardia*==True:

//Relevant for heart diseases//

Diagnosis(heart failure, +1), Diagnosis(congestive heart failure, +1), Diagnosis(coronary artery disease, +1), Diagnosis(heart attack, +1), Diagnosis(congenital heart defects, +1), Diagnosis(valvular heart disease, +1), Diagnosis(hypertension, +1), Diagnosis(cardiomyopathies, +1), etc.

If *nausea*==True:

//This symptom may imply many diseases besides 'heart diseases'. Therefore, addition to score may be less than 1 in other examples//Diagnosis(heart failure, +1), Diagnosis(congestive heart failure, +1), etc.

If *heartbeat_accelerated*==True:

//My be considered synonym to *'tachycardia'*. Relevant to heart diseases//

Diagnosis(heart failure, +1), Diagnosis(congestive heart failure, +1), Diagnosis(coronary artery disease, +1), Diagnosis(heart attack, +1), Diagnosis(congenital heart defects, +1), Diagnosis(valvular heart disease, +1), Diagnosis(hypertension, +1), Diagnosis(cardiomyopathies, +1), etc.

If *persistent_cough*==True:

//This symptom could be caused by many other diseases, but, if physiological measures and family history support case of 'heart disease', it would be determinant//. Diagnosis(heart failure, +1), Diagnosis(congestive heart failure, +1), Diagnosis(COPD, +1), Diagnosis(GERD, +1), Diagnosis(asthma, +1), Diagnosis(coronavirus, +1), etc.

After summing each diagnosis score and selecting the highest four the symptom diagnoses are:

- Heart failure with score=7
- Congestive heart failure with score=7
- Valvular heart disease with score=4
- Asthma with score=3

In this example, any diagnoses appearing in the top four symptom diagnoses as well as the top four physiological diagnoses are preliminary selected. Therefore, the following diagnoses are preliminarily selected:

- Heart failure
- Congestive heart failure
- Valvular heart disease

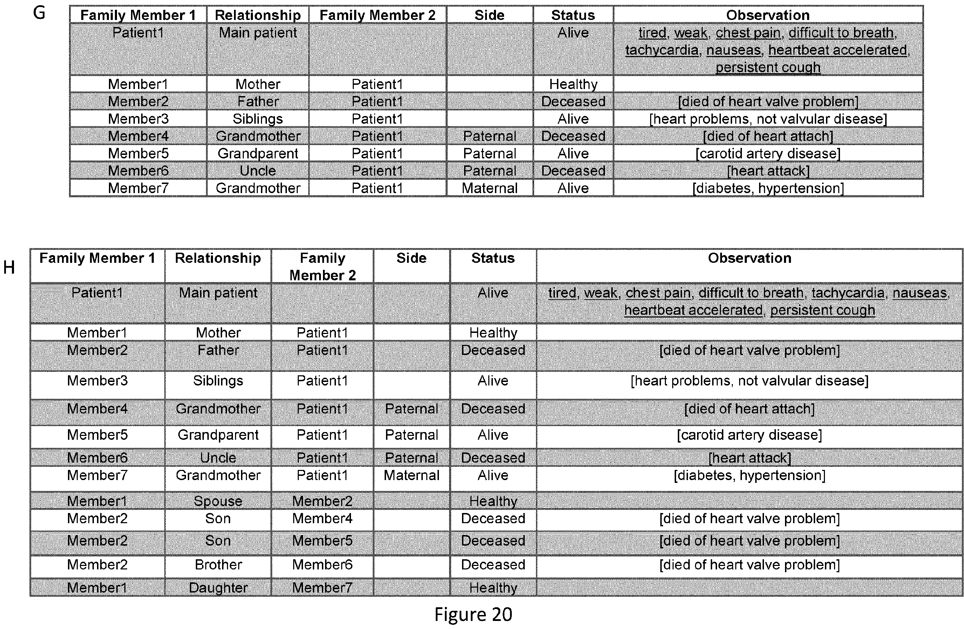

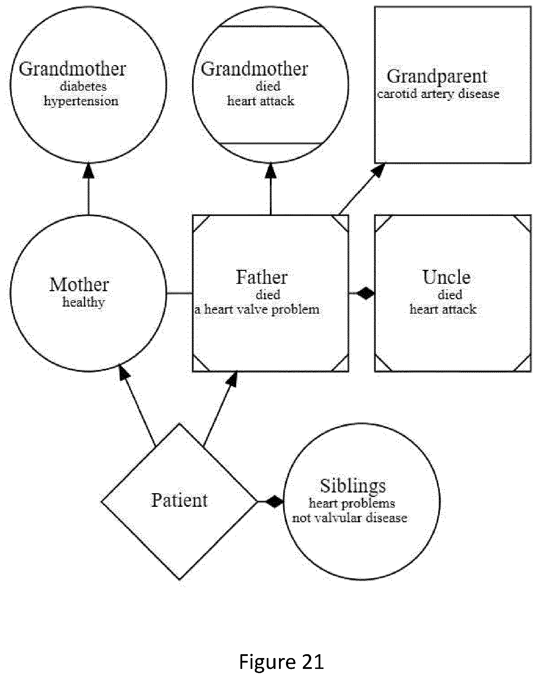

In this example, the preliminarily selected diagnoses are then corroborated through the analysis of the target patient's family medical history. The methods described above with reference to Figure 3 are followed to create a genogram. The initial table outputted by using the trained FMH model is table G in Figure 20. After extracting inner relations between the family members using a rule-based approach as described previously, table H in Figure 20 is obtained. The resultant genogram created from the extracted information in table H is illustrated in Figure 21. For visual representation of the genogram the following template configuration is used:

```
{"template_name":"Template_1", "female_basic_form":"circle",
"male_basic_form":"square", "deceased_form":"M", "relation_line_sibling":"diamond",
"relation_line_spouse":"none", "relation_line_parent":"normal",
"relation_line_nephew":"dot", "relation_line_cousin":"tee"}
```

The graph structure of the genogram is analysed according to a rule-based approach to generate the genogram diagnosis scores for a number of heart diseases. Initially, the genogram diagnosis scores start at zero. In this example, each genogram diagnosis score comprises a primary score based on information in the genogram about at least one sibling of the target patient; a secondary score based on information in the genogram about at least one parent of the target patient; and a tertiary score based on information in the genogram about at least one grandparent of the target patient and/or at least one sibling of a parent of the target patient and/or at least one cousin of the target patient. The rules in this case for calculating the primary, secondary, and tertiary scores comprise adding to the score if a relevant family member has or has had the disease/condition associated with the score. That is, For each diagnosis score relating to the diagnosis of a disease/condition, the rules comprise: adding to the primary score if a sibling of the target patient has or has had the disease/condition; adding to the secondary score if a parent of the target patient has or has had the disease/condition; and adding to the tertiary score if grandparent of the target patient and/or at least one sibling of a parent of the target patient and/or at least one cousin of the target patient has or has had the disease/condition. These rules may be considered derivable from the statement: *"if one of your immediate family members, such as a parent or sibling, has had a heart attack, a stroke, or was diagnosed with heart disease before the age of 60, this may indicate a family history of premature heart disease. This means that your chances of developing the same condition may be higher than normal."*

Computing a primary score may be considered as analysing a "same-degree line" of the genogram, a secondary score as analysing a "first-degree line", and a tertiary score as analysing a "second-degree line".

In this example, using the above FMH data, i.e. the genogram illustrated in Figure 21, the following primary, secondary, and tertiary diagnosis scores are generated:

1. Primary scores (analysis of the same-degree line, i.e., siblings' conditions) (weight of 45%): Diagnosis(heart failure, +1)

2. Secondary scores (analysis of the first-degree line, i.e., parents) (weight of 35%): Diagnosis(valvular heart disease, +1)

3. Tertiary scores (analysis of the second-degree line, i.e., grandparents, parents' siblings, cousins). (weight 20%): Diagnosis(heart attack, +2), Diagnosis(carotid artery disease, +1), Diagnosis(diabetes, +1), Diagnosis(hypertension, +1)

The genogram diagnosis scores are computed by summing the primary, secondary, and tertiary scores using the weightings to generate the following genogram diagnosis scores in this example:

- Heart failure = 1 * 45% = 0.45
- Valvular heart disease = 1 * 35% = 0.35
- Heart attack = 2 * 20% = 0.4
- Carotid artery disease = 1 * 20% = 0.2
- Diabetes = 1 * 20% = 0.2
- Hypertension = 1 * 20% = 0.2

In this example, a threshold of 0.3 is applied to the diagnoses. Then, the genogram diagnoses output are (ranked according to their scores):

- Heart failure
- Heart attack
- Valvular heart disease

In this example, the preliminarily selected diagnoses (based on the physiological measurements and the symptoms) are compared with the genogram diagnoses based on the following heuristic rules:

1. If a diagnosis appears in the genogram diagnoses and in the preliminarily selected diagnoses, the diagnosis will be marked as a primary diagnosis, and the other genogram diagnoses marked as secondary or less relevant.
2. If there is no diagnosis which appears in the genogram diagnoses and in the preliminarily selected diagnoses, all diagnoses extracted will be considered at the same importance.
3. If there are no genogram diagnoses, the preliminarily selected diagnoses are output as at least one final diagnosis.

In this example, the at least one final diagnosis output is:

- Primary diagnoses = Heart failure and Valvular heart disease
- Secondary diagnoses = Heart attack

The extracted and generated data/information may be merged with the EHR of the target patient.

Expose recommendations and prevention guidelines: In this example, recommendations are provided to the target patient based on the diagnoses output following clinical guidelines and optionally physicians' expertise. Any of the following recommendations may be provided.

For *Diagnosis(heart failure) ->* take next actions:

- Visit cardiologist as soon as possible
- Take medications if you have previous prescriptions, such as ACE (Angiotensin-converting enzyme) inhibitors or beta-blockers.
- Contact your specialist if experiencing side effects
- Keep as active as you can
- Report new symptoms immediately to your doctor
- Weight yourself every day, before you drink or eat
- Avoid excess salt

For potential *Diagnosis(valvular heart disease) ->* take next actions:

- Visit cardiologist as soon as possible
- Consult specialist if some kind of intervention for heart valve repair should be required because of your antecedents
- Practise regular and moderate exercise

Since all predicted diagnoses belong to the class of 'heart diseases', the recommendations can be shared and merged. Recommendations of a Primary diagnosis may be useful in preventing the future occurrence of a Secondary diagnosis.

Text-to-Speech techniques may be used to create an audio message that can be transmitted to the target patient. The generated message output in this example will include the diagnoses as well as the associated recommendations. In this example the following message is output to the target patient:

*"Your primary diagnoses are heart failure and valvular heart disease. As secondary diagnosis you could suffer heart attack if you don't take actions. Main action recommendations are to visit the cardiologist as soon as possible or consult with your specialist about medications, interventions and side effects, practise exercise regularly, be aware of your weight before meals to report drastic changes, avoid excess salt in the meals, and, finally, report new symptoms immediately to your doctor."*

Example for diagnosing 'mental illness'

Described below is an example following embodiments to diagnose mental illness (in this specific case a diagnosis of Depression is output).

Collection of information

Implicit data: Physiological measurements (i.e., vital signs) are collected e.g. in real-time from the data extracted with the sensors of the wearable device 200. In this example, the following physiological measurements (values) are collected. Most of them are not relevant to the diagnosis of mental health conditions such as depression. The relevance of some of the physiological measurements is indicated below in brackets.

- ECG sensor: Time-series values of the last 15 minutes. Irregular heart rate variability present (https://www.sciencedaily.com/releases/2017/11/171121095403.html) in the electrocardiogram waveforms. (Relevant to the diagnosis of mental health conditions despite the fact it could indicate other diseases)
- Thermometer: Temperature of 36.5°
- EMG sensor: Time-series values of the last 15 minutes. Normal values shown. [May not be relevant in this example]
- Pacemaker pulse detection: High heart rate. (Relevant to the diagnosis of mental health conditions despite the fact it could indicate other diseases)
- GSR sensors: Abnormal variability measurements. Alternance of increased and decreased skin conductivity and GSR activity values. (Relevant to the diagnosis of mental health conditions)Other measurements from Optical sensors:

  • High blood pressure
  • pH analysis: low pH levels (relevant)
  • Skin light intensity: No values out of the normal range
  • Blood-glucose levels: big fluctuations in blood sugar levels (relevant)

Physiological measurements may be classified e.g. as "normal", "low", "healthy", "abnormal", etc., by comparing (using a computer) those measurements with corresponding measurements of other people (e.g. with healthy and unhealthy hearts, in this case).

The symptoms and family medical history information are collected/acquired according to any of the examples described above (e.g. by the device 200 or from storage or a server or apparatus, etc.).

Receive/Transformation of information

The implicit data, i.e. physiological measurements, is sent to the Motherboard Module 60 and information is extracted from the data so that the physiological measurements may be processed according to the methods described above. Following the physiological measurements and their values described above for this example, the following information is extracted in this example. The relevance of some of the extracted and processed physiological measurements is indicated below in brackets

- ECG sensor: Transform the (15 minutes worth of) time-series to relevant categorical and numerical values. Specific values in this example include irregular heart rate variability=True (not determinant in the diagnosis of mental health conditions)
- Numerical value of temperature=36.5° - within normal range
- EMG: Normal values (not relevant in the diagnosis of mental health conditions)
- Pacemaker pulse detection: heart rate=high (Not determinant in the diagnosis of mental health conditions as this measurement could indicate multiple other diseases).
- GSR sensors: large variability of high and low values. The categorical information extracted is Values_variability=high (Relevant in the diagnosis of mental health conditions).
- Other measures from Optical sensors. Categorical values:

  • Blood pressure=high
  • pH analysis: low-levels
  • Skin light intensity: normal
  • Blood-glucose levels: big fluctuations

An example of the symptom information in this example, for example recorded by the target patient using the microphone 58 and converted to textual data is: *"I feel tired, apathetic and lack of enthusiasm. I usually have episodes of deep sadness and stress. Also I have no appetite and I feel anxious"*.

An example of the family medical history data in this example, for example recorded by the target patient using the microphone 58 and converted to textual data is: *"My father has bipolar disease and depression. My two sisters have*

*anxiety and depression. My paternal aunt has hypertension and depression. My paternal grandmother is died of suicide with mental illness".*

Processing information of Explicit data (symptoms and family medical history)

Real-time symptoms: NER techniques are used to extract the following entities from the textual data relating to symptoms: [tired, apathetic, lack of enthusiasm, sadness, stress, no appetite, anxious]

Family Medical History data: The methods described above are used to extract information from the family medical history data (e.g. step S31 using the trained FMH model). The FMH model may be trained specifically on training data including a family history of heart disease.

The Family History information extracted using the trained FMH model is stored in JSON format in the database of the device 200 or elsewhere in a system comprising the device 200 (e.g. an apparatus or server, etc.).

Determining Diagnoses

A rule-based approach is followed to determine physiological diagnosis scores for a number of mental health conditions (and other diseases/conditions) based on the physiological measurements above in this example. Initially, the physiological diagnosis scores start at 0. A change in a physiological diagnosis score is indicated as follows: a physiological measurement resulting in adding 1 to a diagnosis score is shown below by "Diagnosis([condition], +1)".

```
If heart rate variability=='irregular':
Diagnosis(heart failure, +1), Diagnosis(congestive heart failure, +1), Diagnosis(stroke,
+1), Diagnosis(depression, +1), Diagnosis(anxiety, +1), etc.;
If GSR_fluctuation = 'high':
Diagnosis(depression, +1), Diagnosis(anxiety, +1), etc.;
If blood_pressure_fluid=='high':
Diagnosis(heart failure, +1), Diagnosis(congestive heart failure, +1), Diagnosis(heart
attack, +1), Diagnosis(stroke, +1), Diagnosis(arterial disease, +1), Diagnosis(aortic
aneurysm, +1), Diagnosis(depression, +1), Diagnosis(anxiety, +1), etc.
If pH-levels = 'low':
Diagnosis(depression, +1), Diagnosis(anxiety, +1), Diagnosis(congestive heart failure,
+1), etc.;
If glucose_fluctuation = 'large':
Diagnosis(depression, +1), Diagnosis(anxiety, +1), Diagnosis(diabetes, +1), etc.;
```

There may of course be other diagnosis scores not considered above and in places these are indicated by "etc.". In another example focused on heart disease, only diagnosis scores relating to heart diseases may be included and diagnosis scores related to e.g. depression and anxiety may not be included/considered.

After summing each diagnosis score and selecting the highest four, the physiological diagnoses are:

- Depression with score=5
- Anxiety with score=5
- Congestive heart failure with score=3
- Heart failure=2

A rule-based approach is followed to determine symptom diagnosis scores for a number of heart diseases based on the symptoms above in this example. Initially, the symptom diagnosis scores start at 0. Some comments about the rules are given inside "// //" below.

If *tired_weak*==True:

    // This symptom may imply many diseases besides 'heart diseases'. Therefore, addition to score may be less than 1 in other examples //

Diagnosis(heart failure, +1), Diagnosis(congestive heart failure, +1), Diagnosis(depression, +1), Diagnosis(anxiety, +1), etc.


If *apathetic*==True:

    //Relevant in the diagnosis of mental health conditions//

Diagnosis(depression, +1), Diagnosis(major depressive disorder, +1), etc.


If *lack of enthusiasm*==True:

    //Relevant in the diagnosis of mental health conditions//

Diagnosis(depression, +1), Diagnosis(major depressive disorder, +1), Diagnosis(suicidal ideation, +1), etc.


If *sadness*==True:

    //Relevant in the diagnosis of mental health conditions//

Diagnosis(depression, +1), Diagnosis(major depressive disorder, +1), Diagnosis(suicidal ideation, +1), etc.



If *stress_anxious*==True:

Diagnosis(anxiety, +1)


If *no_appetite*==True:

Diagnosis(depression, +1), Diagnosis(major depressive disorder, +1, Diagnosis(anorexia, +1), Diagnosis(nutrition disorders, +1), etc.

After summing each diagnosis score and selecting the highest four the symptom diagnoses are:

- Depression with score=5
- Major depressive disorder with score=4
- Anxiety with score=2
- Suicidal ideation with score=2

In this example, any diagnoses appearing in the top four symptom diagnoses as well as the top four physiological diagnoses are preliminary selected. Therefore, the following diagnoses are preliminarily selected:

- Depression
- Anxiety

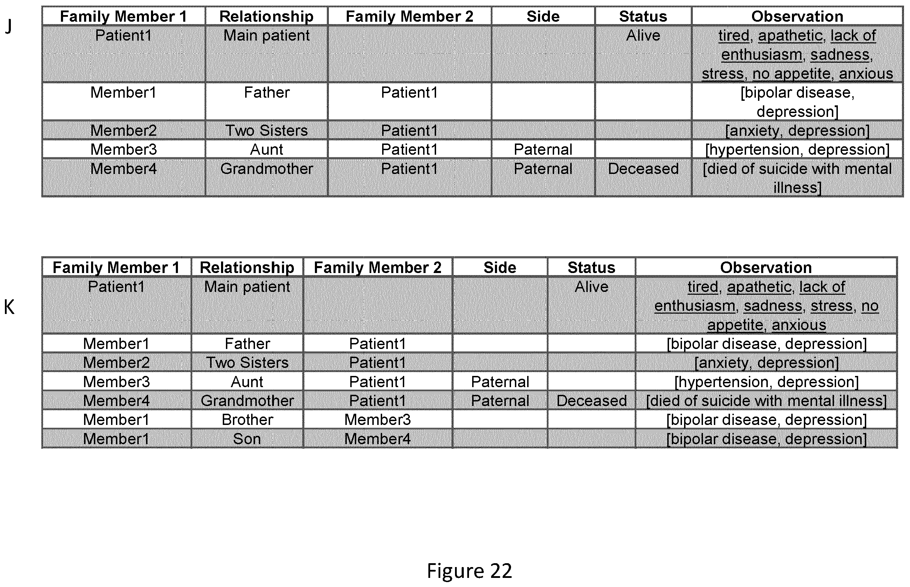

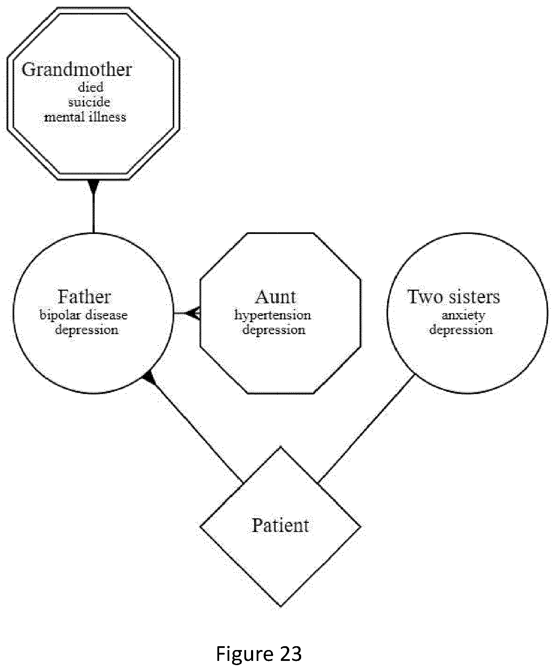

In this example, the preliminarily selected diagnoses are then corroborated through the analysis of the target patient's family medical history. The methods described above with reference to Figure 3 are followed to create a genogram. The initial table outputted by using the trained FMH model is table J in Figure 22. After extracting inner relations between the family members using a rule-based approach as described previously, table K in Figure 22 is obtained. The resultant genogram created from the extracted information in table K is illustrated in Figure 23.

For visual representation of the genogram the following template configuration is used:

```
{"template_name":"Template_4","female_basic_form":"octagon",
male_basic_form":"circle", "deceased_form":"double", "relation_line_sibling":"crow",
"relation_line_spouse":"box", "relation_line_parent":"inv",
"relation_line_nephew":"curve", "relation_line_cousin":"vee"}
```

The graph structure of the genogram is analysed according to a rule-based approach to generate the genogram diagnosis scores for a number of mental illnesses/conditions. Initially, the genogram diagnosis scores start at zero. In this example, each genogram diagnosis score comprises a primary score based on information in the genogram about at least one sibling of the target patient; a secondary score based on information in the genogram about at least one parent of the target patient; and a tertiary score based on information in the genogram about at least one grandparent of the target patient and/or at least one sibling of a parent of the target patient and/or at least one cousin of the target patient. The rules in this case for calculating the primary, secondary, and tertiary scores comprise adding to the score if a relevant family member has or has had the disease/condition associated with the score. That is, For each diagnosis score relating to the diagnosis of a disease/condition, the rules comprise: adding to the primary score if a sibling of the target patient has or has had the disease/condition; adding to the secondary score if a parent of the target patient has or has had the disease/condition; and adding to the tertiary score if grandparent of the target patient and/or at least one sibling of a parent of the target patient and/or at least one cousin of the target patient has or has had the disease/condition. These rules may be considered derivable from the statement: *"A positive family history is the most important risk factor for developing a depressive disorder and is often found in patients with more severe and recurrent disease and an earlier age of onset"*

Computing a primary score may be considered as analysing a "same-degree line" of the genogram, a secondary score as analysing a "first-degree line", and a tertiary score as analysing a "second-degree line".

In this example, using the above FMH data, i.e. the genogram illustrated in Figure 21, the following primary, secondary, and tertiary diagnosis scores are generated:

1. Primary scores (analysis of the same-degree line, i.e., siblings' conditions) (weight of 45%): Diagnosis(depression, +2), Diagnosis(anxiety, +2)
2. Secondary scores (analysis of the first-degree line, i.e., parents) (weight of 35%): Diagnosis(depression, +1), Diagnosis(bipolar disease, +1)
3. Tertiary scores (analysis of the second-degree line, i.e., grandparents, parents' siblings, cousins (weight 20%): Diagnosis(depression, +1), Diagnosis(hypertension, +1), Diagnosis(suicide, +1), Diagnosis(mental illness, +1)

The genogram diagnosis scores are computed by summing the primary, secondary, and tertiary scores using the weightings to generate the following genogram diagnosis scores in this example:

- Depression = 2 * 45% + 1 * 35% + 1 * 20% = 1.45
- Anxiety = 2 * 45% = 0.9
- Bipolar disease = 1 * 35% = 0.35
- Suicide = 1 * 20% = 0.2
- Mental illness = 1 * 20% = 0.2
- Hypertension = 1 * 20% = 0.2

In this example, a threshold of 0.3 is applied to the diagnoses. Then, the genogram diagnoses output are (ranked according to their scores):

- Depression
- Anxiety
- Bipolar disease

In this example, the preliminarily selected diagnoses (based on the physiological measurements and the symptoms) are compared with the genogram diagnoses based on the following heuristic rules:

1. If a diagnosis appears in the genogram diagnoses and in the preliminarily selected diagnoses, the diagnosis will be marked as a primary diagnosis, and the other genogram diagnoses marked as secondary or less relevant.
2. If there is no diagnosis which appears in the genogram diagnoses and in the preliminarily selected diagnoses, all diagnoses extracted will be considered at the same importance.
3. If there are no genogram diagnoses, the preliminarily selected diagnoses are output as at least one final diagnosis.

In this example, the at least one final diagnosis output is:

- Primary diagnoses = Depression and Anxiety
- Secondary diagnoses = Bipolar disease

It may be considered that for diagnosis of mental health conditions the physiological measurements are the least useful among physiological measurements, symptoms, and family medical history data. In this example all three factors are considered in generating at least one diagnosis. However in other examples the physiological measurements may not be considered. In fact, the at least one diagnosis may be based on any of the physiological measurements, symptoms, and family medical history data.

The extracted and generated data/information may be merged with the EHR of the target patient.

Expose recommendations and prevention guidelines: In this example, recommendations are provided to the target patient based on the diagnoses output following clinical guidelines and optionally physicians' expertise. Any of the following recommendations may be provided.

For *Diagnosis(Depression)* -> take next actions:

- Visit psychiatrist or psychologist
- Make a psychiatric evaluation
- Make blood lab tests to check your thyroid levels
- Ask appointment for psychotherapy
- Take antidepressants if physician recommends
- If nothing works, try alternative treatment options like electroconvulsive therapy or Transcranial magnetic stimulations.

For *Diagnosis(Anxiety)* -> take next actions:

- Take deep breaths
- Get enough sleep
- Eat well-balanced meals
- Limit alcohol and caffeine
- Exercise daily
- If nothing works, visit professional and take serotonin inhibitors

Since all predicted diagnoses belong to the class of 'mental illnesses', the recommendations can be shared and merged.

Text-to-Speech techniques may be used to create an audio message that can be transmitted to the target patient. The generated message output in this example will include the diagnoses as well as the associated recommendations. In this example the following message is output to the target patient:

*"Your primary diagnoses are depression and anxiety. Action recommendations are to visit psychiatrist, make psychiatric evaluations or blood lab tests, try psychotherapy or alternative treatments, eat well-balanced meals and limit alcohol and caffeine, take exercise and get enough sleep."*

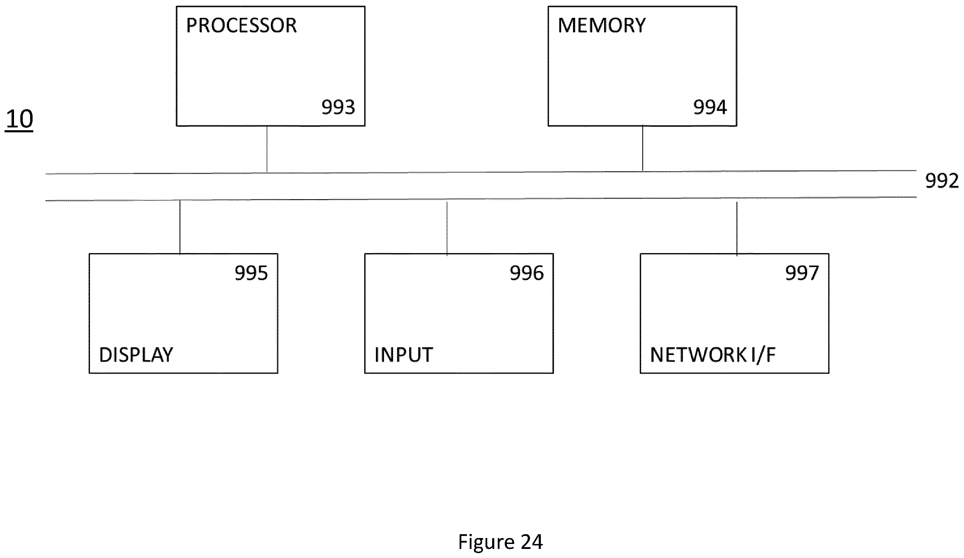

Figure 24 is a block diagram of an information processing apparatus 10 or a computing device 10, such as a data storage server, which embodies the present invention, and which may be used to implement some or all of the operations of a method embodying the present invention, and perform some or all of the tasks of apparatus of an embodiment. The computing device 10 may be used to implement any of the method steps described above, e.g. any of S21-S28 in Figure 2 and/or any of S31-S38 in Figure 3.

The computing device 10 comprises a processor 993 and memory 994. Optionally, the computing device also includes a network interface 997 for communication with other such computing devices, for example with other computing devices of invention embodiments. Optionally, the computing device also includes one or more input mechanisms such as keyboard and mouse 996, and a display unit such as one or more monitors 995. These elements may facilitate user interaction. The components are connectable to one another via a bus 992.

The memory 994 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a general purpose computer, special purpose computer, or special purpose processing device (e.g., one or more processors) to perform one or more functions or operations. For example, the computer-executable instructions may include those instructions for implementing a method disclosed herein, or any method steps disclosed herein, for example the method or any method steps illustrated in Figure 2 (steps S21-S28) and/or Figure 3 (steps S31-S38). Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement any of the method steps described herein. The memory 994 stores data being read and written by the processor 993 and may store unstructured (medical history) data and/or unstructured (medical history) training data and/or any of the extracted family history information described above and/or programs for executing any of the method steps S21-S28 and S31-S38 and/or any of the rule-based algorithms described above and/or genogram templates and/or at least one JSON configuration file and/or at least one properties file and/or at least one genogram and/or at least one report generated with recommendations and/or at least one physiological measurement and/or speech recording data and/or textual data converted from speech data and/or any of the diagnosis scores described above and the associated diagnoses. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 993 may be considered to comprise any of the modules 20, 22, 24, 42, 44, 441, 46, 461, and 40 in Figure 1. Any operations described as being implemented by a module may be implemented as a method by a computer and e.g. by the processor 993.

The display unit 995 may display a representation of data stored by the computing device, such as a genogram or a table or a diagnosis or a recommendation and/or GUI windows as described above and/or interactive representations enabling a user to interact with the apparatus 10 as described above with reference to any of steps S33-S37, S42, and S44 by e.g. drag and drop or selection interaction, and/or any other output described above, and may also display a cursor and dialog boxes and screens enabling interaction between a user and the programs and data stored on the computing device. The input mechanisms 996 may enable a user to input data and instructions to the computing device, such as enabling a user to input any of the user input described above.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention may be carried out on a computing device/apparatus 10 such as that illustrated in Figure 24. Such a computing device need not have every component illustrated in Figure 24, and may be composed of a subset of those components. For example, the apparatus 10 may comprise the processor 993 and the memory 994 connected to the processor 993. Or the apparatus 10 may comprise the processor 993, the memory 994 connected to the processor 993, and the display 995. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

In summary, embodiments may involve the following technical innovations:

- Automatic genogram generation from unstructured data (textual documents), including extracting data from unstructured texts with Natural Language Processing and Deep Learning techniques
- Automatic annotation of Family Medical History input datasets through a rule-based approach for text processing. Such input datasets will be needed as training data for the learning process that creates the Family Medical History model.
- Novel interface definition and implementation of form-based configuration templates to load/create ad-hoc genogram representations
  - GUI windows, new configuration scheme via JSON format, automatic processing of form-based templates, database for storing configurations for templates
- Automatic processing of inner-relations between family member roles from a patient's medical document (unstructured text), using NLP and a rule-based approach over known family relations
- Cross-validation of information using NLP and text processing techniques, showing recommendations for editing the genogram with explanations in natural text-style.
- Provision of automatic updates of the genogram following such recommendations.

Further benefits of embodiments include:

- Improved tools for Family Medical History Extraction
- Framework for useful exploitation of Family History information to detect inheritance conditions in patients
- Service that could be integrated in a bigger system for Clinical Decisions Support Methods disclosed herein may be used in Preventive Medicine, a relevant topic in AI

**Claims**

1. A computer-implemented method comprising:

   using a trained family medical history, FMH, model trained to use Named Entity Recognition, NER, and Relation Extraction, RE, to extract entities and relations between the entities from unstructured data, extracting information from unstructured medical data for at least one family member of a target patient, the information including: a family member role indicating a relationship of the family member with the target patient; a status of the family member; and at least one observation of the family member; and

   transforming the extracted information into a genogram comprising a plurality of nodes respectively representing a plurality of people including the target patient and the at least one family member, wherein an edge between two nodes indicates a relationship between the people represented by the two nodes.

EP 4 270 402 A1

2. The computer-implemented method as claimed in claim 1, further comprising training a family medical history model to obtain the trained FMH model, the training comprising:

receiving unstructured training medical data;
dividing the unstructured training medical data into sentences and tokenizing each sentence to extract tokens;
performing noun detection among the tokens;
identifying family member role keywords among the detected nouns and storing the family member role keywords as part of an annotation dataset;
identifying among the tokens at least one status keyword associated with a family member role keyword and storing the at least one status keyword in association with the family member role keyword in the annotation dataset;
identifying among the tokens at least one observation associated with a family member role keyword and storing the at least one observation in association with the family member role keyword in the annotation dataset;
using the annotation dataset to annotate the unstructured training medical data; and
using the annotations in the unstructured training medical data as ground truth information training the family medical history model to use NER and RE to extract as entities, from the unstructured training medical data, information included in the annotations.

3. The computer-implemented method as claimed in claim 1 or claim 2, wherein identifying at least one observation associated with a family member role keyword includes, the family member role keyword being a subject family member role keyword:
analyzing tokens occurring in the unstructured training medical data between the appearance of the subject family member role keyword and the next appearance of a family member role keyword and identifying at least one token that fulfils at least one observation criterion as at least one observation associated with the subject family member role keyword.

4. The computer-implemented method as claimed in any of claims 1 to 3, wherein identifying at least one status keyword associated with a family member role keyword comprises implementing a first status search comprising:
analyzing tokens occurring in the unstructured training medical data between the appearance of a family member role keyword, being a first family member role keyword, and the next appearance of a family member role keyword to identify at least one status keyword, and identifying the at least one status keyword as being associated with the first family member role keyword.

5. The computer-implemented method as claimed in any of the preceding claims, further comprising:

using the trained family medical history model to extract further said information from further unstructured medical data for at least one of the family members of the target patient;
comparing the extracted information with the extracted further information and, when there is at least one inconsistency comprising a first entity in the extracted information and a second entity in the extracted further information, identifying the inconsistency; and
displaying information indicating the inconsistency.

6. The computer-implemented method as claimed in any of the preceding claims, comprising using a template to transform the extracted information into the genogram.

7. The computer-implemented method as claimed in claim 6, comprising:

receiving user input to generate the template; or
receiving user input to select the template from a list of templates; or
receiving user input to edit the template.

8. The computer-implemented method as claimed in any of the preceding claims, comprising, after transforming the extracted information into the genogram, receiving user input to edit:

a form of the nodes; and/or
a form of the edges; and/or
a configuration in which information will be displayed for each node; and/or
text associated with each node.

9.  The computer-implemented method as claimed in claim 7 or claim 8, comprising using a graphical user interface, GUI, to receive the user input.

10. The computer-implemented method as claimed in any of the preceding claims, further comprising outputting a diagnosis for the target patient based on the genogram.

11. The computer-implemented method as claimed in any of claims 1 to 9, further comprising receiving at least one physiological measurement of the target patient and outputting a diagnosis for the target patient based on the at least one physiological measurement and the genogram.

12. The computer-implemented method as claimed in any of claims 1 to 9, comprising:

    predicting at least one genogram diagnosis, the at least one genogram diagnosis being at least one diagnosis of the target patient based on the genogram;
    predicting at least one physiological diagnosis, the at least one physiological diagnosis being at least one diagnosis of the target patient based on at least one symptom of the target patient and/or at least one physiological measurement of the target patient; and
    comparing the at least one genogram diagnosis with the at least one physiological diagnosis and, when at least one diagnosis among the at least one genogram diagnosis is the same as a diagnosis among the at least one physiological diagnosis, outputting the at least one diagnosis as at least one final diagnosis.

13. A wearable device comprising:

    at least one processor configured to carry out the computer-implemented method as claimed in claim 11 or claim 12;
    at least one sensor configured to obtain the at least one physiological measurement from the target patient; and/or
    a microphone configured to record speech input from the user and obtain the at least one symptom of the target patient.

14. A computer program which, when run on a computer, causes the computer to carry out a method comprising:

    using a trained family medical history, FMH, model trained to use Named Entity Recognition, NER, and Relation Extraction, RE, to extract entities and relations between the entities from unstructured data, extracting information from unstructured medical data for at least one family member of a target patient, the information including: a family member role indicating a relationship of the family member with the target patient; a status of the family member; and at least one observation of the family member; and
    transforming the extracted information into a genogram comprising a plurality of nodes respectively representing a plurality of people including the target patient and the at least one family member, wherein an edge between two nodes indicates a relationship between the people represented by the two nodes.

15. An information processing apparatus comprising a memory and a processor connected to the memory, wherein the processor is configured to:

    using a trained family medical history, FMH, model trained to use Named Entity Recognition, NER, and Relation Extraction, RE, to extract entities and relations between the entities from unstructured data, extract information from unstructured medical data for at least one family member of a target patient, the information including: a family member role indicating a relationship of the family member with the target patient; a status of the family member; and at least one observation of the family member; and
    transform the extracted information into a genogram comprising a plurality of nodes respectively representing a plurality of people including the target patient and the at least one family member, wherein an edge between two nodes indicates a relationship between the people represented by the two nodes.

**Amended claims in accordance with Rule 137(2) EPC.**

1.  A computer-implemented method comprising:

    using a trained family medical history, FMH, model trained to use Named Entity Recognition, NER, and Relation Extraction, RE, to extract entities and relations between the entities from unstructured data, extracting information from unstructured medical data for at least one family member of a target patient, the information including: a family member role indicating a relationship of the family member with the target patient; a status of the family member; and at least one observation of the family member; and

transforming the extracted information into a genogram comprising a plurality of nodes respectively representing a plurality of people including the target patient and the at least one family member, wherein an edge between two nodes indicates a relationship between the people represented by the two nodes, wherein the method further comprises predicting and outputting a diagnosis, wherein predicting the diagnosis comprises:

> maintaining a genogram diagnosis score for each of a plurality of possible diagnoses;
> adjusting at least one of the genogram diagnosis scores based on a set of genogram diagnosis rules and based on the genogram; and
> determining at least one of the possible diagnoses having the highest genogram diagnosis score as at least one genogram diagnosis,

wherein the set of genogram diagnosis rules comprises adding to a diagnosis score for at least one disease and/or condition if a family member of the target patient has or has had that at least one disease and/or condition.

2. The computer-implemented method as claimed in claim 1, further comprising training a family medical history model to obtain the trained FMH model, the training comprising:

> receiving unstructured training medical data;
> dividing the unstructured training medical data into sentences and tokenizing each sentence to extract tokens;
> performing noun detection among the tokens;
> identifying family member role keywords among the detected nouns and storing the family member role keywords as part of an annotation dataset;
> identifying among the tokens at least one status keyword associated with a family member role keyword and storing the at least one status keyword in association with the family member role keyword in the annotation dataset;
> identifying among the tokens at least one observation associated with a family member role keyword and storing the at least one observation in association with the family member role keyword in the annotation dataset;
> using the annotation dataset to annotate the unstructured training medical data; and
> using the annotations in the unstructured training medical data as ground truth information training the family medical history model to use NER and RE to extract as entities, from the unstructured training medical data, information included in the annotations.

3. The computer-implemented method as claimed in claim 1 or claim 2, wherein identifying at least one observation associated with a family member role keyword includes, the family member role keyword being a subject family member role keyword:

> analyzing tokens occurring in the unstructured training medical data between the appearance of the subject family member role keyword and the next appearance of a family member role keyword and identifying at least one token that fulfils at least one observation criterion as at least one observation associated with the subject family member role keyword,
> wherein the at least one observation criterion comprises the criterion that a token must have or be inside a syntax tag of pobj or dobj or conj to be considered an observation.

4. The computer-implemented method as claimed in any of claims 1 to 3, wherein identifying at least one status keyword associated with a family member role keyword comprises implementing a first status search comprising: analyzing tokens occurring in the unstructured training medical data between the appearance of a family member role keyword, being a first family member role keyword, and the next appearance of a family member role keyword to identify at least one status keyword, and identifying the at least one status keyword as being associated with the first family member role keyword.

5. The computer-implemented method as claimed in any of the preceding claims, further comprising:

> using the trained family medical history model to extract further said information from further unstructured medical data for at least one of the family members of the target patient;

comparing the extracted information with the extracted further information and, when there is at least one inconsistency comprising a first entity in the extracted information and a second entity in the extracted further information, identifying the inconsistency, wherein the first and second entities are observations or statuses of one of the family members of the target patient; and

displaying information indicating the inconsistency.

6. The computer-implemented method as claimed in any of the preceding claims, comprising using a template to transform the extracted information into the genogram.

7. The computer-implemented method as claimed in claim 6, comprising:

receiving user input to generate the template; or
receiving user input to select the template from a list of templates; or
receiving user input to edit the template.

8. The computer-implemented method as claimed in any of the preceding claims, comprising, after transforming the extracted information into the genogram, receiving user input to edit:

a form of the nodes; and/or
a form of the edges; and/or
a configuration in which information will be displayed for each node; and/or
text associated with each node.

9. The computer-implemented method as claimed in claim 7 or claim 8, comprising using a graphical user interface, GUI, to receive the user input.

10. The computer-implemented method as claimed in any of claims 1 to 9, comprising:
predicting at least one physiological diagnosis, the at least one physiological diagnosis being at least one diagnosis of the target patient based on at least one physiological measurement of the target patient, wherein predicting the at least one physiological diagnosis comprises:

maintaining a physiological diagnosis score for each of a plurality of possible diagnoses;
adjusting at least one of the physiological diagnosis scores based on a set of physiological diagnosis rules and based on the at least one physiological measurement of the target patient; and
determining at least one of the possible diagnoses having the highest physiological diagnosis score as the at least one physiological diagnosis; and

comparing the at least one genogram diagnosis with the at least one physiological diagnosis and, when at least one diagnosis among the at least one genogram diagnosis is the same as a diagnosis among the at least one physiological diagnosis, outputting the at least one diagnosis as at least one final diagnosis.

11. A wearable device (200) comprising:

at least one processor (66) configured to carry out the computer-implemented method as claimed in claim 10;
at least one sensor (81, 82, 83, 84, 85, 86) configured to obtain the at least one physiological measurement from the target patient; and/or
a microphone (58) configured to record speech input from the user and obtain the at least one symptom of the target patient.

12. A computer program which, when run on a computer, causes the computer to carry out a method comprising:

using a trained family medical history, FMH, model trained to use Named Entity Recognition, NER, and Relation Extraction, RE, to extract entities and relations between the entities from unstructured data, extracting information from unstructured medical data for at least one family member of a target patient, the information including: a family member role indicating a relationship of the family member with the target patient; a status of the family member; and at least one observation of the family member; and
transforming the extracted information into a genogram comprising a plurality of nodes respectively representing a plurality of people including the target patient and the at least one family member, wherein an edge between two nodes indicates a relationship between the people represented by the two nodes, wherein the method further comprises predicting and outputting a diagnosis, wherein predicting the diagnosis comprises:

maintaining a genogram diagnosis score for each of a plurality of possible diagnoses;
adjusting at least one of the genogram diagnosis scores based on a set of genogram diagnosis rules and based on the genogram; and
determining at least one of the possible diagnoses having the highest genogram diagnosis score as at least one genogram diagnosis,

wherein the set of genogram diagnosis rules comprises adding to a diagnosis score for at least one disease and/or condition if a family member of the target patient has or has had that at least one disease and/or condition.

13. An information processing apparatus (10) comprising a memory (994) and a processor (993) connected to the memory (994), wherein the processor (993) is configured to:

using a trained family medical history, FMH, model trained to use Named Entity Recognition, NER, and Relation Extraction, RE, to extract entities and relations between the entities from unstructured data, extract information from unstructured medical data for at least one family member of a target patient, the information including: a family member role indicating a relationship of the family member with the target patient; a status of the family member; and at least one observation of the family member; and
transform the extracted information into a genogram comprising a plurality of nodes respectively representing a plurality of people including the target patient and the at least one family member, wherein an edge between two nodes indicates a relationship between the people represented by the two nodes, wherein the processor (993) is further configured to predict and output a diagnosis, wherein predicting the diagnosis comprises:

maintaining a genogram diagnosis score for each of a plurality of possible diagnoses;
adjusting at least one of the genogram diagnosis scores based on a set of genogram diagnosis rules and based on the genogram; and
determining at least one of the possible diagnoses having the highest genogram diagnosis score as at least one genogram diagnosis,

wherein the set of genogram diagnosis rules comprises adding to a diagnosis score for at least one disease and/or condition if a family member of the target patient has or has had that at least one disease and/or condition.

**Model Training Module 20**

20

Training data

BioBERT    mBERT

Pre-trained Language Models

Label Annotator Engine 22

Family Medical History (FMH) Trainer 24

FMH Model

Family Medical information in new clinical document & Historical documents (from patient and family)

Family Medical History (FMH) Extractor 42

**Genogram Module 40**

Genogram Editor 46

Cross-Validation Checker & Recommender Module 461

Automatic Genogram Creator 44

Genogram Template Representation Setup Module 441

Final Genogram Diagram

Standards & Nomenclatures

100                    Figure 1

S21 | Process Tokenization, Noun Detection, POS tagging and Syntax Tree Dependencies

S22 | Extract family member roles

S23 | Extract family side associated to each family role

S24 | Extract status associated to each family role

S25 | Extract observations associated to each family role

S26 | Extract modality of observations (pos, neg) associated to each family role

S27 | Build annotated dataset in JSON from extracted entities and relations

S28 | Transform annotated dataset to BIO format

Figure 2

EP 4 270 402 A1

S31 | Data processing of FMH results

Genogram Template Representation Setup Module

Load pre-saved templates   S32

Select template or create new one   S33

S34 New?

S35 Define new form template of representation

yes

no

S37 Init representation template

S36 Save new template defined

S38 | Transform results to representation template

Figure 3

| Family Member 1 | Relationship | Family Member 2 | Status | Observation |
|---|---|---|---|---|
| Diego_a3e4 | Main patient | | Alive | [tiredness] |
| Luna_r4s5 | Mother | Diego_a3e4 | Alive | [diabetes, brain tumour] |
| Alejandro_u4e2 | Father | Diego_a3e4 | Deceased | [died of diabetes] |
| Alejandro_u4e2 | Spouse | Luna_r4s5 | - | None |
| María_y34s | Sister | Alejandro_u4e2 | Deceased | [died of diabetes] |

Figure 4

EP 4 270 402 A1

Figure 5

EP 4 270 402 A1

Figure 7

Figure 6

58

Figure 8

S45 Data processing of FM historical (from patient and relatives)

S46 Cross historical information with current genogram & changes

S47 Validate and Detect contradictions

S48 Prepare Report and Recommendations

S49 Show report & recommendations to user in a pop-up window

Cross-Validation Checker & Recommender Module

S44 User includes changes

S41 Load Editor GUI window

S42 Edit?

yes

no

S43 Return final Genogram representation

Figure 9

Node shape modifications

Male   Female   Gender unknown   Pet

Observations and status modifications

Edge modifications

Engagement

Engagement and cohabitation

Marriage

Separation in fact

Alejandro_u4e2

[Died of diabetes]

Figure 10

| Family Member 1 | Relation ship | Family Member 2 | Status | Observation |
|---|---|---|---|---|
| Diego_a3e4 | Main patient | | Alive | [tiredness] |
| Luna_r4s5 | Mother | Diego_a3e4 | Alive | [mental disease] |
| Alejandro_u4e2 | Father | Diego_a3e4 | Deceased | [died of diabetes] |
| Alejandro_u4e2 | Spouse | Luna_r4s5 | Deceased | [died of diabetes] |
| María_y34s | Sister | Alejandro_u4e2 | Deceased | [died of diabetes] |
| María_y34s | Sister-in-law | Luna_r4s5 | Deceased | [died of diabetes] |
| María_y34s | Aunt | Diego_a3e4 | Deceased | [died of diabetes] |

A (ID: 01)

| Family Member 1 | Relation ship | Family Member 2 | Status | Observation |
|---|---|---|---|---|
| Luna_r4s5 | Main patient | | Alive | [schizophrenia] |
| Diego_a3e4 | Son | Luna_r4s5 | Alive | None |
| Alejandro_u4e2 | Father | Diego_a3e4 | Deceased | [diabetes, died of heart failure] |
| Alejandro_u4e2 | Spouse | Luna_r4s5 | Deceased | [diabetes, died of heart failure] |
| María_y34s | Sister-in-law | Luna_r4s5 | Deceased | [died of diabetes] |
| María_y34s | Aunt | Diego_a3e4 | Deceased | [died of diabetes] |
| María_y34s | Sister | Alejandro_u4e2 | Deceased | [died of diabetes] |

B (ID: 02)

| Table ID 1 | Table ID 2 | Family Member | Contradiction Field | Contradiction Value 1 | Contradiction Value 2 |
|---|---|---|---|---|---|
| 01 | 02 | Luna_r4s5 | Observation | mental disease | schizophrenia |
| 01 | 02 | Alejandro_u4e2 | Observation | died of diabetes | diabetes, died of heart failure |

C

Figure 11

EP 4 270 402 A1

**Validation Report**

[Patient Name]
[Patient Sex, Age, Location, etc.]
[Doctor Name]
[Excerpt of origin clinical report]

### Information in Genogram (Table ID=01)

| Family Member 1 | Relationship | Family Member 2 | Status | Observation |
|---|---|---|---|---|
| Diego_a3e4 | Main patient | | Alive | [tiredness] |
| Luna_r4s5 | Mother | Diego_a3e4 | Alive | [mental disease] |
| Alejandro_u4e2 | Father | Diego_a3e4 | Deceased | [died of diabetes] |
| Alejandro_u4e2 | Spouse | Luna_r4s5 | Deceased | [died of diabetes] |
| María_y34s | Sister | Alejandro_u4e2 | Deceased | [died of diabetes] |
| María_y34s | Sister-in-law | Luna_r4s5 | Deceased | [died of diabetes] |
| María_y34s | Aunt | Diego_a3e4 | Deceased | [died of diabetes] |

### Summary of Table of Inconsistencies

| Table ID 1 | Table ID 2 | Family Member | Contradiction Field | Contradiction Value 1 | Contradiction Value 2 |
|---|---|---|---|---|---|
| 01 | 02 | Luna_r4s5 | Observation | mental disease | schizophrenia |
| 01 | 02 | Alejandro_u4e2 | Observation | died of diabetes | diabetes, died of heart failure |
| 01 | 03 | Alejandro_u4e2 | Observation | died of diabetes | diabetes, died of heart failure |

### Recommendations

The family member 'Luna_r4s5' should have an Observation of 'schizophrenia' instead 'mental disease', because the Table with ID 02 belongs to the main patient of 'Luna_r4s5', and, in such table the observation is 'schizophrenia'. Information of main patient table should prevail over Table 01.

The family member 'Alejandro_u4e2' should have an Observation of 'diabetes, died of heart failure' instead 'died of diabetes', because the Table with ID 03 belongs to the main patient of 'Alejandro_u4e2', and, in such table the observation is 'diabetes, died of heart failure'. Besides, the Table with ID 02 has the same Observation than Table with ID 03.

Figure 12

**Edition Recommendations**

The family member '**Luna_r4s5**' should have an Observation of 'schizophrenia' instead 'mental disease

The family member '**Alejandro_u4e2**' should have an Observation of 'diabetes, died of heart failure' instead 'died of diabetes'

Download    Update

Figure 13

| Family Member 1 | Relationship | Family Member 2 | Status | Observation |
|---|---|---|---|---|
| Diego_a3e4 | Main patient | | Alive | [tiredness] |
| Luna_r4s5 | Mother | Diego_a3e4 | Alive | [mental disease] |
| Alejandro_u4e2 | Father | Diego_a3e4 | Deceased | [died of diabetes] |
| Alejandro_u4e2 | Spouse | Luna_r4s5 | Deceased | [died of diabetes] |
| María_y34s | Sister | Alejandro_u4e2 | Deceased | [died of diabetes] |
| María_y34s | Sister-in-law | Luna_r4s5 | Deceased | [died of diabetes] |
| María_y34s | Aunt | Diego_a3e4 | Deceased | [died of diabetes] |

Figure 14

| Family Member 1 | Relationship | Family Member 2 | Side | Status | Observation |
|---|---|---|---|---|---|
| Diego_a3e4 | Main patient | | | Alive | [tiredness] |
| Luna_r4s5 | Mother | Diego_a3e4 | | Alive | [mental disease] |
| Alejandro_u4e2 | Father | Diego_a3e4 | | Deceased | [died of diabetes] |
| María_y34s | Aunt | Diego_a3e4 | Paternal | Deceased | [died of diabetes] |

D

| Family Member 1 | Relationship | Family Member 2 | Side | Status | Observation |
|---|---|---|---|---|---|
| Diego_a3e4 | Main patient | | | Alive | [tiredness] |
| Luna_r4s5 | Mother | Diego_a3e4 | | Alive | [mental disease] |
| Alejandro_u4e2 | Father | Diego_a3e4 | | Deceased | [died of diabetes] |
| Alejandro_u4e2 | Spouse | Luna_r4s5 | | Deceased | [died of diabetes] |
| María_y34s | Aunt | Diego_a3e4 | Paternal | Deceased | [died of diabetes] |
| María_y34s | Sister | Alejandro_u4e2 | | Deceased | [died of diabetes] |
| María_y34s | Sister-in-law | Luna_r4s5 | | Deceased | [died of diabetes] |

E

Figure 15

| Family Member 1 | Relationship | Family Member 2 | Status | Observation |
|---|---|---|---|---|
| Diego_a3e4 | Main patient | | Alive | [respiratory issues during consult] |
| Natalia_r84w | Brother | Diego_a3e4 | Alive | [respiratory issues during consult] |

ID: 03

| Family Member 1 | Relationship | Family Member 2 | Status | Observation |
|---|---|---|---|---|
| Luna_r4s5 | Main patient | | Alive | [schizophrenia] |
| Natalia_r84w | Son | Luna_r4s5 | Alive | [None] |
| Diego_a3e4 | Son | Luna_r4s5 | Alive | [None] |
| Natalia_r84w | Brother | Diego_a3e4 | Alive | [None] |

ID: 04

Figure 16

EP 4 270 402 A1

| Table ID 1 | Table ID 2 | Family Member | Contradiction Field | Contradiction Value 1 | Contradiction Value 2 |
|---|---|---|---|---|---|
| 00 | 03 | Luna_r4s5 | Observation | mental disease | schizophrenia |
| 00 | 03 | Diego_a3e4 | Observation | None | respiratory issues during consult |
| 03 | 04 | Diego_a3e4 | Observation | respiratory issues during consult | None |
| 03 | 04 | Natalia_r84w | Observation | respiratory issues during consult | None |

F

Figure 17

(A)

(B)

Figure 18

Figure 19

| Family Member 1 | Relationship | Family Member 2 | Side | Status | Observation |
|---|---|---|---|---|---|
| Patient1 | Main patient | | | Alive | tired, weak, chest pain, difficult to breath, tachycardia, nauseas, heartbeat accelerated, persistent cough |
| Member1 | Mother | Patient1 | | Healthy | |
| Member2 | Father | Patient1 | | Deceased | [died of heart valve problem] |
| Member3 | Siblings | Patient1 | | Alive | [heart problems, not valvular disease] |
| Member4 | Grandmother | Patient1 | Paternal | Deceased | [died of heart attach] |
| Member5 | Grandparent | Patient1 | Paternal | Alive | [carotid artery disease] |
| Member6 | Uncle | Patient1 | Paternal | Deceased | [heart attack] |
| Member7 | Grandmother | Patient1 | Maternal | Alive | [diabetes, hypertension] |

| Family Member 1 | Relationship | Family Member 2 | Side | Status | Observation |
|---|---|---|---|---|---|
| Patient1 | Main patient | | | Alive | tired, weak, chest pain, difficult to breath, tachycardia, nauseas, heartbeat accelerated, persistent cough |
| Member1 | Mother | Patient1 | | Healthy | |
| Member2 | Father | Patient1 | | Deceased | [died of heart valve problem] |
| Member3 | Siblings | Patient1 | | Alive | [heart problems, not valvular disease] |
| Member4 | Grandmother | Patient1 | Paternal | Deceased | [died of heart attach] |
| Member5 | Grandparent | Patient1 | Paternal | Alive | [carotid artery disease] |
| Member6 | Uncle | Patient1 | Paternal | Deceased | [heart attack] |
| Member7 | Grandmother | Patient1 | Maternal | Alive | [diabetes, hypertension] |
| Member1 | Spouse | Member2 | | Healthy | |
| Member2 | Son | Member4 | | Deceased | [died of heart valve problem] |
| Member2 | Son | Member5 | | Deceased | [died of heart valve problem] |
| Member2 | Brother | Member6 | | Deceased | [died of heart valve problem] |
| Member1 | Daughter | Member7 | | Healthy | |

Figure 20

EP 4 270 402 A1

Figure 21

J

| Family Member 1 | Relationship | Family Member 2 | Side | Status | Observation |
|---|---|---|---|---|---|
| Patient1 | Main patient | | | Alive | tired, apathetic, lack of enthusiasm, sadness, stress, no appetite, anxious |
| Member1 | Father | Patient1 | | | [bipolar disease, depression] |
| Member2 | Two Sisters | Patient1 | | | [anxiety, depression] |
| Member3 | Aunt | Patient1 | Paternal | | [hypertension, depression] |
| Member4 | Grandmother | Patient1 | Paternal | Deceased | [died of suicide with mental illness] |

K

| Family Member 1 | Relationship | Family Member 2 | Side | Status | Observation |
|---|---|---|---|---|---|
| Patient1 | Main patient | | | Alive | tired, apathetic, lack of enthusiasm, sadness, stress, no appetite, anxious |
| Member1 | Father | Patient1 | | | [bipolar disease, depression] |
| Member2 | Two Sisters | Patient1 | | | [anxiety, depression] |
| Member3 | Aunt | Patient1 | Paternal | | [hypertension, depression] |
| Member4 | Grandmother | Patient1 | Paternal | Deceased | [died of suicide with mental illness] |
| Member1 | Brother | Member3 | | | [bipolar disease, depression] |
| Member1 | Son | Member4 | | | [bipolar disease, depression] |

Figure 22

Figure 23

10

PROCESSOR

993

MEMORY

994

992

DISPLAY

995

INPUT

996

NETWORK I/F

997

Figure 24

Europäisches
Patentamt

European
Patent Office

Office européen
des brevets

# EUROPEAN SEARCH REPORT

**Application Number**

EP 22 38 2392

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/181128 A1 (RISKIN DANIEL J [US] ET AL) 26 June 2014 (2014-06-26)<br>* claims 1-14 *<br>* paragraph [0010] *<br>* paragraph [0012] *<br>* paragraph [0062] *<br>* paragraph [0067] *<br>* paragraph [0070] *<br>* paragraph [0072] *<br>* paragraph [0084] *<br>* paragraph [0112] *<br>* paragraph [0127] – paragraph [0130] *<br>* Facsimile page 2 to page 3 *<br>----- | 1-15 | INV.<br>G16H10/20<br>G16H10/60<br>G16H15/00<br>G16H50/70 |
| X | US 2021/334462 A1 (KUKREJA MANSI [US] ET AL) 28 October 2021 (2021-10-28)<br>* claims 1-2 *<br>* paragraph [0019] *<br>* paragraph [0022] *<br>* paragraph [0036] *<br>* paragraph [0039] *<br>* paragraph [0043] *<br>* paragraph [0051] – paragraph [0052] *<br>* paragraph [0084] *<br>* paragraph [0115] – paragraph [0116] *<br>* paragraph [0121] – paragraph [0123] *<br>* Facsimile page 3 to page 4 *<br>----- | 1-15 | |
| X | JP 2019 101608 A (TOYOTA CENTRAL RES & DEV) 24 June 2019 (2019-06-24)<br>* claims 1-2 *<br>* paragraph [0002] – paragraph [0009] *<br>* paragraph [0014] – paragraph [0018] *<br>* paragraph [0043] – paragraph [0049] *<br>* paragraph [0063] – paragraph [0066] *<br>* Facsimile page 10 *<br>----- | 1-15 | **TECHNICAL FIELDS SEARCHED (IPC)**<br><br>G16H |

The present search report has been drawn up for all claims

| Place of search | Date of completion of the search | Examiner |
|---|---|---|
| Munich | 6 October 2022 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 2392

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2022

| Patent document cited in search report | | Publication date | Patent family member(s) | | Publication date |
|---|---|---|---|---|---|
| US 2014181128 | A1 | 26-06-2014 | AU | 2012225661 A1 | 19-09-2013 |
| | | | US | 2014181128 A1 | 26-06-2014 |
| | | | WO | 2012122122 A1 | 13-09-2012 |
| US 2021334462 | A1 | 28-10-2021 | NONE | | |
| JP 2019101608 | A | 24-06-2019 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BENNETT, ROBIN L. et al.** Standardized human pedigree nomenclature: update and assessment of the recommendations of the National Society of Genetic Counselors. *Journal of genetic counseling,* 2008, vol. 17 (5), 424-433
- **RICH, EUGENE C. et al.** Reconsidering the family history in primary care. *Journal of general internal medicine,* 2004, vol. 19 (3), 273-280
- **BENNETT, ROBIN L. et al.** Standardized human pedigree nomenclature: update and assessment of the recommendations of the National Society of Genetic Counselors. *Journal of genetic counseling,* 2008, vol. 17 (5), 424-433